# EUROPEAN PATENT APPLICATION

(11) **EP 4 257 586 A1**
(43) Date of publication of application: **11.10.2023**
(21) Application number: 21900804.2
(22) Date of filing: 21.10.2021
(51) Int. Cl.: C07D 405/14, C07D 409/14, C07D 487/04, C07D 495/04, C07D 491/048, C07D 405/04, C07D 409/04, H01L 51/00, H01L 51/50

(54) **HETEROCYCLIC COMPOUND, ORGANIC LIGHT-EMITTING DEVICE COMPRISING SAME, COMPOSITION FOR ORGANIC LAYER OF ORGANIC LIGHT-EMITTING DEVICE, AND METHOD FOR MANUFACTURING ORGANIC LIGHT-EMITTING DEVICE**

(30) Priority: 01.12.2020 KR 20200165889
(71) Applicant: LT Materials Co., Ltd., Yongin-si, Gyeonggi-do 17118 (KR)
(72) Inventor: PARK, Geon-Yu, Yongin-si, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/014775
(87) International publication number: WO 2022/119116

(57) **Abstract**

The present specification provides a heterocyclic compound represented by Chemical Formula 1, an organic light emitting device including the same, a composition for an organic material layer of an organic light emitting device, and a method for manufacturing an organic light emitting device.

## Description

### [Technical Field]

This application claims priority to and the benefits of Korean Patent Application No. 10-2020-0165889, filed with the Korean Intellectual Property Office on December 1, 2020, the entire contents of which are incorporated herein by reference.

The present specification relates to a heterocyclic compound, an organic light emitting device including the same, a composition for an organic material layer of an organic light emitting device, and a method for manufacturing an organic light emitting device.

### [Background Art]

An organic electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device including a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent No. 4,356,429

### [Disclosure]

### [Technical Problem]

The present application relates to a heterocyclic compound, an organic light emitting device including the same, a composition for an organic material layer of an organic light emitting device, and a method for manufacturing an organic light emitting device.

### [Technical Solution]

One embodiment of the present application provides a heterocyclic compound represented by the following Chemical Formula 1.

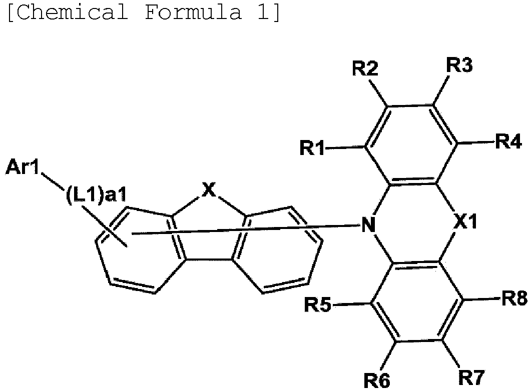

In Chemical Formula 1, substitution positions of substituents of -(L1)a1-Ar1 and 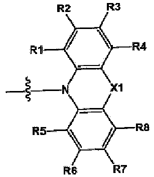 satisfy any one of the following Structural Formulae 1-A to 1-J, and in the following Structural Formulae 1-A to 1-J, * and ** mean positions linked to -(L1)a1-Ar1 and

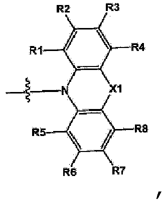

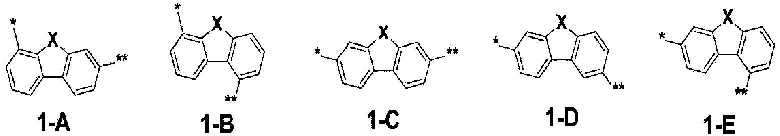

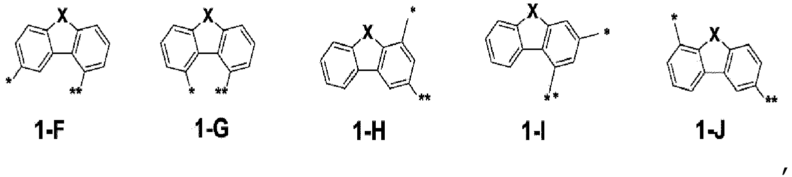

L1 is a direct bond; a substituted or unsubstituted C6 to C60 arylene group; or a substituted or unsubstituted C2 to C60 heteroarylene group,
X is O; or S,
X1 is a direct bond; O; S; NR; or CRR',
Ar1 is a C6 to C60 aryl group unsubstituted or substituted with deuterium, a C6 to C60 aryl group or a C1 to C60 alkyl group; or a C2 to C60 heteroaryl group unsubstituted or substituted with deuterium,
R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group including O or S as a heteroatom; -SiRR'R"; -P(=O)RR'; and - NRR', or two or more groups adjacent to each other bond to each other to form a substituted or unsubstituted C6 to C60 aromatic hydrocarbon ring or a substituted or unsubstituted C2 to C60 heteroring,
a1 is an integer of 1 to 4,
when, in the structural formulae of 1-B and 1-J, * is the linking position of 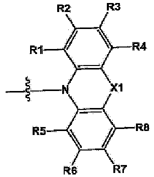 and ** is the linking position of -(L1)a1-Ar1, and R1 to R8 are all hydrogen or at least one of R1 to R8 has a phenyl group, Ar1 is a C6 to C60 aryl group unsubstituted or substituted with deuterium, a C6 to C60 aryl group or a C1 to C60 alkyl group,
when not expressed as substituents in Chemical Formula 1, they are hydrogen; or deuterium, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In addition, one embodiment of the present application provides an organic light emitting device including a first electrode; a second electrode provided opposite to the first electrode; and one or more organic material layers provided between the first electrode and the second electrode, wherein one or more layers of the organic material layers include one or more of the heterocyclic compound represented by Chemical Formula 1.

In addition, one embodiment of the present application provides a composition for an organic material layer of an organic light emitting device, the composition including the heterocyclic compound represented by Chemical Formula 1; and a heterocyclic compound represented by the following Chemical Formula 2 or a heterocyclic compound represented by the following Chemical Formula 3. 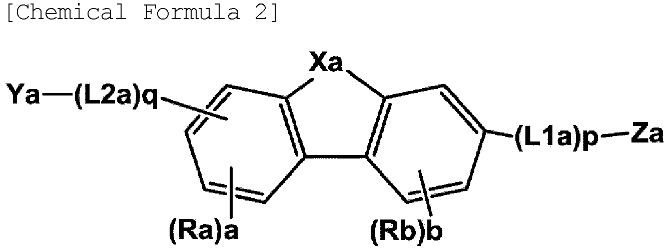 In Chemical Formula 2,
Xa is O or S,
Ya is a hole transferring group or a substituted or unsubstituted aryl group,
Za is an electron transferring group,
L1a and L2a are the same as or different from each other, and each independently a direct bond; a substituted or unsubstituted C6 to C60 arylene group; or a substituted or unsubstituted C2 to C60 heteroarylene group, p and q are each an integer of 0 to 3, when p is 2 or greater, L1as are the same as or different from each other, and when q is 2 or greater, L2as are the same as or different from each other, and
Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, a and b are each an integer of 0 to 3, when a is 2 or greater, Ras are the same as or different from each other, and when b is 2 or greater, Rbs are the same as or different from each other, 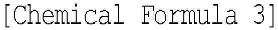 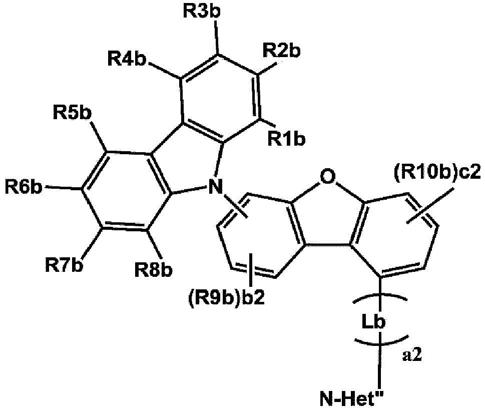 in Chemical Formula 3,
   N-Het" is a monocyclic or polycyclic heterocyclic group substituted or unsubstituted and including one or more Ns,
   Lb is a direct bond; a substituted or unsubstituted arylene group; or a substituted or unsubstituted heteroarylene group, a2 is an integer of 1 to 3, and when a2 is 2 or greater, Lbs are the same as or different from each other, and
   R1b to R10b are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, b2 and c2 are each an integer of 0 to 3, when b2 is 2 or greater, R9bs are the same as or different from each other, and when c2 is 2 or greater, R10bs are the same as or different from each other.

Lastly, one embodiment of the present application provides a method for manufacturing an organic light emitting device, the method including preparing a substrate; forming a first electrode on the substrate; forming one or more organic material layers on the first electrode; and forming a second electrode on the organic material layers, wherein the forming of organic material layers includes forming one or more organic material layers using the composition for an organic material layer according to one embodiment of the present application.

### [Advantageous Effects]

A compound described in the present specification can be used as a material of an organic material layer of an organic light emitting device. The compound is capable of performing a role of a hole injection material, a hole transfer material, a light emitting material, an electron transfer material, an electron injection material or the like in an organic light emitting device. Particularly, the compound can be used as a light emitting layer material of an organic light emitting device. For example, the compound can be used alone as a light emitting material, two of the compounds can be used together as a light emitting material, and may be used as a host material of a light emitting layer.

Particularly, the heterocyclic compound of Chemical Formula 1 has properties satisfying requirements required for green and blue devices, and when including the heterocyclic compound of Chemical Formula 1, a driving voltage is lowered, and excellent lifetime and efficiency are obtained.

In addition, when using the heterocyclic compound represented by Chemical Formula 1 and the heterocyclic compound represented by Chemical Formula 2 or the heterocyclic compound represented by Chemical Formula 3 in an organic light emitting device at the same time, a driving voltage of the device can be lowered, light efficiency can be enhanced, and lifetime properties of the device can be enhanced by thermal stability of the compound.

### [Description of Drawings]

FIG. 1 to FIG. 3 are diagrams each schematically illustrating a lamination structure of an organic light emitting device according to one embodiment of the present application.
FIG. 4 is a diagram explaining an exciplex phenomenon.
FIG. 5 is a diagram showing PL values of a P type host and an N type host of the present application.
FIG. 6 is a diagram showing a PL value when a P type host and an N type host of the present application are mixed.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium unlike a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 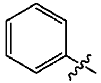 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

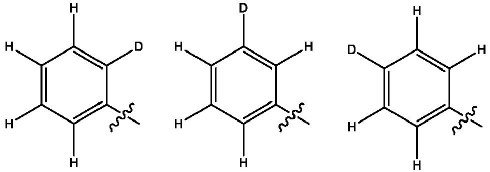

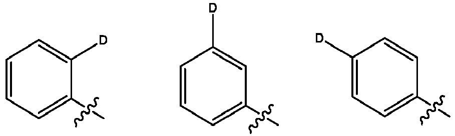

In addition, in one embodiment of the present application, "a phenyl group having a deuterium content of 0%" may mean a phenyl group that does not include a deuterium atom, that is, a phenyl group that has 5 hydrogen atoms.

In the present specification, the halogen may be fluorine, chlorine, bromine or iodine.

In the present specification, the alkyl group includes linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may include a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group includes linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may include a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group includes linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may include methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group includes monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may include a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group includes O, S, Se, N or Si as a heteroatom, includes monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group includes monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group includes a spiro group. The number of carbon atoms of the aryl group may be from 6 to 60, specifically from 6 to 40 and more specifically from 6 to 25. Specific examples of the aryl group may include a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the fluorenyl group may be substituted, and adjacent substituents may bond to each other to form a ring.

When the fluorenyl group is substituted, the following structures and the like may be included, however, the structure is not limited thereto.

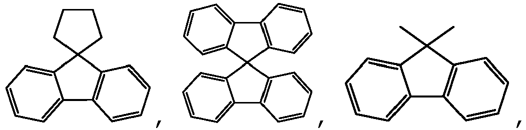

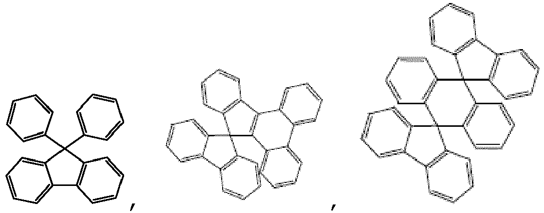

In the present specification, the heteroaryl group includes S, O, Se, N or Si as a heteroatom, includes monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may include a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may include a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P(=O)R101R102, and R101 and R102 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may include a diphenylphosphine oxide group, a dinaphthylphosphine oxide group and the like, but are not limited thereto.

In the present specification, the silyl group is a substituent including Si, having the Si atom directly linked as a radical, and is represented by -SiR104R105R106. R104 to R106 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent is capable of substituting, and when two or more substituents substitute, the two or more substituents may be the same as or different from each other.

In the present specification, "substituted or unsubstituted" means being substituted with one or more substituents selected from the group consisting of deuterium; a cyano group; a halogen group; C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine or being unsubstituted, or being substituted with a substituent linking two or more substituents selected from among the substituents illustrated above or being unsubstituted.

In one embodiment of the present application, substitution positions of substituents of -(L1)a1-Ar1 and 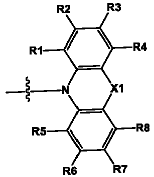 satisfy any one of the following Structural Formulae 1-A to 1-J, and in the following Structural Formulae 1-A to 1-J, * and ** may mean positions linked to -(L1)a1-Ar1 and

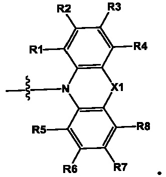

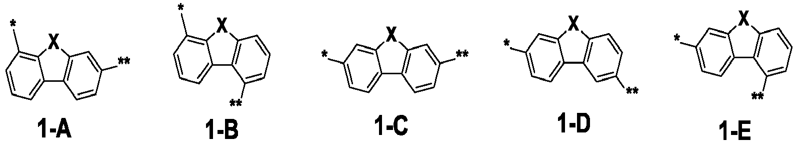

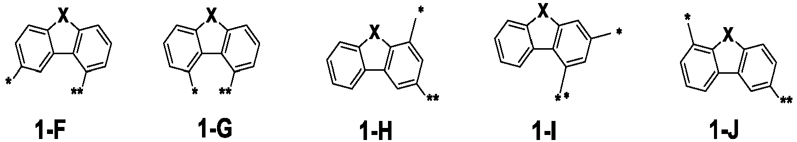

In one embodiment of the present application, R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and each independently a substituted or unsubstituted C1 to C40 alkyl group; a substituted or unsubstituted C6 to C40 aryl group; or a substituted or unsubstituted C2 to C40 heteroaryl group.

In another embodiment, R, R' and R" are the same as or different from each other, and each independently a substituted or unsubstituted C1 to C40 alkyl group; or a substituted or unsubstituted C6 to C40 aryl group.

In another embodiment, R, R' and R" are the same as or different from each other, and each independently a substituted or unsubstituted C1 to C20 alkyl group; or a substituted or unsubstituted C6 to C20 aryl group.

In another embodiment, R, R' and R" are the same as or different from each other, and each independently a C1 to C20 alkyl group; or a C6 to C20 aryl group.

In another embodiment, R, R' and R" are the same as or different from each other, and each independently a C1 to C10 alkyl group; or a C6 to C10 aryl group.

In another embodiment, R, R' and R" are the same as or different from each other, and each independently a linear C1 to C10 alkyl group; or a monocyclic C6 to C10 aryl group.

In another embodiment, R, R' and R" are the same as or different from each other, and each independently a methyl group; or a phenyl group.

In one embodiment of the present application, L1 may be a direct bond; a substituted or unsubstituted C6 to C60 arylene group; or a substituted or unsubstituted C2 to C60 heteroarylene group.

In another embodiment, L1 may be a direct bond; or a substituted or unsubstituted C6 to C60 arylene group.

In another embodiment, L1 may be a direct bond; or a substituted or unsubstituted C6 to C40 arylene group.

In another embodiment, L1 may be a direct bond; or a C6 to C40 arylene group.

In another embodiment, L1 may be a direct bond; or a C6 to C20 arylene group.

In another embodiment, L1 may be a direct bond; a phenylene group; or a biphenylene group.

In one embodiment of the present application, Ar1 may be a C6 to C60 aryl group unsubstituted or substituted with a C6 to C60 aryl group or a C1 to C60 alkyl group; or a C2 to C60 heteroaryl group unsubstituted or substituted with deuterium.

In another embodiment, Ar1 may be a C6 to C40 aryl group unsubstituted or substituted with a C6 to C40 aryl group or a C1 to C40 alkyl group; or a C2 to C40 heteroaryl group unsubstituted or substituted with deuterium.

In another embodiment, Ar1 may be a C6 to C40 aryl group unsubstituted or substituted with a C6 to C40 aryl group or a C1 to C40 alkyl group; or a C2 to C40 heteroaryl group including O or S as a heteroatom unsubstituted or substituted with deuterium.

In another embodiment, Ar1 may be a C6 to C30 aryl group unsubstituted or substituted with a C6 to C20 aryl group or a C1 to C20 alkyl group; or a C2 to C30 heteroaryl group including O or S as a heteroatom unsubstituted or substituted with deuterium.

In another embodiment, Ar1 may be a dibenzofuran group unsubstituted or substituted with deuterium; a dibenzothiophene group unsubstituted or substituted with deuterium; a phenyl group; a biphenyl group unsubstituted or substituted with a phenyl group; a terphenyl group; a dimethylfluorenyl group; a diphenylfluorenyl group; or a spirobifluorenyl group.

In another embodiment, -(L1)a1-Ar1 may be represented by any one of the following Chemical Formulae 4 to 6.

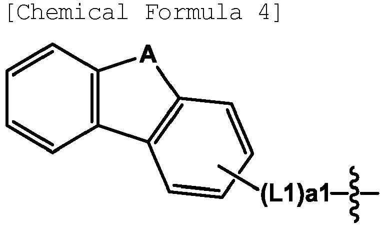

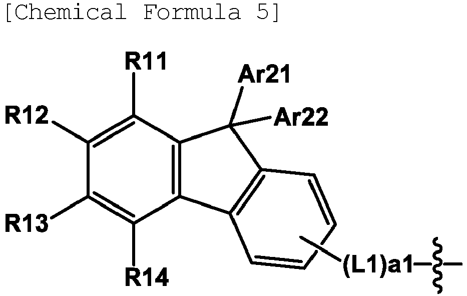

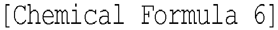

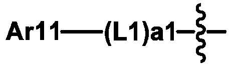

In Chemical Formulae 4 to 6,
L1 and a1 have the same definitions as in Chemical Formula 1,
A is O; or S,
R11 to R14 are the same as or different from each other, and each independently hydrogen; deuterium; a C6 to C60 aryl group; or a C1 to C60 alkyl group,
Ar21 and Ar22 are the same as or different from each other, and each independently a C1 to C60 alkyl group; or a C6 to C60 aryl group, or two groups adjacent to each other bond to each other to form a C6 to C60 aromatic hydrocarbon ring, and
Ar11 is a C6 to C60 aryl group.

In one embodiment of the present application, R11 to R14 are the same as or different from each other, and may be each independently hydrogen; deuterium; a C6 to C60 aryl group; or a C1 to C60 alkyl group.

In another embodiment, R11 to R14 are the same as or different from each other, and may be each independently hydrogen; deuterium; a C6 to C40 aryl group; or a C1 to C40 alkyl group.

In another embodiment, R11 to R14 are the same as or different from each other, and may be each independently hydrogen; deuterium; a C6 to C20 aryl group; or a C1 to C20 alkyl group.

In another embodiment, R11 to R14 may be hydrogen or deuterium.

In one embodiment of the present application, Ar21 and Ar22 are the same as or different from each other, and each independently a C1 to C60 alkyl group; or a C6 to C60 aryl group, or two groups adjacent to each other may bond to each other to form a substituted or unsubstituted C6 to C60 aromatic hydrocarbon ring.

In another embodiment, Ar21 and Ar22 are the same as or different from each other, and each independently a C1 to C40 alkyl group; or a C6 to C40 aryl group, or two groups adjacent to each other may bond to each other to form a C6 to C40 aromatic hydrocarbon ring.

In another embodiment, Ar21 and Ar22 are the same as or different from each other, and each independently a C1 to C20 alkyl group; or a C6 to C20 aryl group, or two groups adjacent to each other may bond to each other to form a C6 to C20 aromatic hydrocarbon ring.

In another embodiment, Ar21 and Ar22 are the same as or different from each other, and each independently a methyl group; or a phenyl group, or two groups adjacent to each other may bond to each other to form a fluorenyl ring.

In one embodiment of the present application, Ar11 is a C6 to C60 aryl group.

In another embodiment, Ar11 is a C6 to C40 aryl group.

In another embodiment, Ar11 may be a monocyclic C6 to C10 aryl group; or a polycyclic C10 to C40 aryl group.

In another embodiment, Ar11 may be a phenyl group; a biphenyl group; or a terphenyl group.

In one embodiment of the present application, R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group including O or S as a heteroatom; -P(=O)RR'; and -NRR', or two or more groups adjacent to each other may bond to each other to form a substituted or unsubstituted C6 to C60 aromatic hydrocarbon ring or a substituted or unsubstituted C2 to C60 heteroring.

In another embodiment, R1 to R8 are the same as or different from each other, and each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group including O or S as a heteroatom; -P(=O)RR'; and -NRR', or two or more groups adjacent to each other may bond to each other to form a substituted or unsubstituted C6 to C60 aromatic hydrocarbon ring or a substituted or unsubstituted C2 to C60 heteroring.

In another embodiment, R1 to R8 are the same as or different from each other, and each independently selected from the group consisting of hydrogen; deuterium; a substituted or unsubstituted C6 to C40 aryl group; and a substituted or unsubstituted C2 to C40 heteroaryl group including O or S as a heteroatom, or two or more groups adjacent to each other may bond to each other to form a substituted or unsubstituted C6 to C40 aromatic hydrocarbon ring or a substituted or unsubstituted C2 to C40 heteroring.

In another embodiment, R1 to R8 are the same as or different from each other, and each independently selected from the group consisting of hydrogen; deuterium; a C6 to C40 aryl group unsubstituted or substituted with a C1 to C40 alkyl group; and a C2 to C40 heteroaryl group including O or S as a heteroatom, or two or more groups adjacent to each other may bond to each other to form a C6 to C40 aromatic hydrocarbon ring unsubstituted or substituted with a C1 to C40 alkyl group or a C2 to C40 heteroring unsubstituted or substituted with a C2 to C40 heteroaryl group.

In another embodiment, R1 to R8 are the same as or different from each other, and each independently hydrogen; deuterium; a dibenzofuran group; a dibenzothiophene group; a dimethylfluorenyl group; or a triphenylenyl group, or two or more groups adjacent to each other may bond to each other to form an indole ring unsubstituted or substituted with a phenyl group; a benzothiophene ring; a benzofuran ring; or an indene ring unsubstituted or substituted with a methyl group.

Particularly, in the structural formulae of 1-B and 1-J, when * is the linking position of 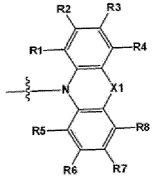 and ** is the linking position of -(L1)a1-Ar1, at least one of R1 to R8 is selected from the group consisting of a substituted or unsubstituted C6 to C60 aryl group; a substituted or unsubstituted C2 to C60 heteroaryl group including O or S as a heteroatom; -P(=O)RR'; and -NRR', or two or more groups adjacent to each other may bond to each other to form a substituted or unsubstituted C6 to C60 aromatic hydrocarbon ring or a substituted or unsubstituted C2 to C60 heteroring.

In the structural formulae of 1-B and 1-J, when * is the linking position of 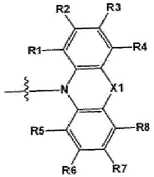 and ** is the linking position of -(L1)a1-Ar1, at least one of R1 to R8 has a substituent.

In addition, in the structural formulae of 1-B and 1-J, when * is the linking position of 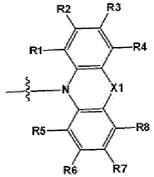 and ** is the linking position of -(L1)a1-Ar1, and R1 to R8 are all hydrogen or at least one of R1 to R8 has a phenyl group, Ar1 may be a C6 to C60 aryl group unsubstituted or substituted with deuterium, a C6 to C60 aryl group or a C1 to C60 alkyl group.

In one embodiment of the present application, Chemical Formula 4 may be represented by any one of the following Chemical Formulae 4-1 to 4-4.

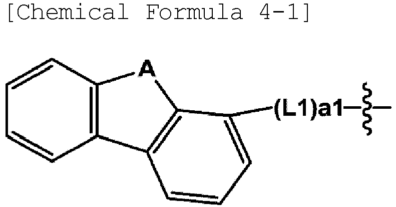

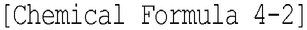

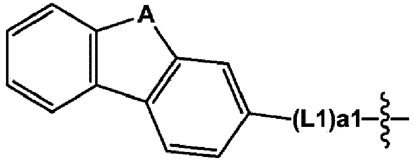

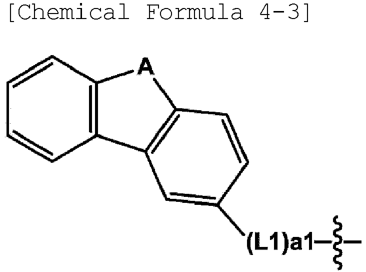

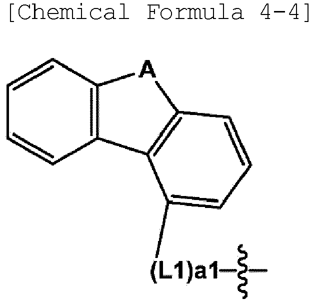

In Chemical Formulae 4-1 to 4-4,
L1, a1 and A have the same definitions as in Chemical Formula 4.

In one embodiment of the present application, Chemical Formula 5 may be represented by the following Chemical Formula 5-1 or 5-2.

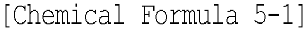

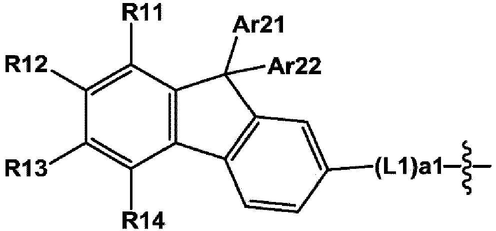

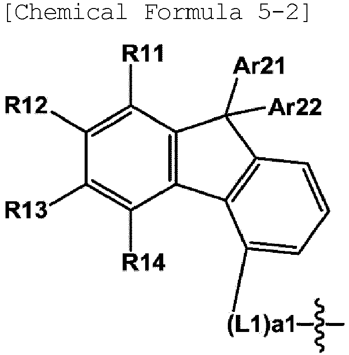

In Chemical Formulae 5-1 and 5-2,
each substituent has the same definition as in Chemical Formula 5.

According to one embodiment of the present application, Chemical Formula 1 may be represented by any one of the following compounds, but is not limited thereto.

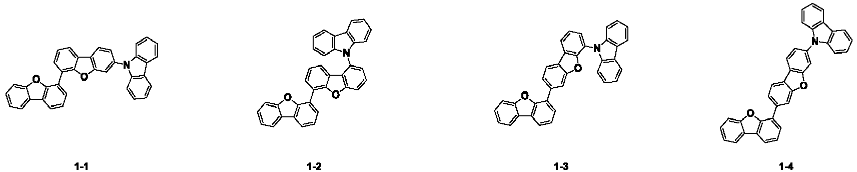

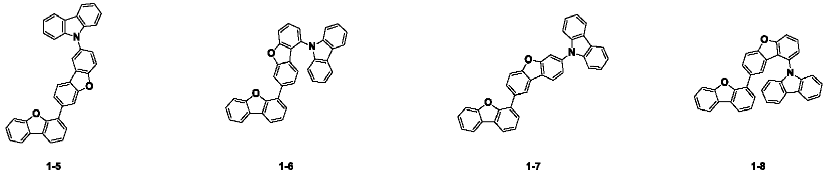

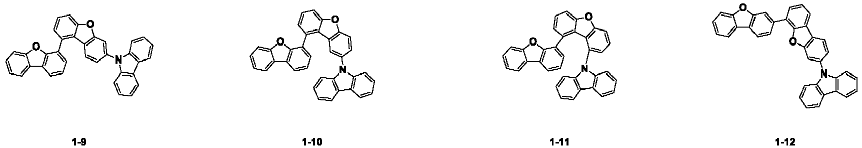

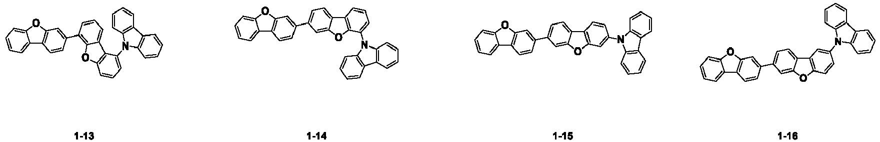

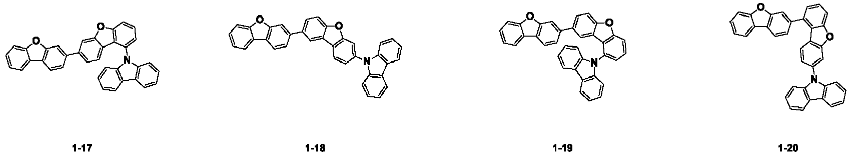

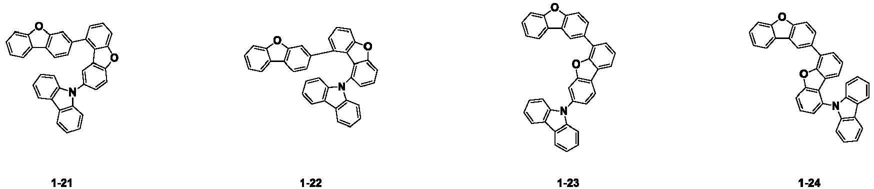

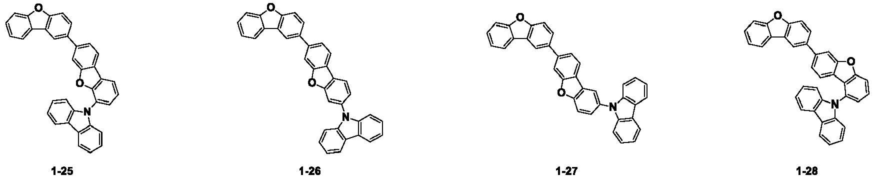

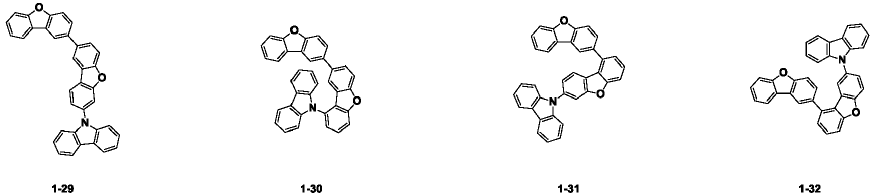

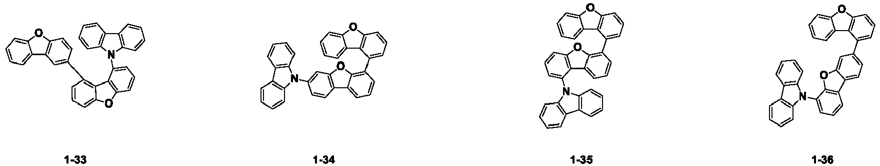

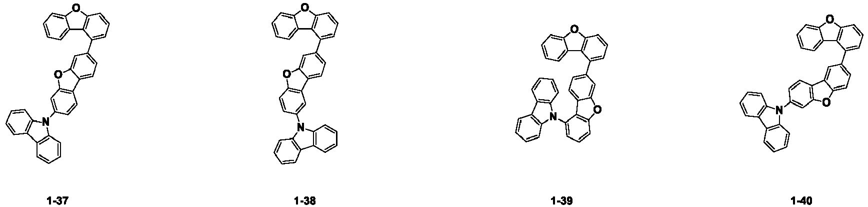

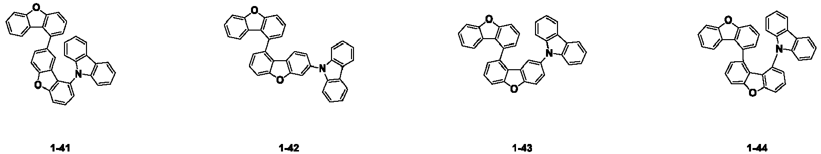

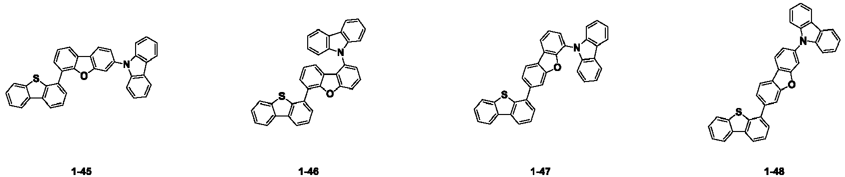

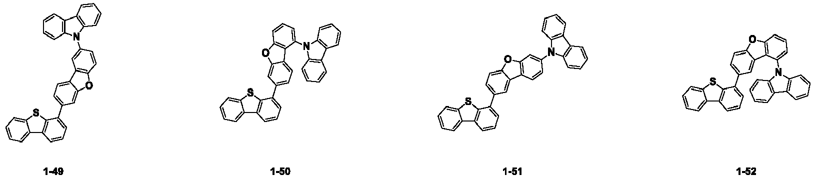

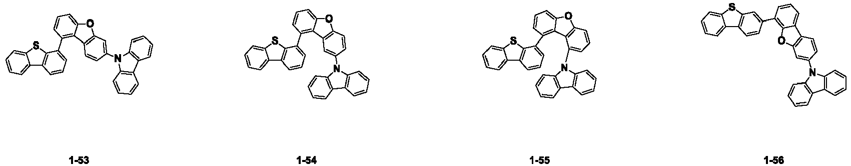

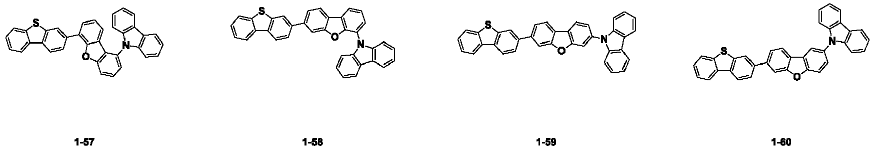

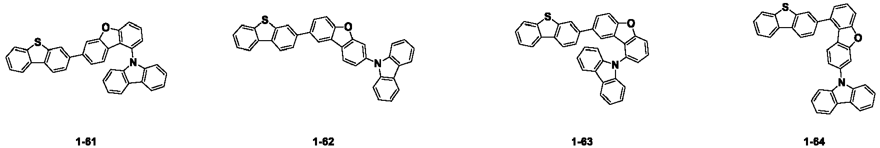

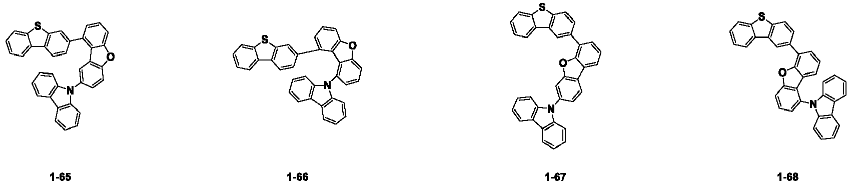

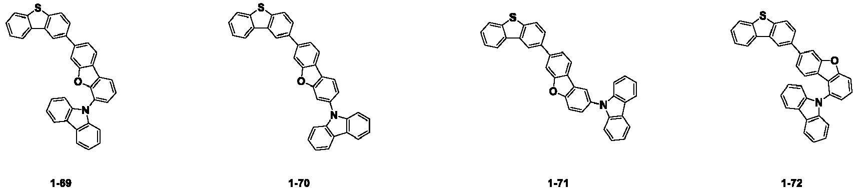

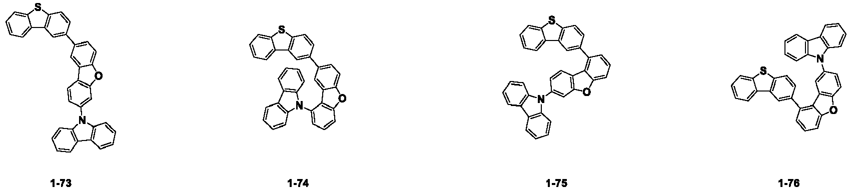

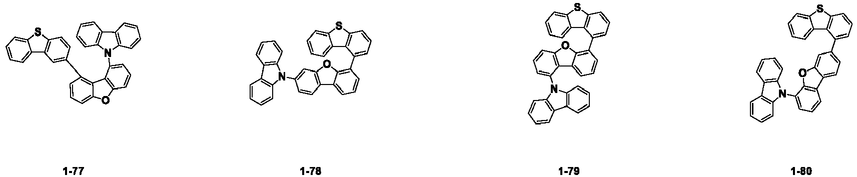

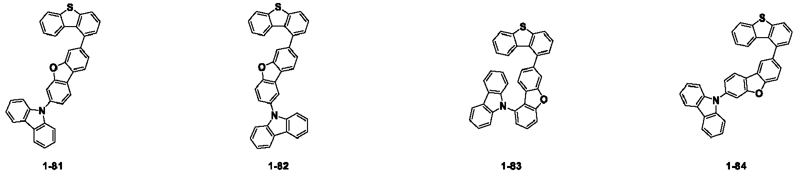

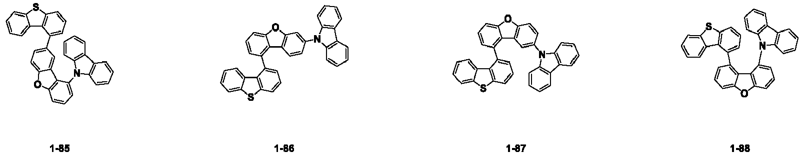

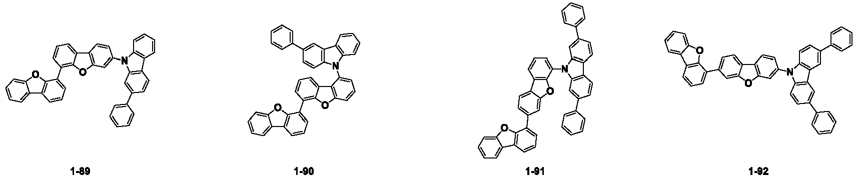

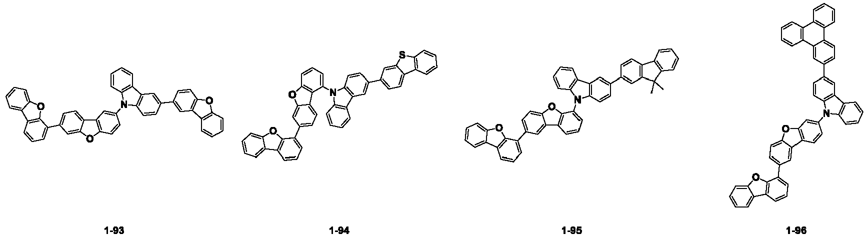

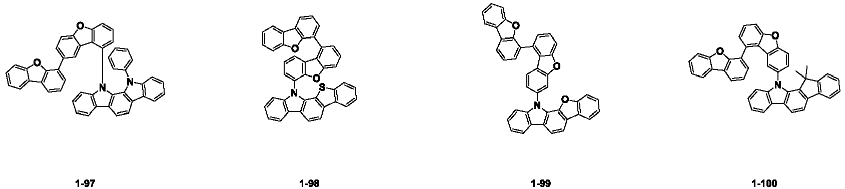

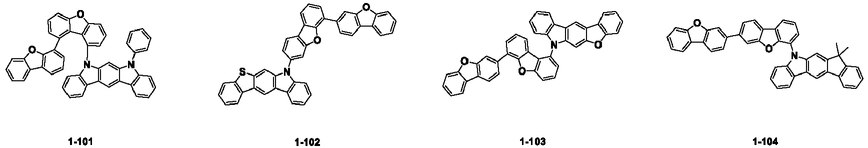

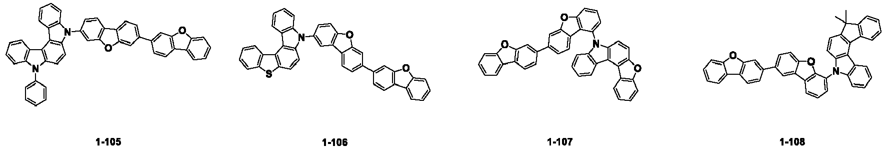

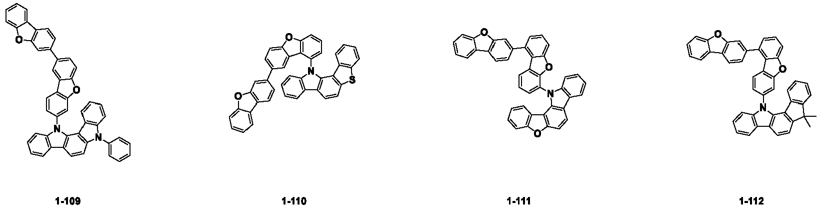

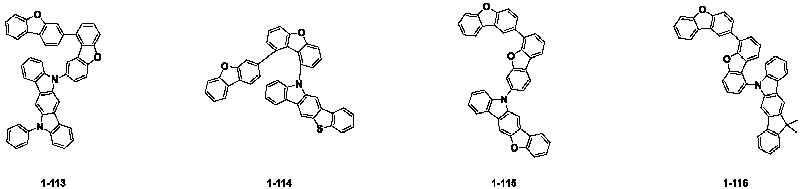

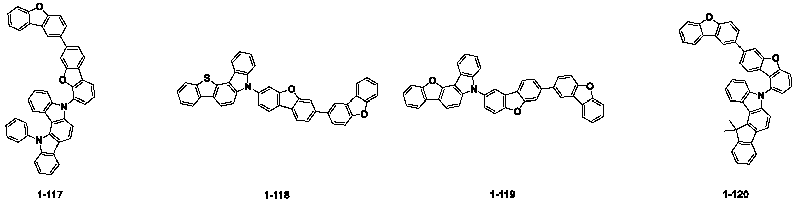

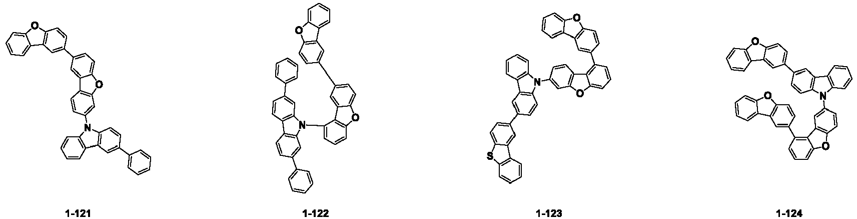

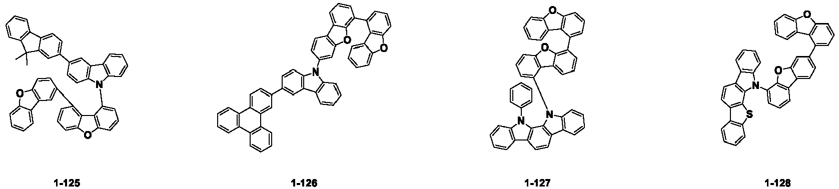

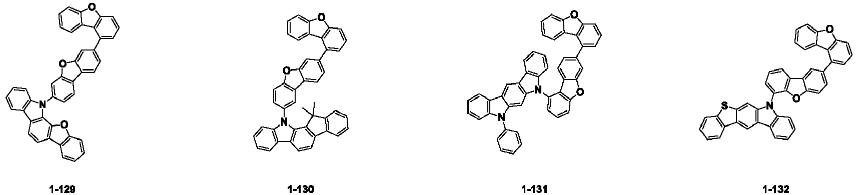

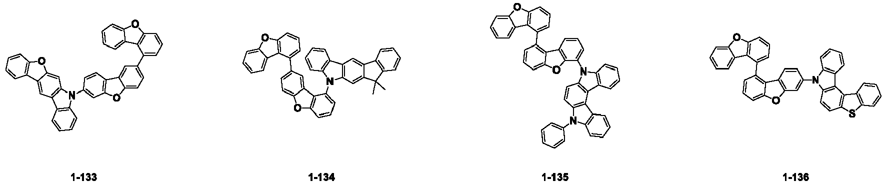

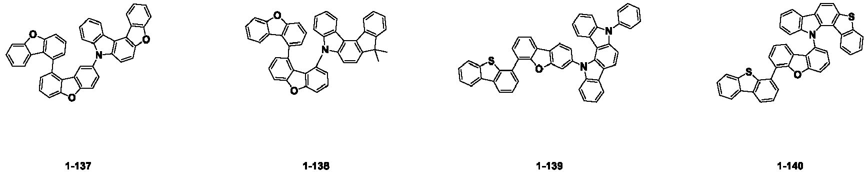

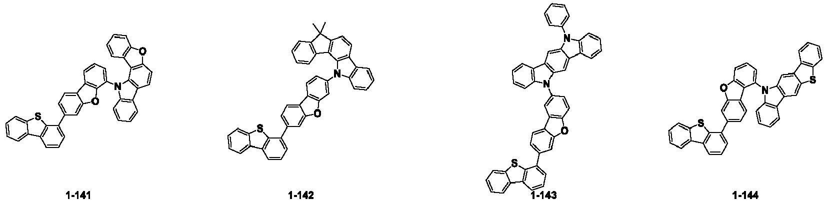

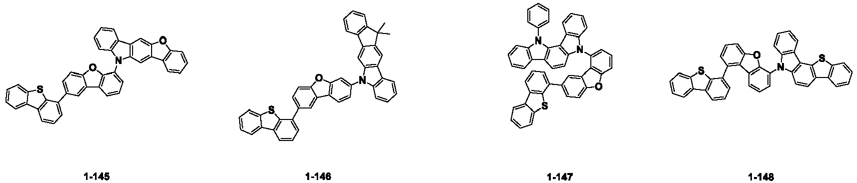

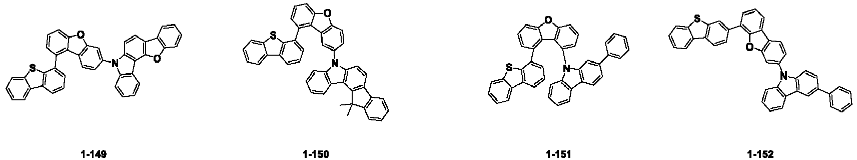

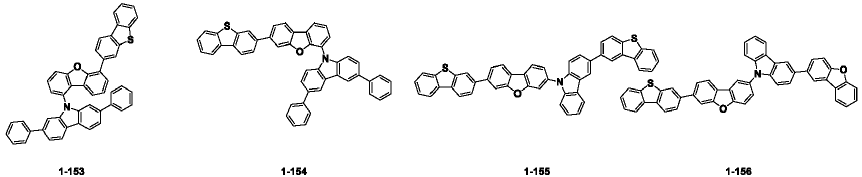

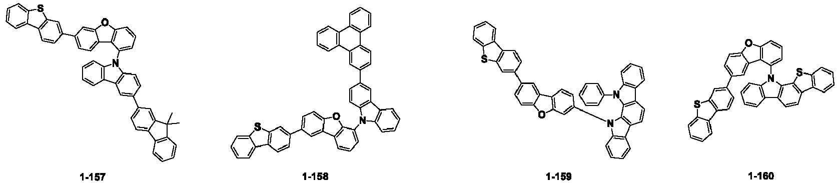

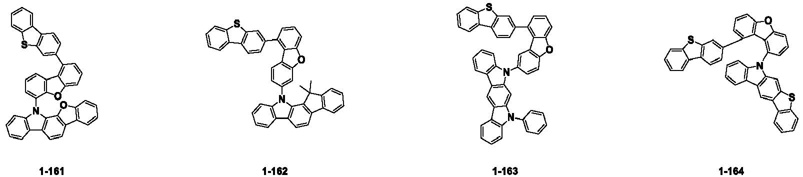

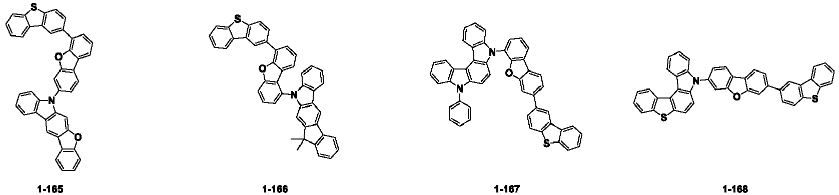

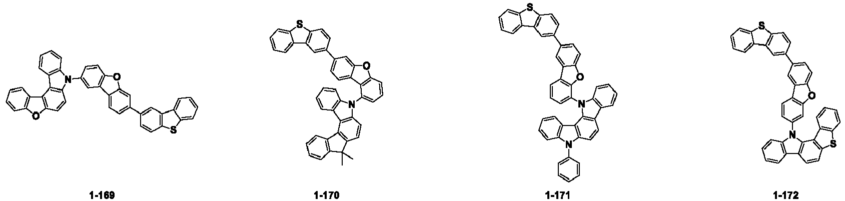

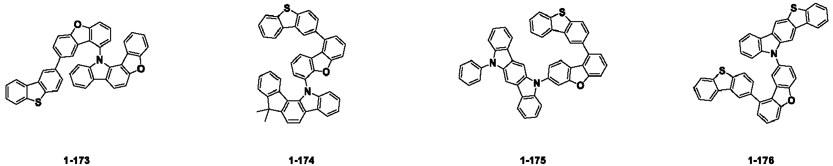

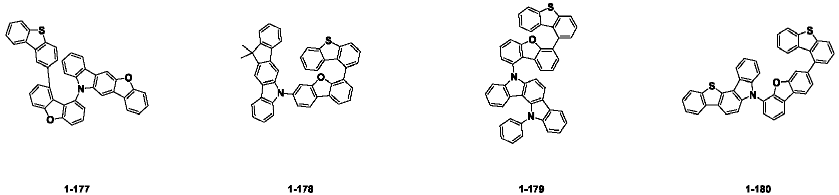

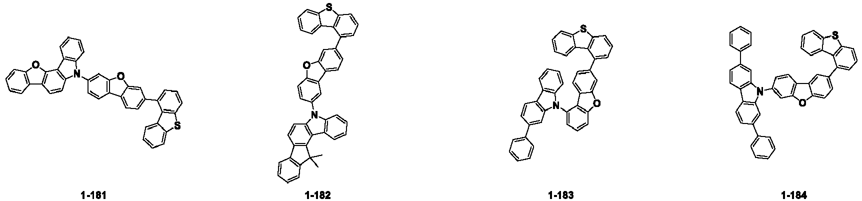

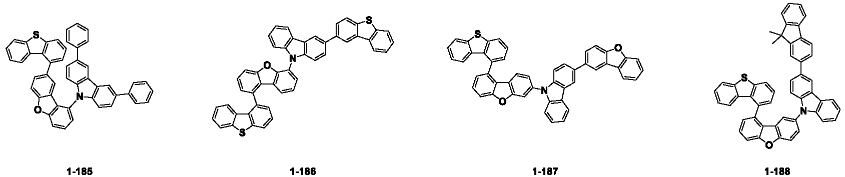

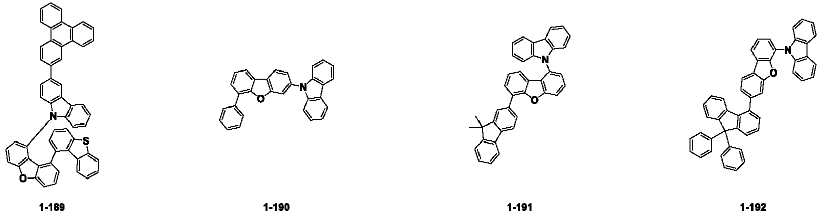

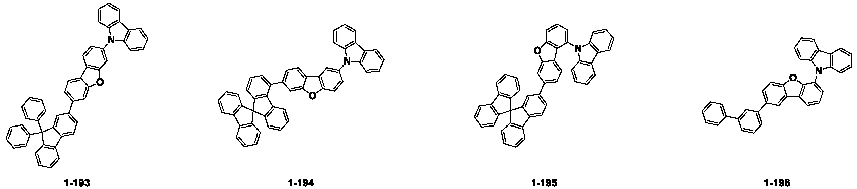

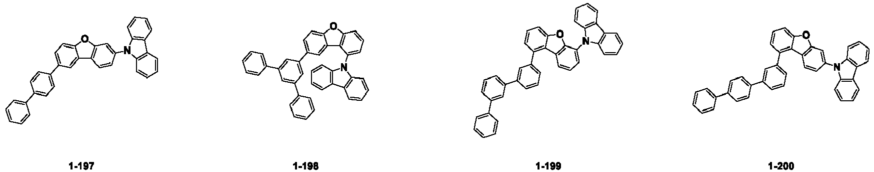

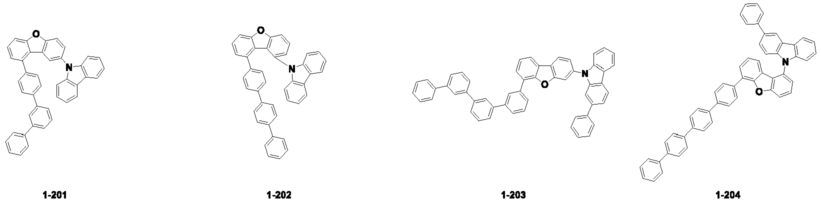

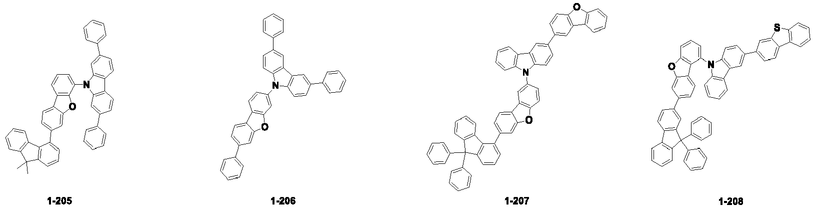

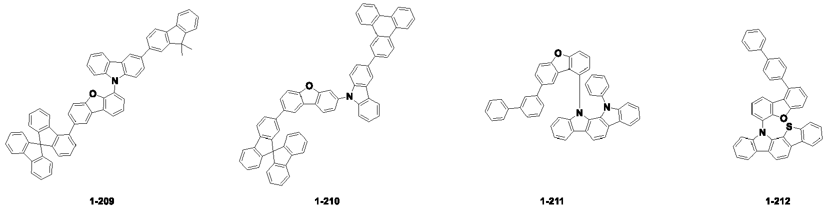

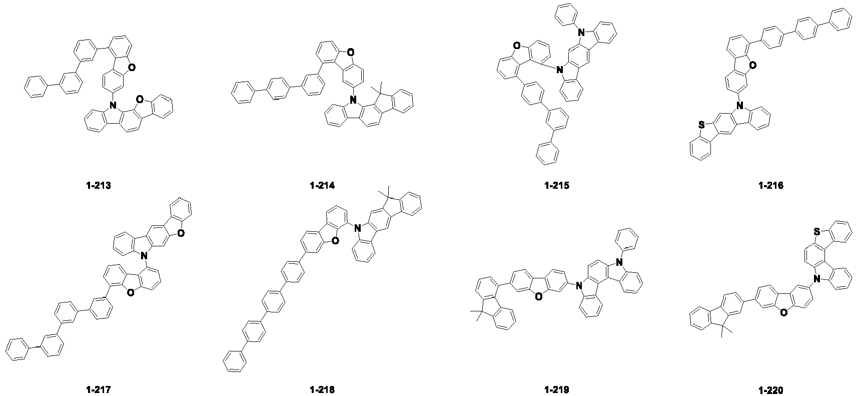

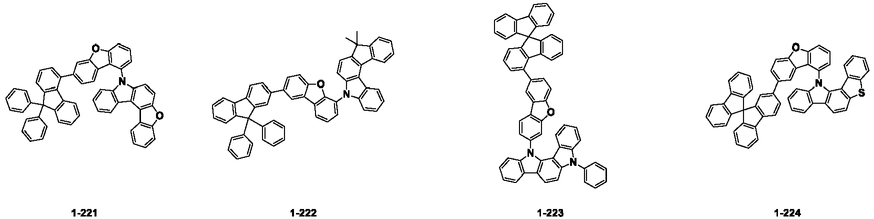

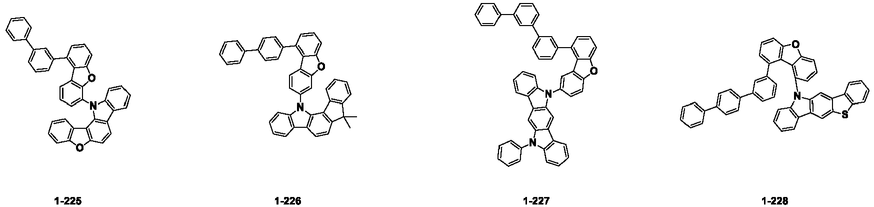

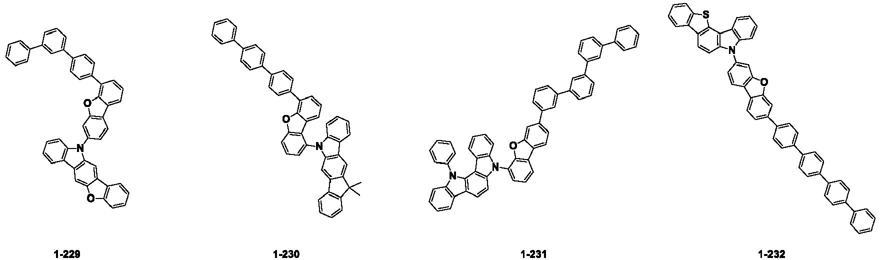

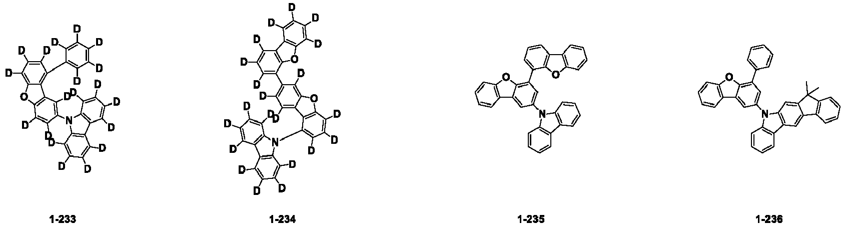

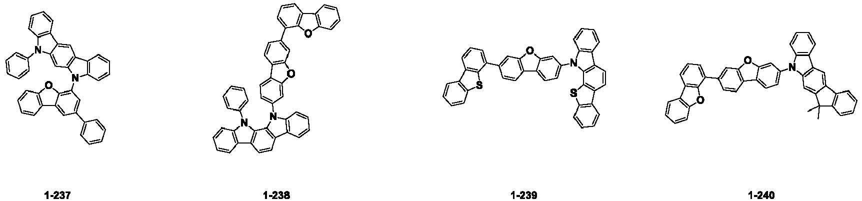

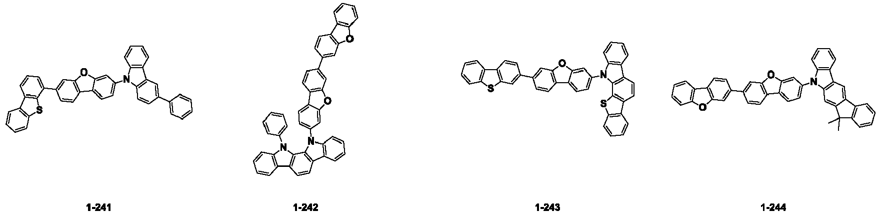

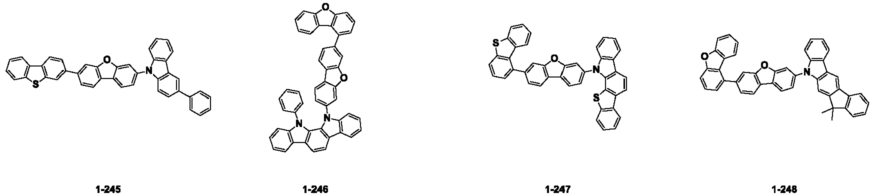

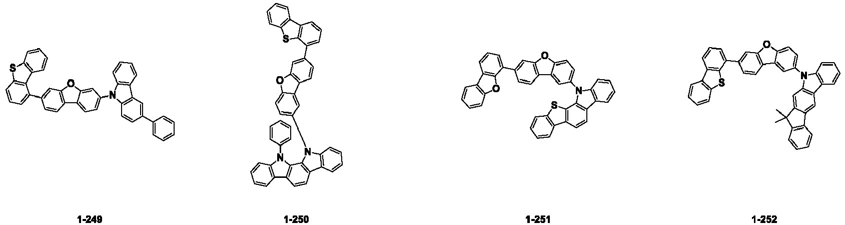

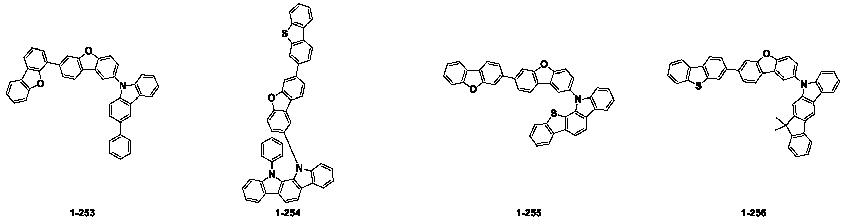

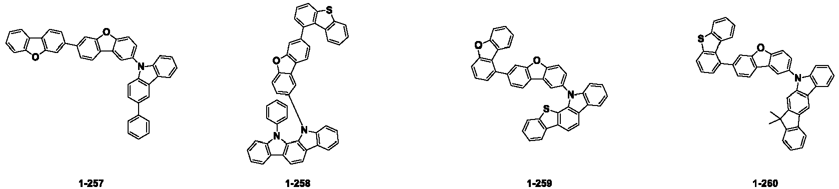

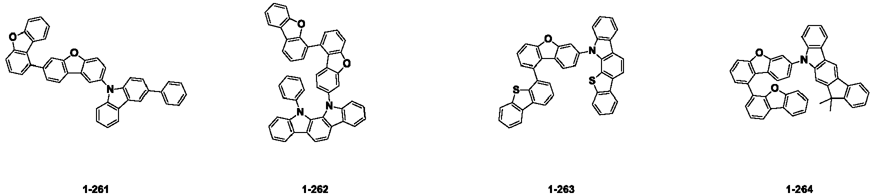

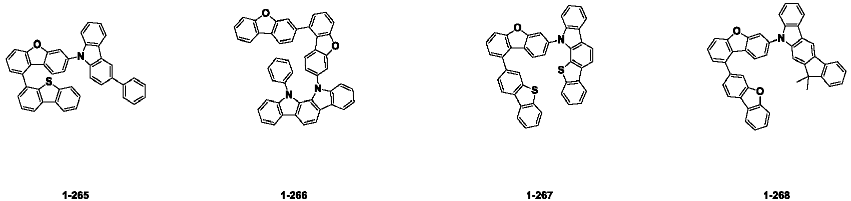

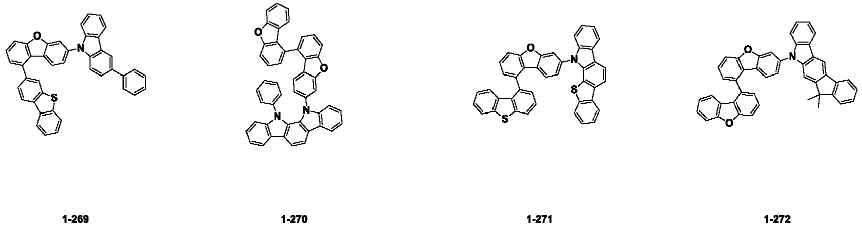

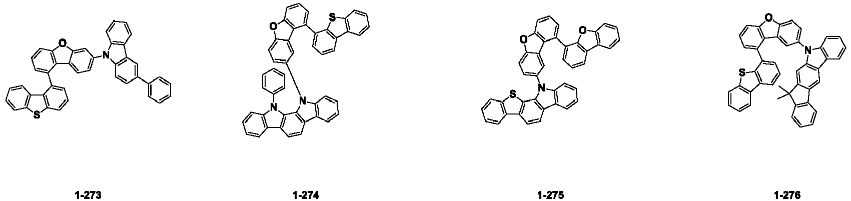

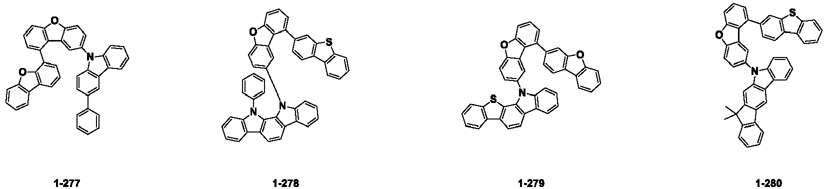

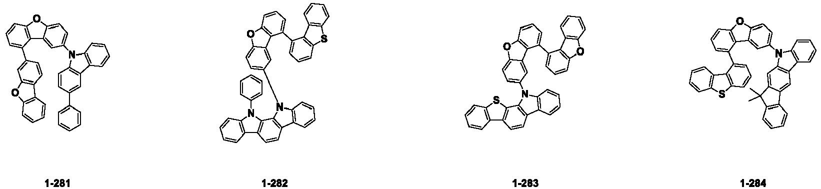

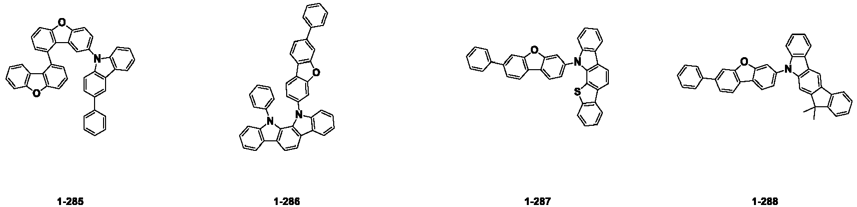

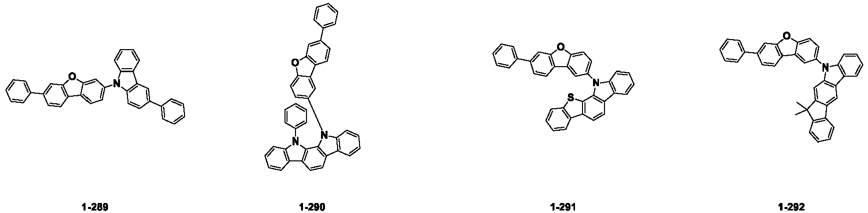

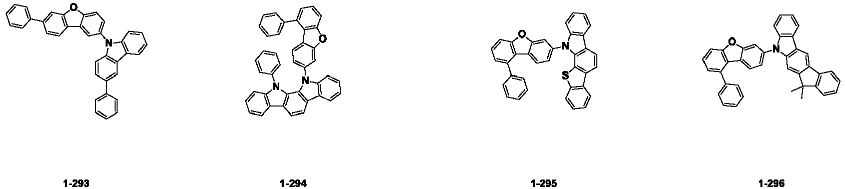

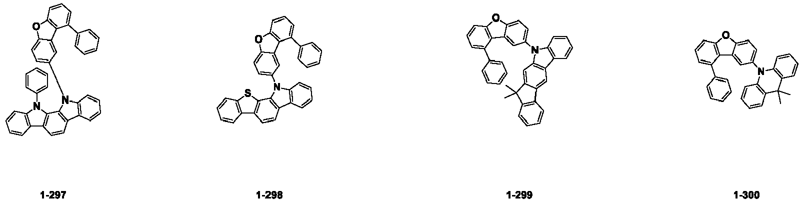

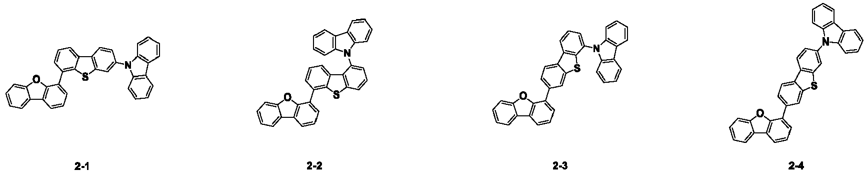

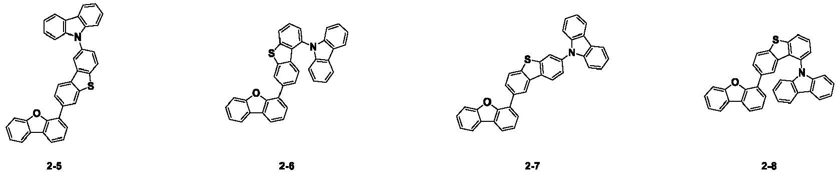

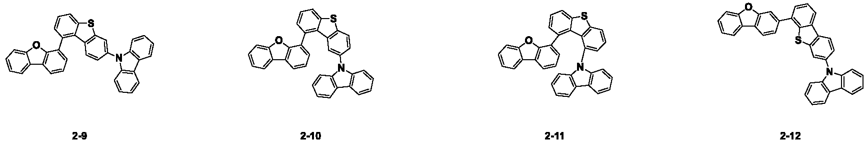

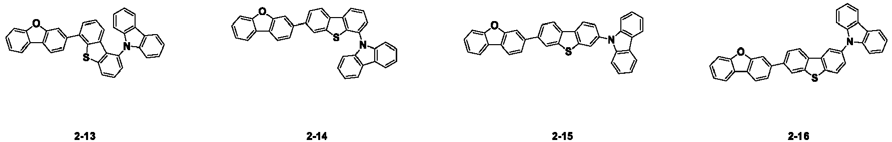

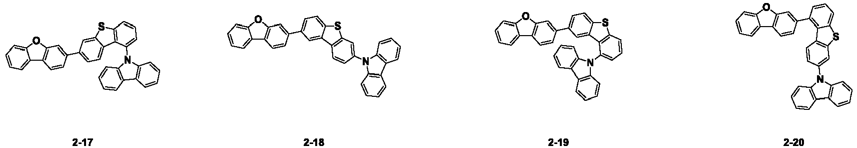

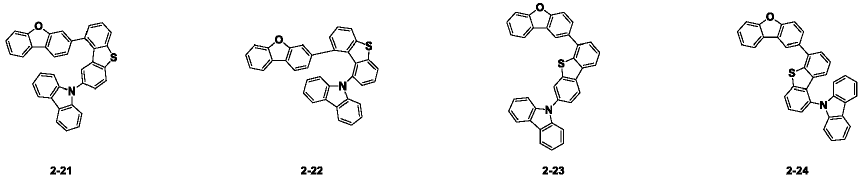

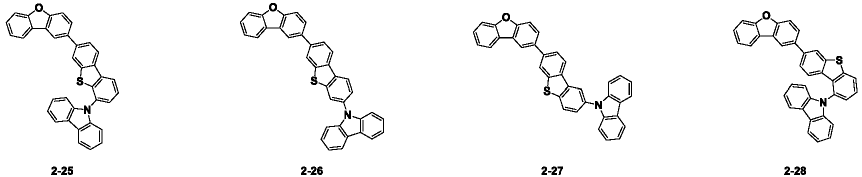

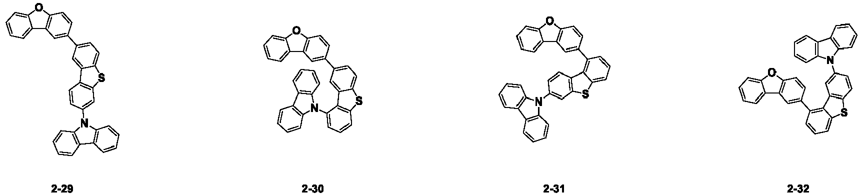

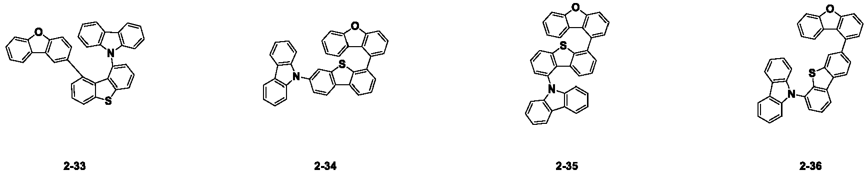

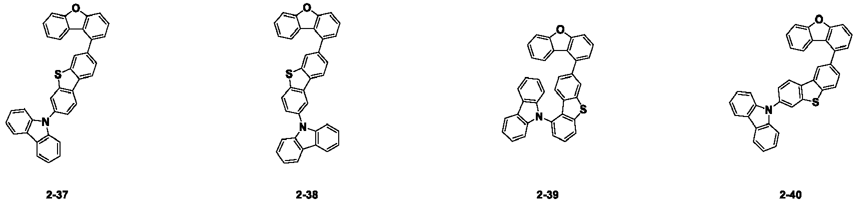

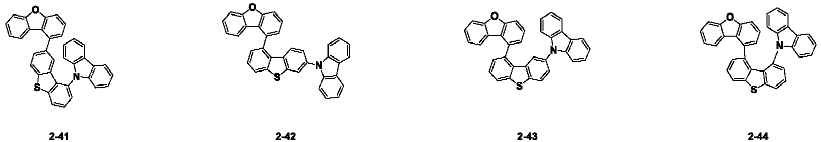

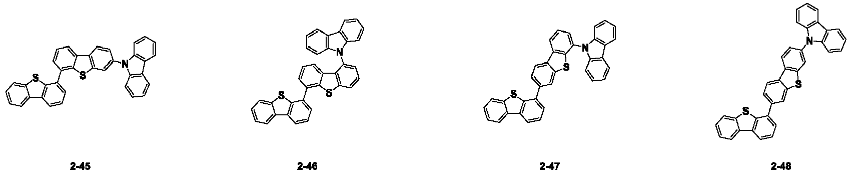

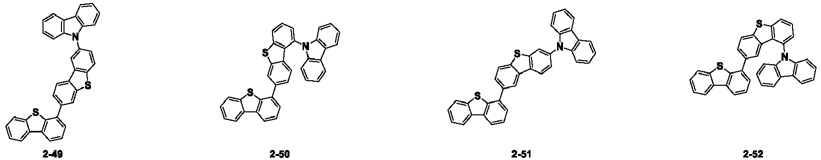

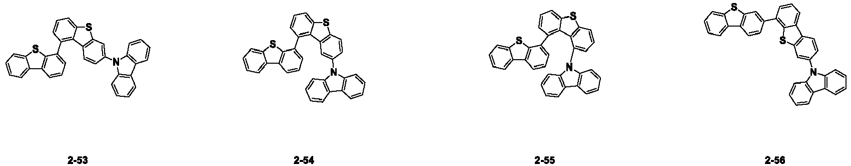

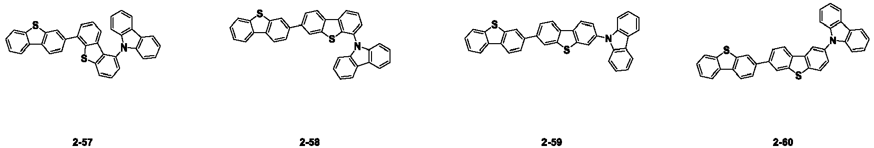

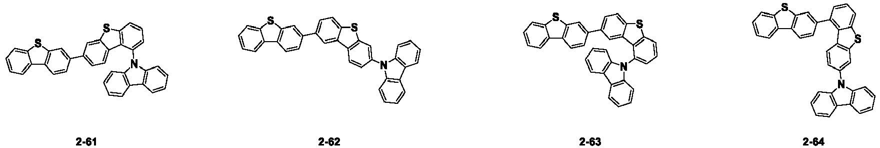

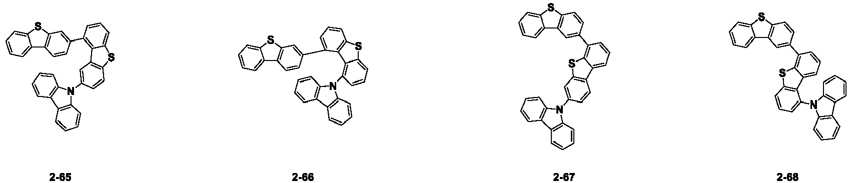

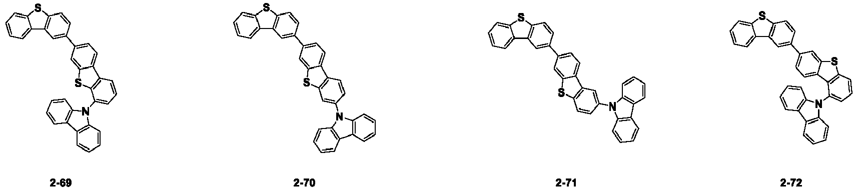

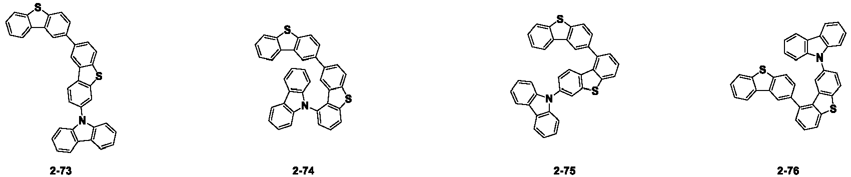

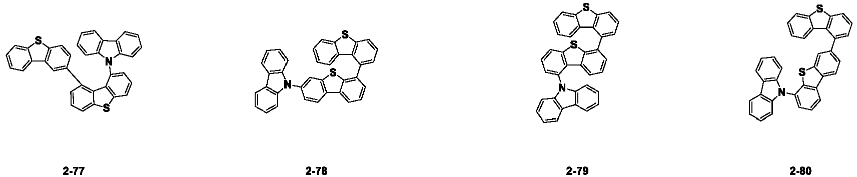

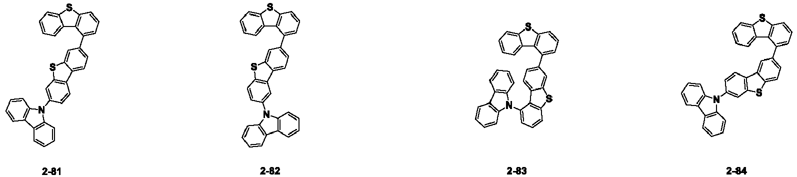

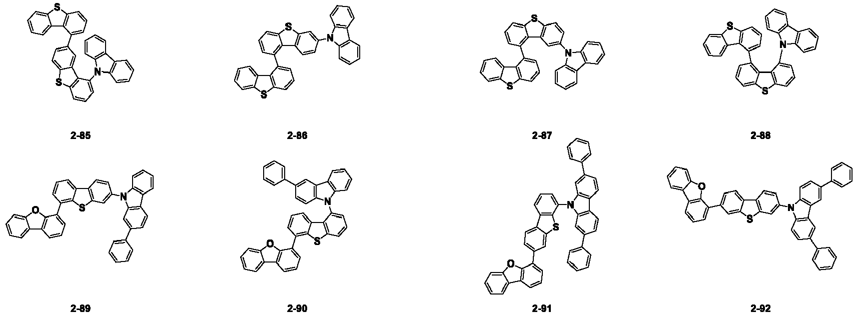

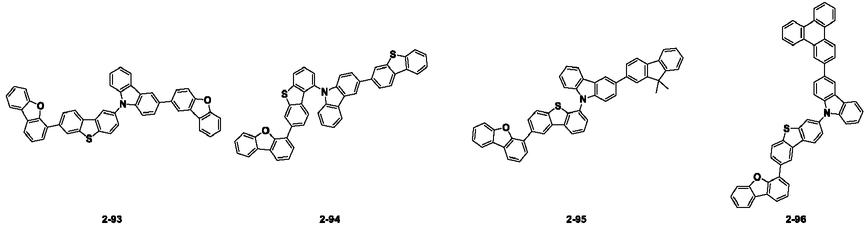

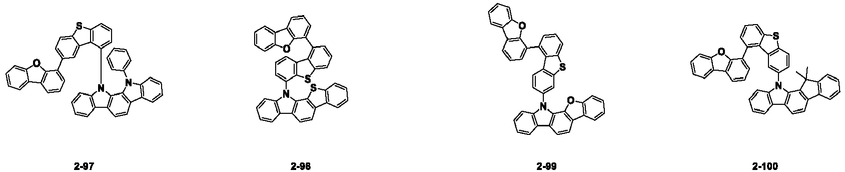

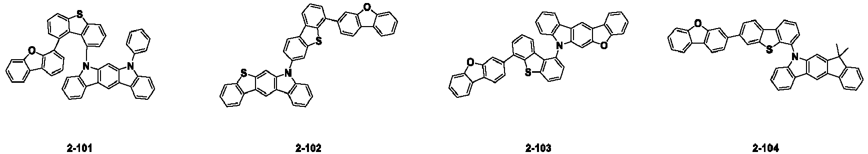

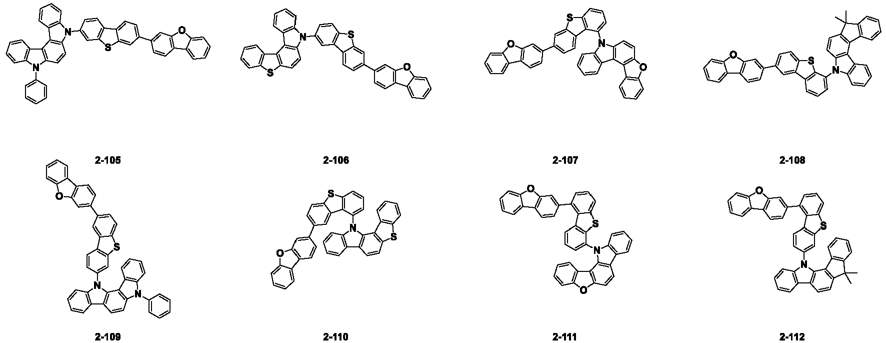

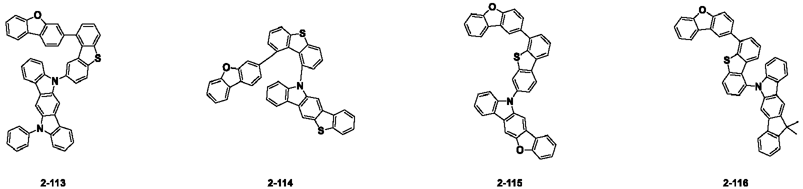

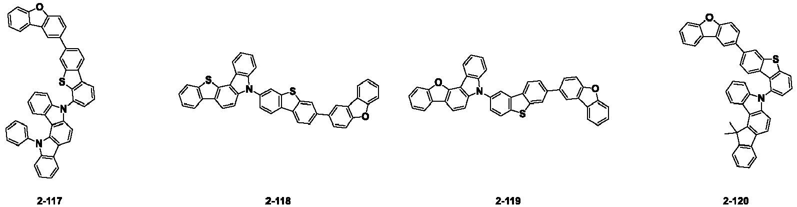

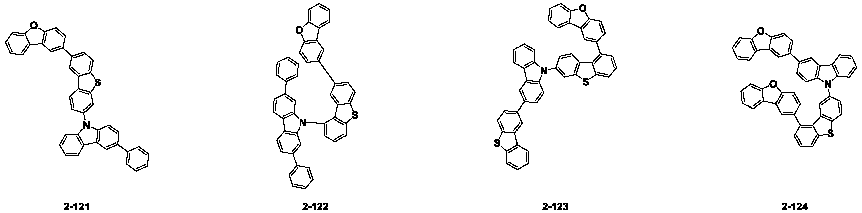

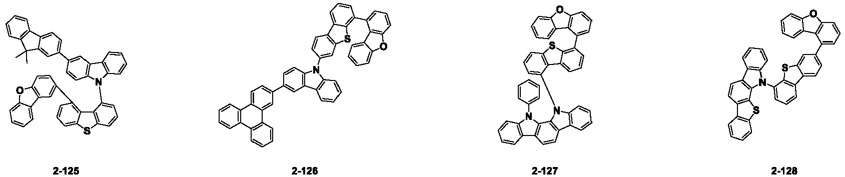

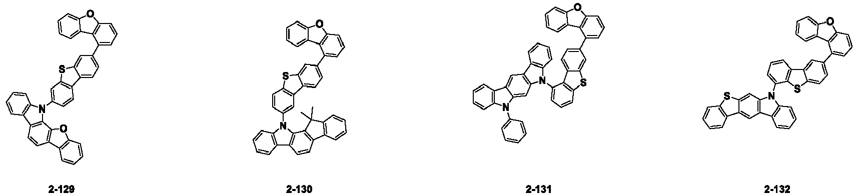

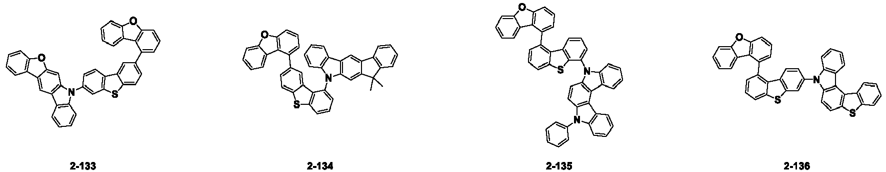

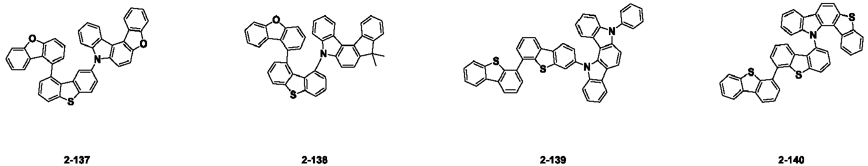

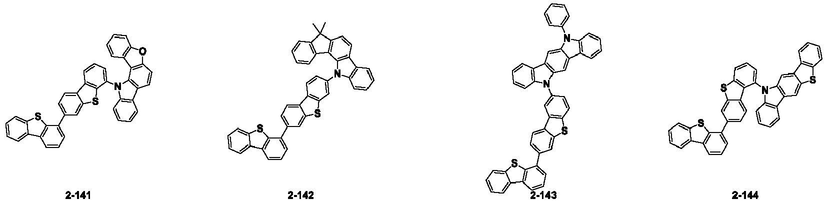

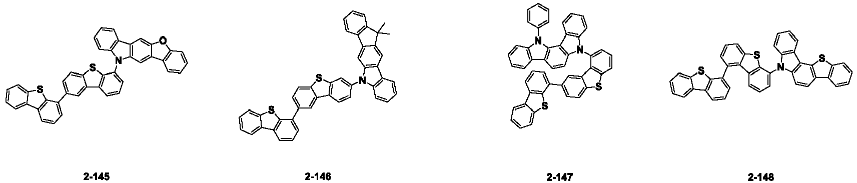

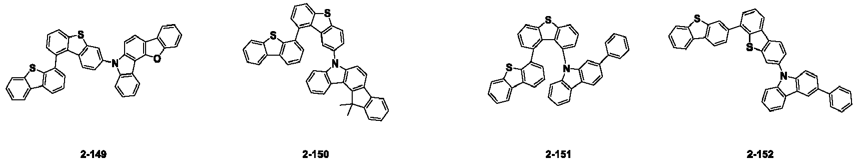

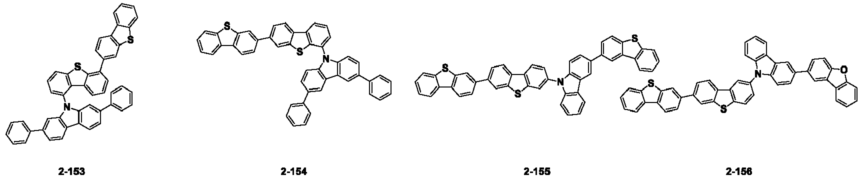

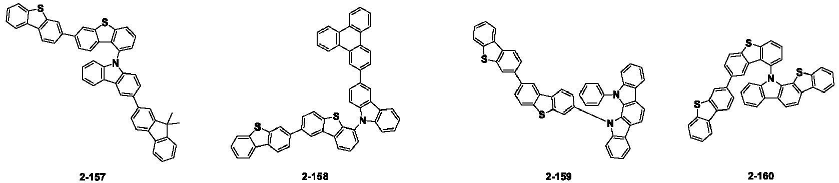

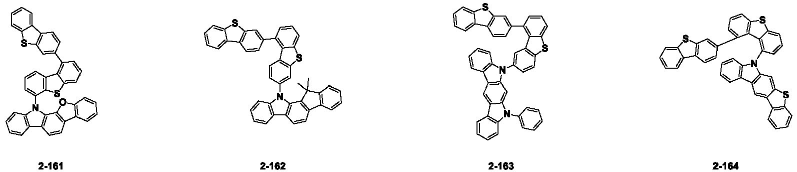

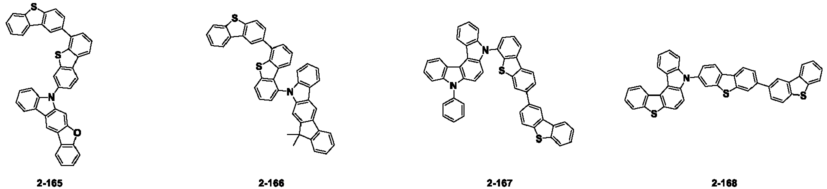

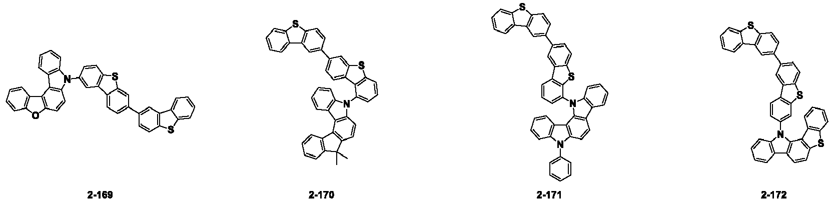

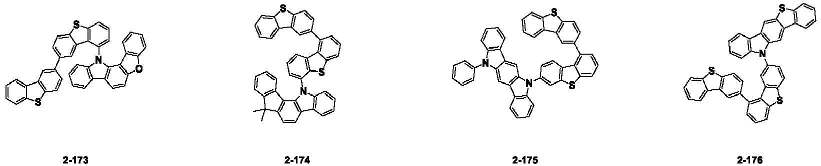

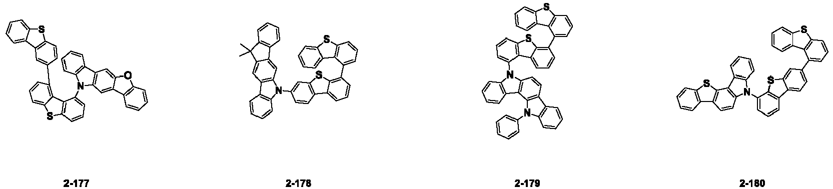

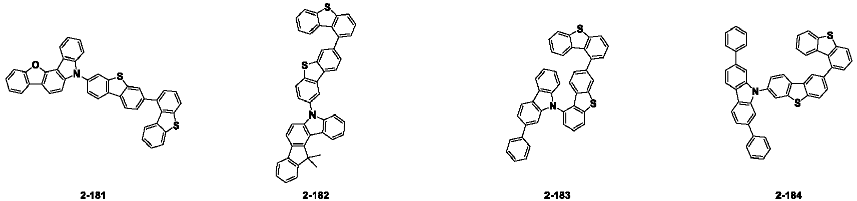

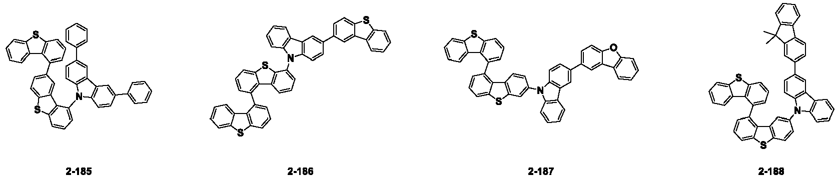

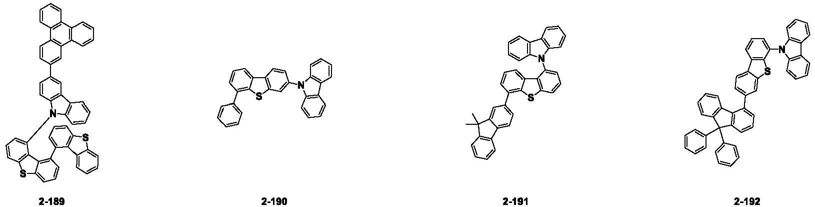

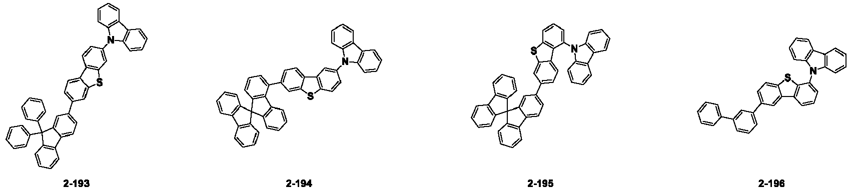

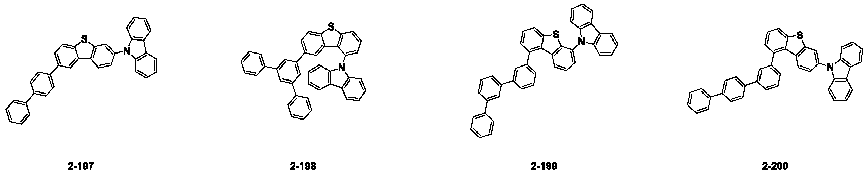

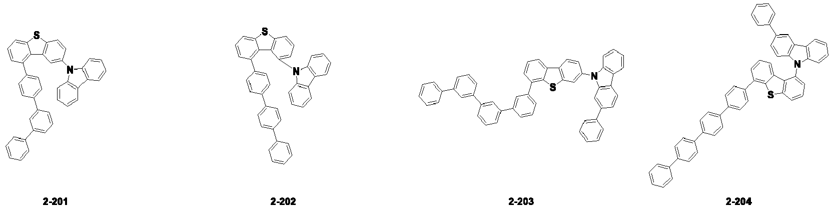

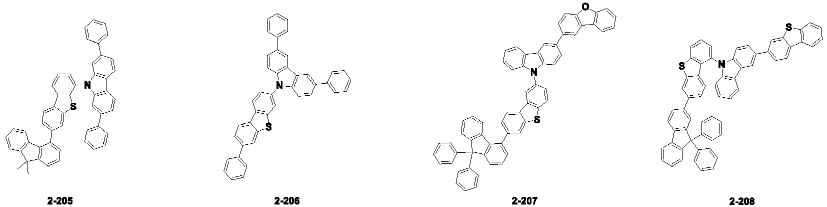

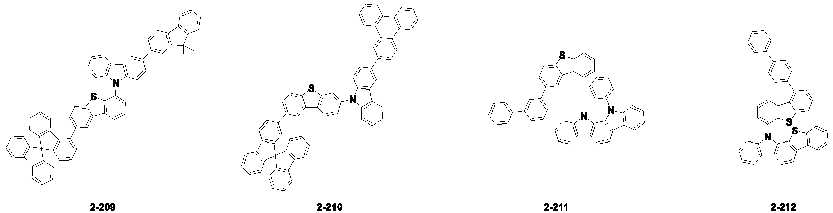

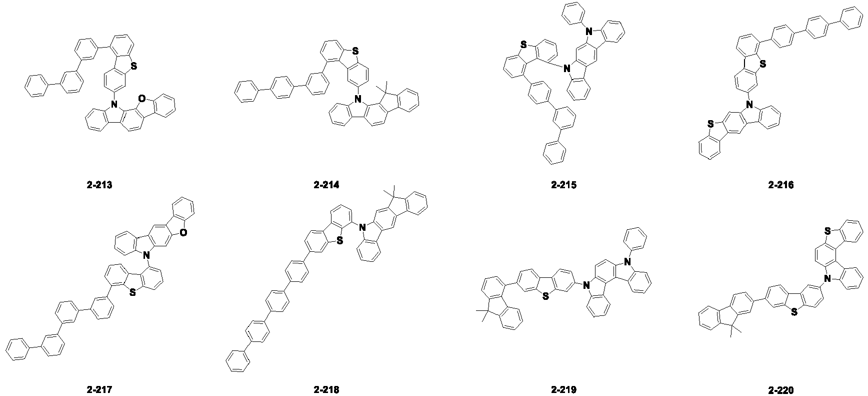

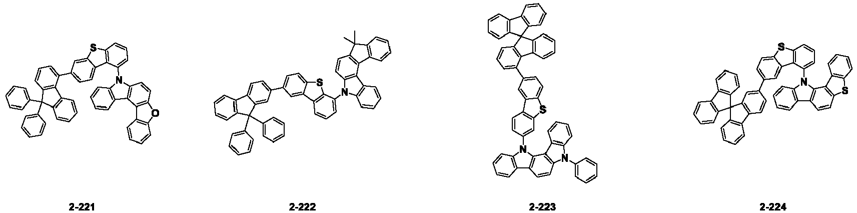

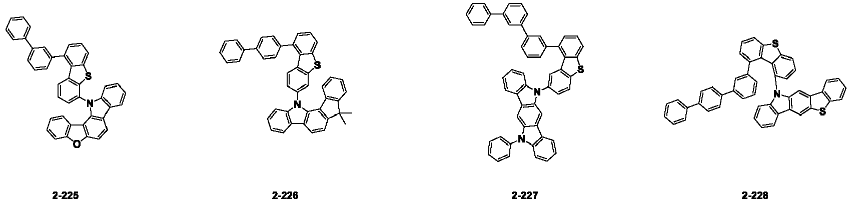

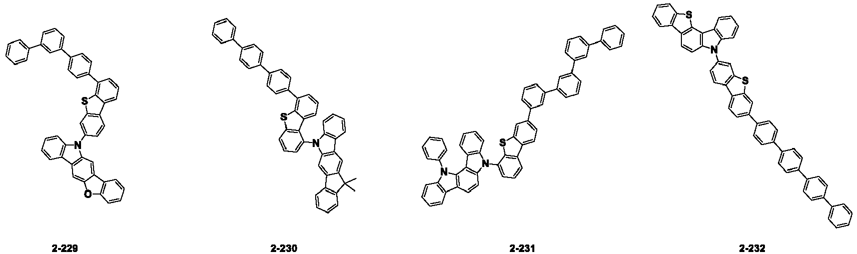

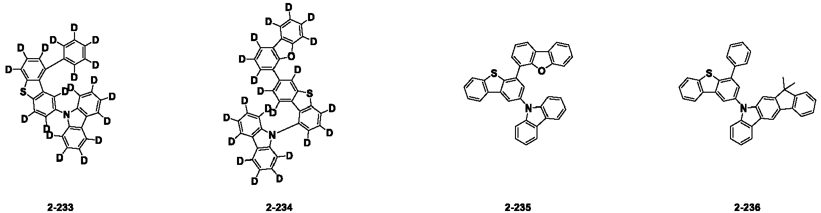

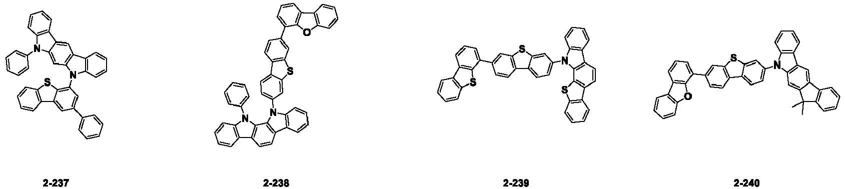

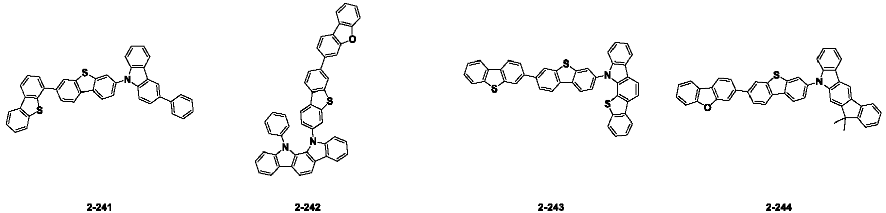

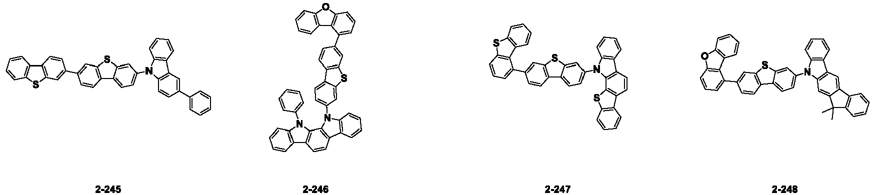

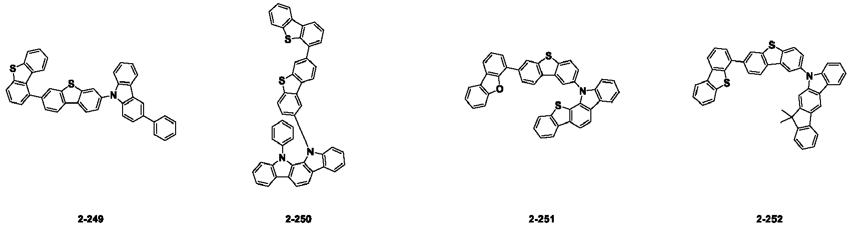

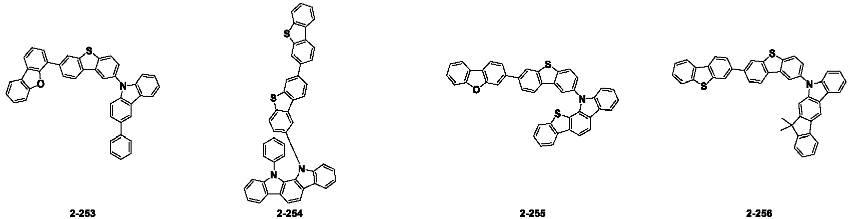

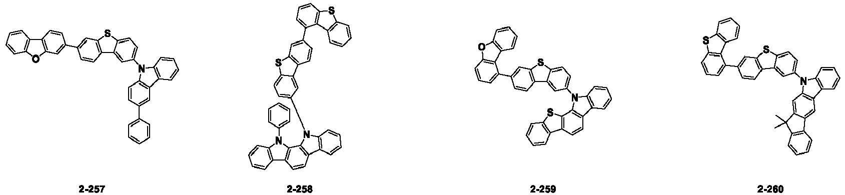

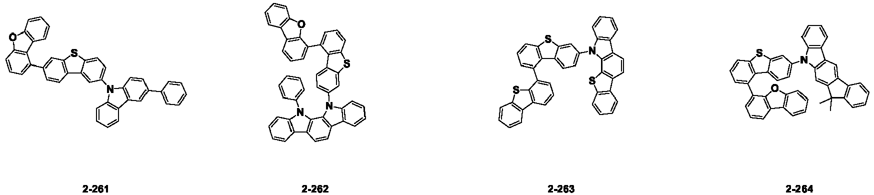

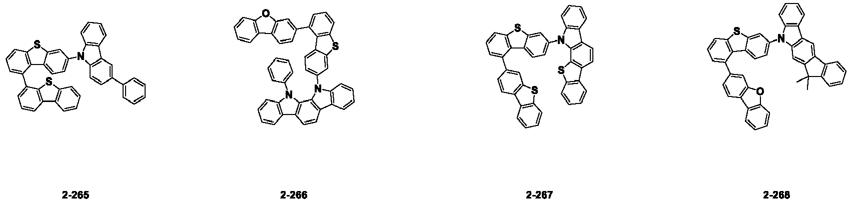

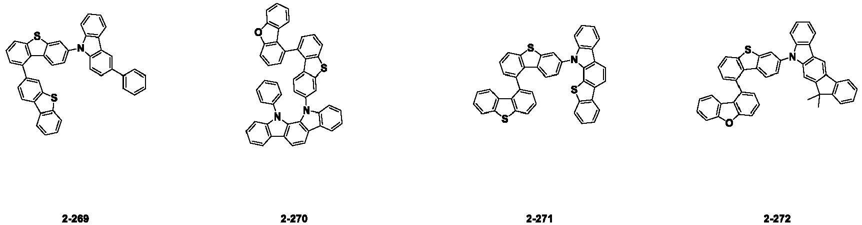

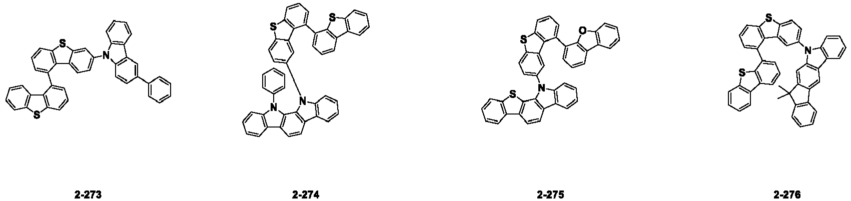

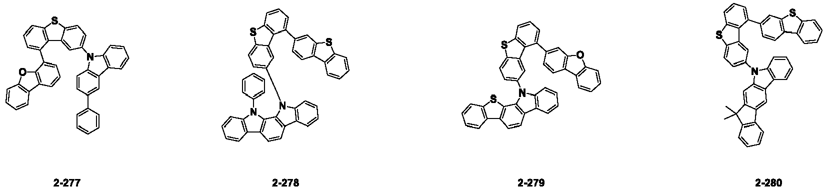

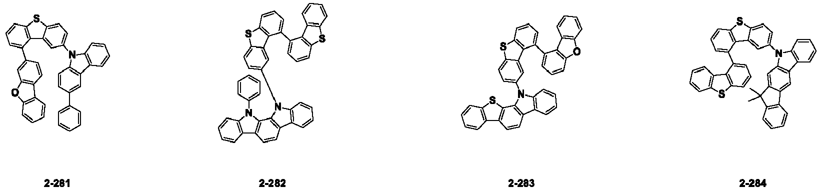

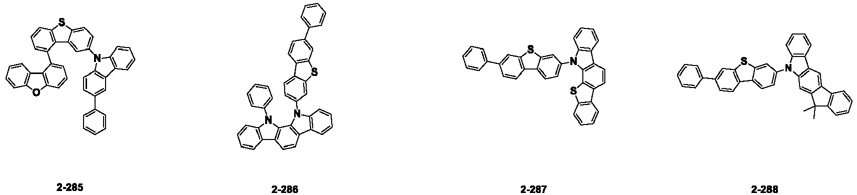

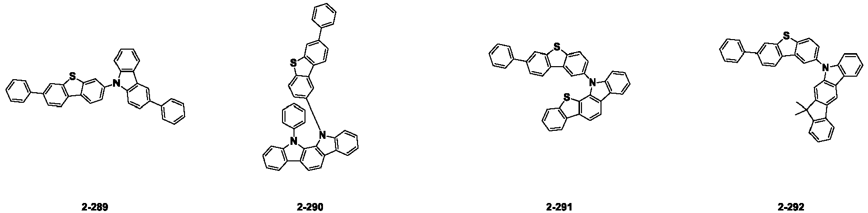

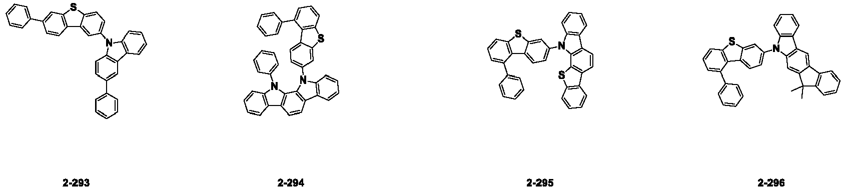

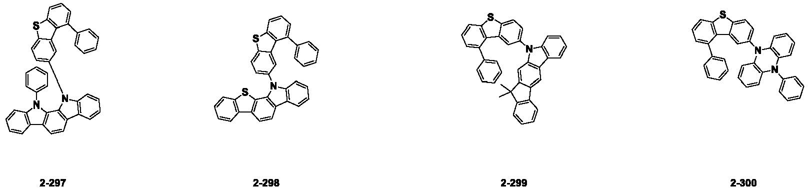

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device including a first electrode; a second electrode provided opposite to the first electrode; and one or more organic material layers provided between the first electrode and the second electrode, wherein one or more layers of the organic material layers include one or more of the heterocyclic compound according to Chemical Formula 1.

Another embodiment provides an organic light emitting device including a first electrode; a second electrode provided opposite to the first electrode; and one or more organic material layers provided between the first electrode and the second electrode, wherein one or more layers of the organic material layers include one of the heterocyclic compound according to Chemical Formula 1.

Another embodiment provides an organic light emitting device including a first electrode; a second electrode provided opposite to the first electrode; and one or more organic material layers provided between the first electrode and the second electrode, wherein one or more layers of the organic material layers include two of the heterocyclic compound according to Chemical Formula 1.

In the organic light emitting device, when including two or more of the heterocyclic compound, types of the heterocyclic compound may be the same as or different from each other.

Specific descriptions on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of a blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of a green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of a red organic light emitting device.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure including a hole injection layer, a hole transfer layer, a light emitting layer, an electron transfer layer, an electron injection layer and the like as the organic material layer. However, the structure of the organic light emitting device is not limited thereto, and may include a smaller number of organic material layers.

In the organic light emitting device of the present disclosure, the organic material layer may include a light emitting layer, and the light emitting layer may include the heterocyclic compound.

In another organic light emitting device, the organic material layer includes a light emitting layer, the light emitting layer includes a host material, and the host material may include the heterocyclic compound.

As another example, the organic material layer including the heterocyclic compound includes the heterocyclic compound represented by Chemical Formula 1 as a host, and an iridium-based dopant may be used therewith.

In the organic light emitting device of the present disclosure, the organic material layer includes an electron injection layer or an electron transfer layer, and the electron injection layer or the electron transfer layer may include the heterocyclic compound.

In another organic light emitting device, the organic material layer includes an electron blocking layer or a hole blocking layer, and the electron blocking layer or the hole blocking layer may include the heterocyclic compound.

The organic light emitting device of the present disclosure may further include one, two or more layers selected from the group consisting of a light emitting layer, a hole injection layer, a hole transfer layer, an electron injection layer, an electron transfer layer, an electron blocking layer and a hole blocking layer.

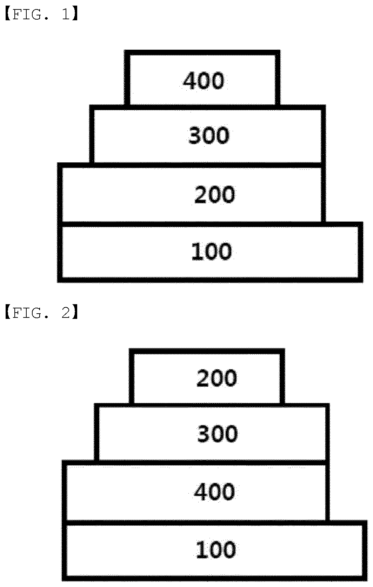

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

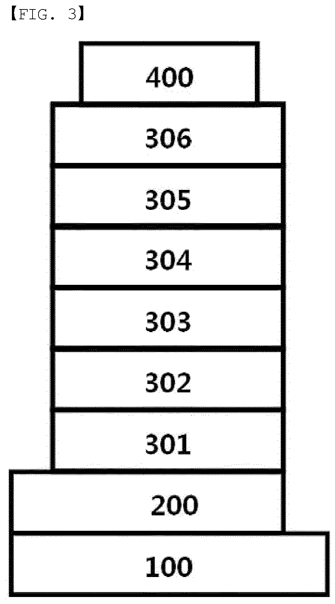

FIG. 3 illustrates a case of the organic material layer being a multilayer. The organic light emitting device according to FIG. 3 includes a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer including the compound of Chemical Formula 1 may further include other materials as necessary.

In the organic light emitting device according to one embodiment of the present application, the organic material layer may further include a heterocyclic compound represented by the following Chemical Formula 2; or a heterocyclic compound represented by the following Chemical Formula 3.

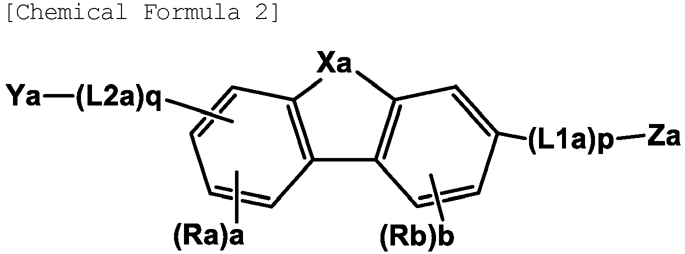

In Chemical Formula 2,
Xa is O or S,
Ya is a hole transferring group or a substituted or unsubstituted aryl group,
Za is an electron transferring group,
L1a and L2a are the same as or different from each other, and each independently a direct bond; a substituted or unsubstituted C6 to C60 arylene group; or a substituted or unsubstituted C2 to C60 heteroarylene group, p and q are each an integer of 0 to 3, when p is 2 or greater, L1as are the same as or different from each other, and when q is 2 or greater, L2as are the same as or different from each other, and
Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, a and b are each an integer of 0 to 3, when a is 2 or greater, Ras are the same as or different from each other, and when b is 2 or greater, Rbs are the same as or different from each other, 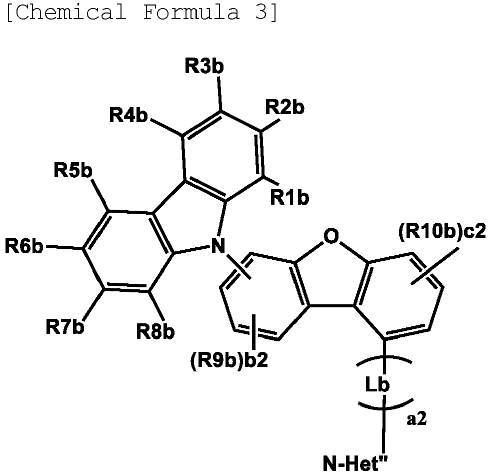 in Chemical Formula 3,
   N-Het" is a monocyclic or polycyclic heterocyclic group substituted or unsubstituted and including one or more Ns,
   Lb is a direct bond; a substituted or unsubstituted arylene group; or a substituted or unsubstituted heteroarylene group, a2 is an integer of 1 to 3, and when a is 2 or greater, Lbs are the same as or different from each other, and
   R1b to R10b are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, b2 and c2 are each an integer of 0 to 3, when b2 is 2 or greater, R9bs are the same as or different from each other, and when c2 is 2 or greater, R10bs are the same as or different from each other.

Particularly, when further including the heterocyclic compound represented by Chemical Formula 2 or Chemical Formula 3, an exciplex phenomenon occurs. The exciplex phenomenon refers to forming a bicomplex in an excited state due to electron exchanges between a molecule having strong donor properties and a molecule having strong acceptor properties.

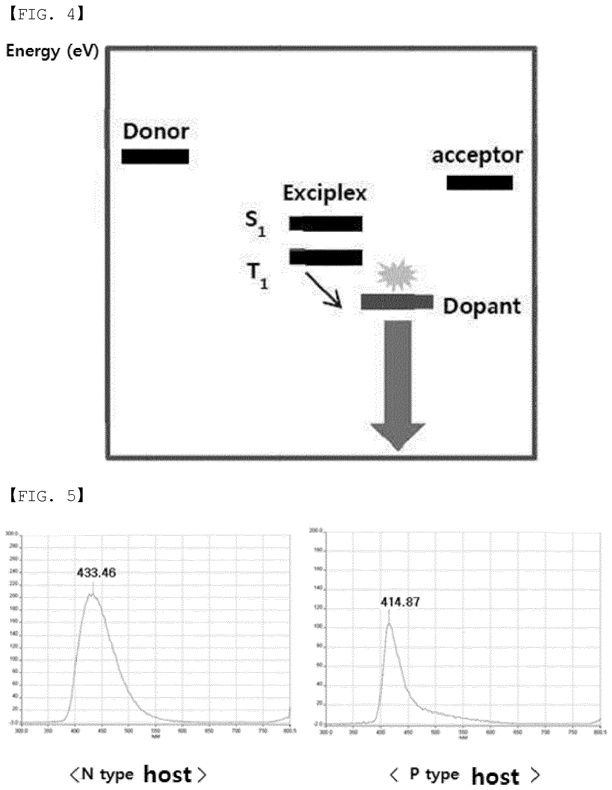

FIG. 4 is a diagram explaining an exciplex phenomenon. When an exciplex phenomenon occurs as in FIG. 4, new S1 energy level and T1 energy level are formed, and a change in the PL red shifted compared to each molecule may be identified.

In other words, when an exciplex having a bicomplex form in an excited state between donor and acceptor molecules is formed, a new energy level different from the energy levels of the donor and the acceptor is formed, and herein, light emitted from this energy level is red shifted compared to the light emitted by each of the donor and the accepter, and in order to identify this phenomenon, PL is measured. Accordingly, by comparing an emission wavelength of the single host and an emission wavelength of the mixed host from the PL data, an occurrence of exciplex in the molecule may be identified.

When the exciplex phenomenon occurs between two molecules as above, reverse intersystem crossing (RISC) occurs, and as a result, internal quantum efficiency may increase up to 100%.

Hereinafter, the heterocyclic compound represented by Chemical Formula 2 will be described.

In one embodiment of the present application, Chemical Formula 2 may be represented by the following Chemical Formula 2-1-1.

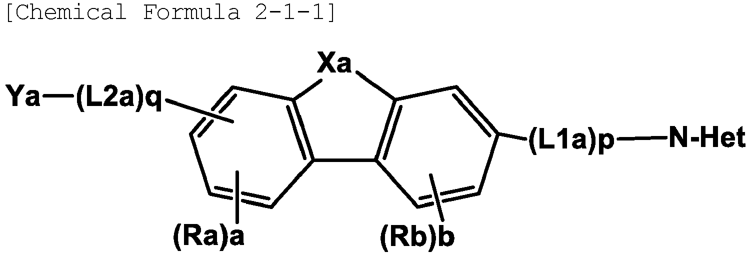

In Chemical Formula 2-1-1,
N-Het is a monocyclic or polycyclic heteroaryl group substituted or unsubstituted and including one or more Ns, and the rest of the substituents have the same definitions as in Chemical Formula 2.

In one embodiment of the present application, N-Het of Chemical Formula 2-1-1 is a monocyclic heteroaryl group substituted or unsubstituted and including one or more Ns.

In one embodiment of the present application, N-Het of Chemical Formula 2-1-1 is a dicyclic or higher polycyclic heteroaryl group substituted or unsubstituted and including one or more Ns.

In one embodiment of the present application, N-Het of Chemical Formula 2-1-1 is a monocyclic or polycyclic heteroaryl group substituted or unsubstituted and including two or more Ns.

In one embodiment of the present application, N-Het of Chemical Formula 2-1-1 is a dicyclic or higher polycyclic heteroaryl group including two or more Ns.

In one embodiment of the present application, N-Het of Chemical Formula 2-1-1 is a monocyclic heteroaryl group including three Ns.

In one embodiment of the present application, N-Het of Chemical Formula 2-1-1 forms a carbon-carbon bond with L1a.

In one embodiment of the present application, N forming the ring in N-Het of Chemical Formula 2-1-1 has an sp2 bond with neighboring C.

In one embodiment of the present application, Ya is a substituted or unsubstituted dibenzofuran group; a substituted or unsubstituted dibenzothiophene group; a substituted or unsubstituted carbazole group; a substituted or unsubstituted group in which a monocyclic or polycyclic ring is fused to carbazole; a substituted or unsubstituted silyl group; or a substituted or unsubstituted aryl group.

In one embodiment of the present application, Chemical Formula 2 may be represented by one of the following Chemical Formulae 3-1-1 to 5-1-1.

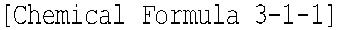

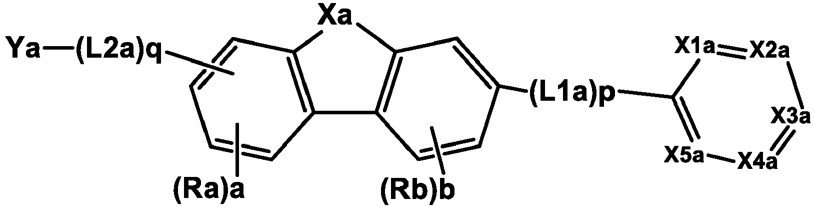

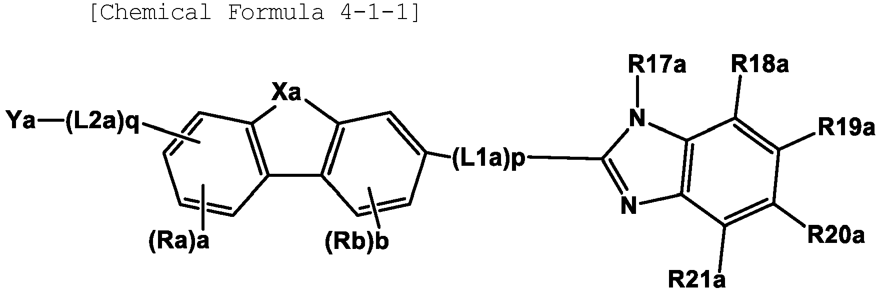

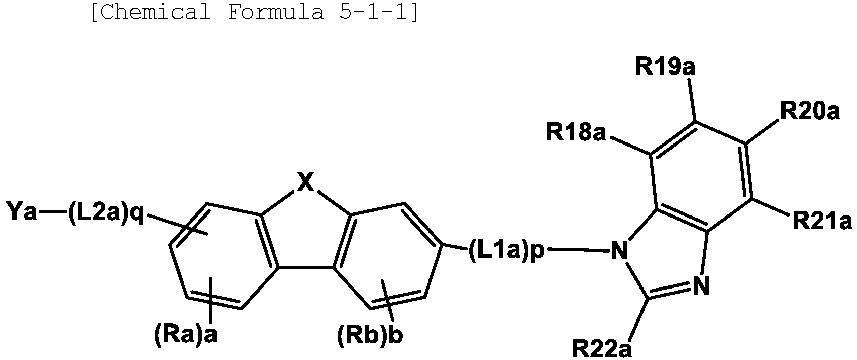

In Chemical Formulae 3-1-1 to 5-1-1,
X1a is CR11a or N, X2a is CR12a or N, X3a is CR13a or N, X4a is CR14a or N, and X5a is CR15a or N,
R11a to R15a and R17a to R22a are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, and
the rest of the substituents have the same definitions as in Chemical Formula 2.

In one embodiment of the present application, Chemical Formula 2 may be represented by the following Chemical Formulae 6-1-1 to 8-1-1.

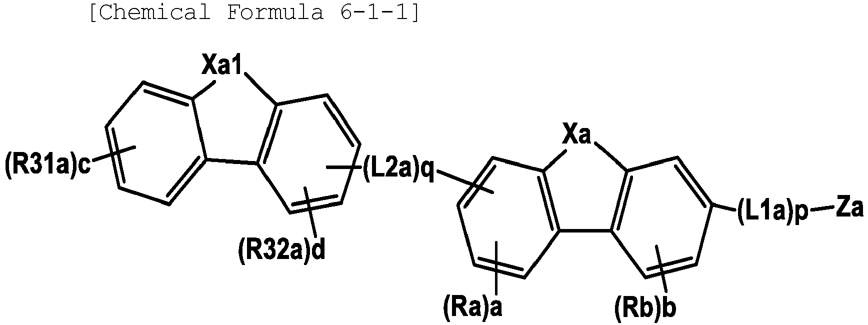

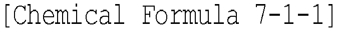

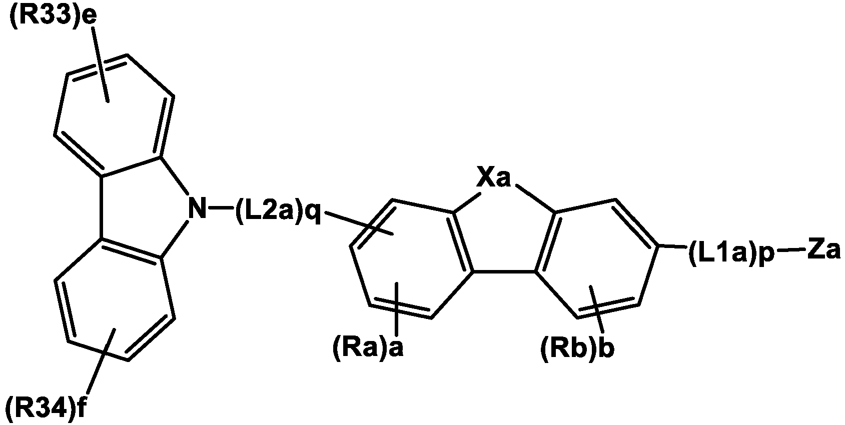

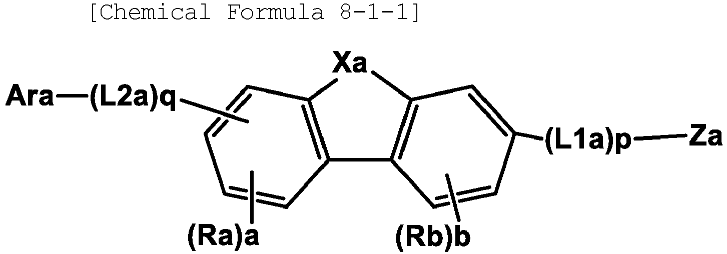

In Chemical Formulae 6-1-1 to 8-1-1,
Xa1 is S, O, CRcRd or NRe,
R31a to R34a, Rc, Rd and Re are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, c, e and f are each an integer of 0 to 4, d is an integer of 0 to 3, when c is 2 or greater, R31as are the same as or different from each other, when d is 2 or greater, R32as are the same as or different from each other, when e is 2 or greater, R33as are the same as or different from each other, and when f is 2 or greater, R34as are the same as or different from each other,
Ara is a substituted or unsubstituted C6 to C60 aryl group; or a substituted or unsubstituted silyl group, and
the rest of the substituents have the same definitions as in Chemical Formula 2.

In one embodiment of the present application, Xa is S, and Chemical Formula 2 may be represented by one of Chemical Formulae 6-1-1 to 8-1-1.

In one embodiment of the present application, Xa is O, Chemical Formula 2 is represented by one of Chemical Formulae 6-1-1 to 8-1-1, and L2a of Chemical Formula 7-1-1 is a direct bond.

In one embodiment of the present application, Xa is O, and Chemical Formula 2 may be represented by Chemical Formula 6-1-1 or Chemical Formula 8-1-1.

In one embodiment of the present application, 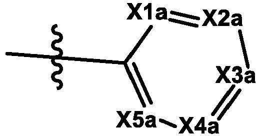 may be represented by one of the following Chemical Formulae 9-1-1 to 11-1-1. Herein, 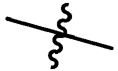 is a site linked to L1a.

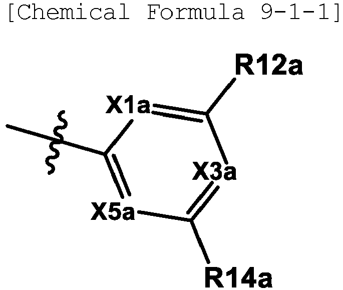

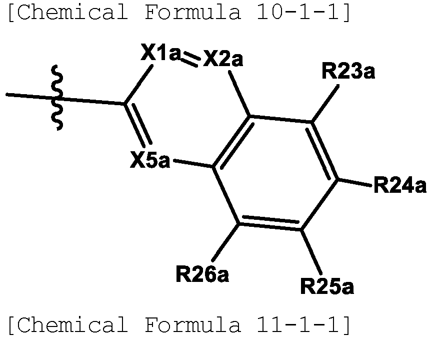

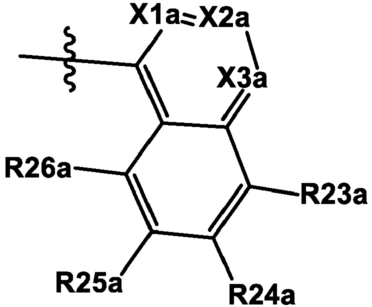

In Chemical Formula 9-1-1, one or more of X1a, X3a and X5a are N, and the rest have the same definitions as in Chemical Formula 3-1-1,
in Chemical Formula 10-1-1, one or more of X1a, X2a and X5a are N, and the rest have the same definitions as in Chemical Formula 3-1-1,
in Chemical Formula 11-1-1, one or more of X1a to X3a are N, and the rest have the same definitions as in Chemical Formula 3-1-1,
R12a, R14a and R23a to R26a are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring.

In one embodiment of the present application, Chemical Formula 9-1-1 may be selected from among the following structural formulae.

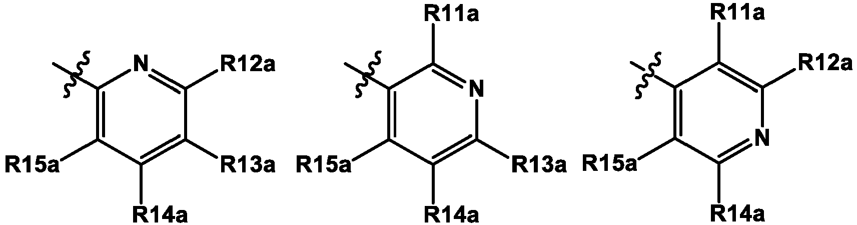

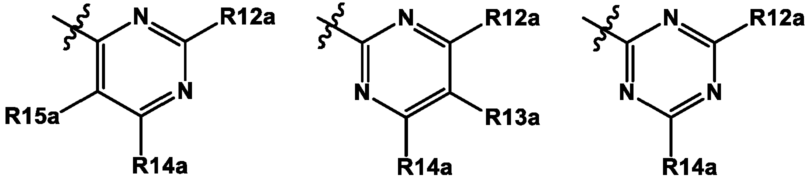

In the structural formulae, each substituent has the same definition as in Chemical Formula 9-1-1.

In one embodiment of the present application, Chemical Formula 10-1-1 may be represented by the following Chemical Formula 12-1-1. 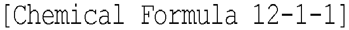 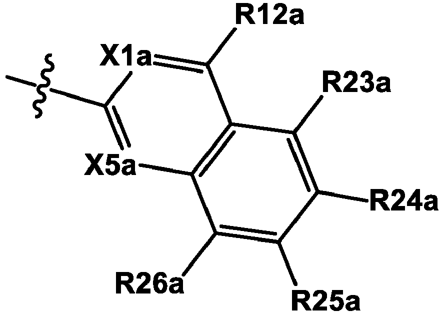 substituents of Chemical Formula 12-1-1 have the same definitions as in Chemical Formula 10-1-1.

In one embodiment of the present application, Chemical Formula 11-1-1 may be represented by the following Chemical Formula 13-1-1. 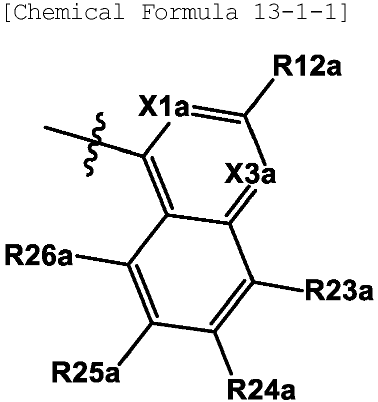 substituents of Chemical Formula 13-1-1 have the same definitions as in Chemical Formula 11-1-1.

In one embodiment of the present application, Chemical Formula 10-1-1 may be represented by the following Chemical Formula 14-1-1.

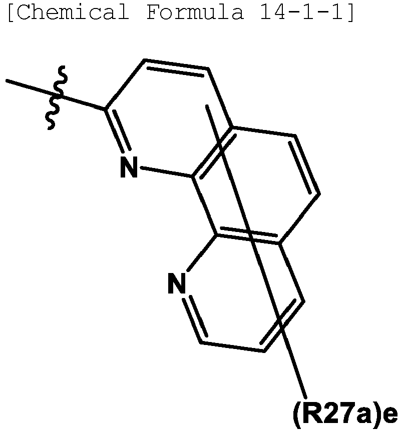

In Chemical Formula 14-1-1,
R27as are the same as or different from each other, and selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, e is an integer of 0 to 7, and when e is 2 or greater, R27as are the same as or different from each other.

In one embodiment of the present application, Chemical Formula 2 may be represented by the following Chemical Formula 15-1-1.

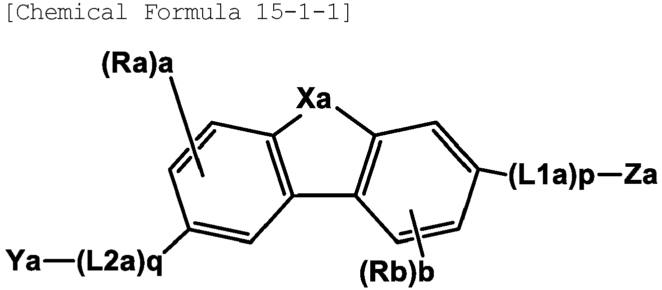

In Chemical Formula 15-1-1,
substituents have the same definitions as in Chemical Formula 2.

In one embodiment of the present application, Chemical Formula 2 may be represented by the following Chemical Formula 16-1-1.

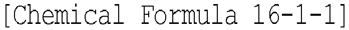

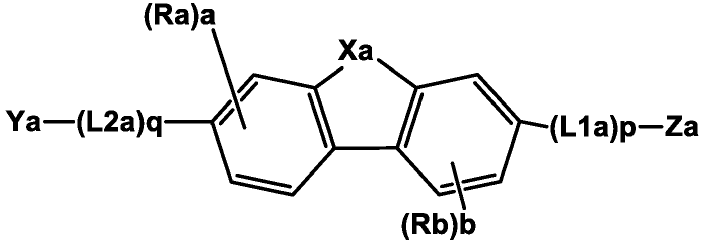

In Chemical Formula 16-1-1,
substituents have the same definitions as in Chemical Formula 2.

In one embodiment of the present application, Chemical Formula 2 may be represented by any one of the following compounds.

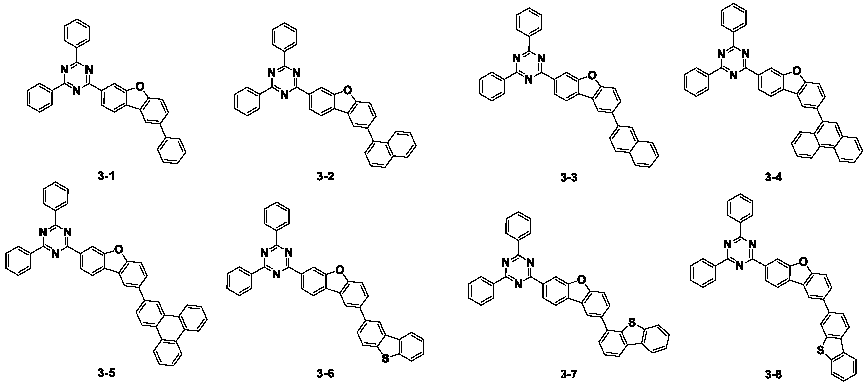

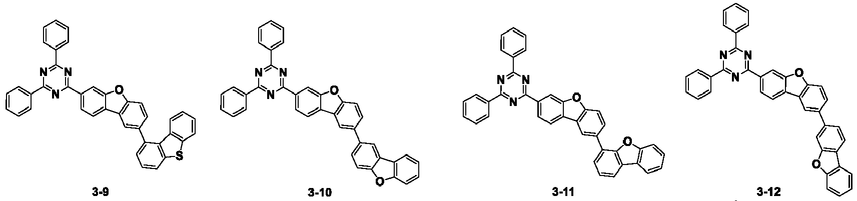

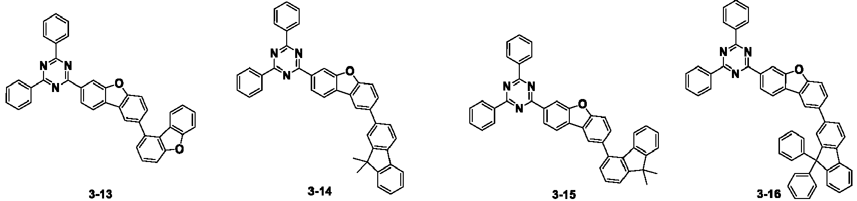

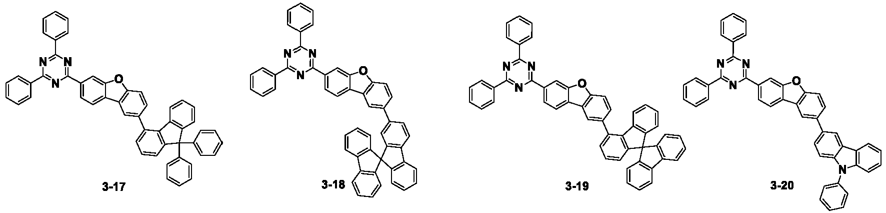

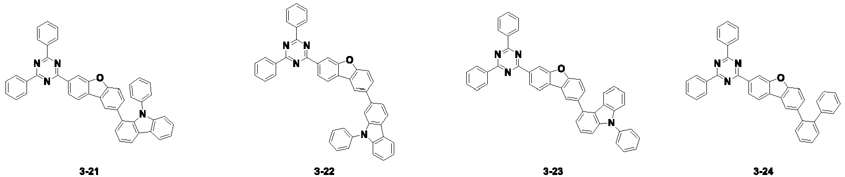

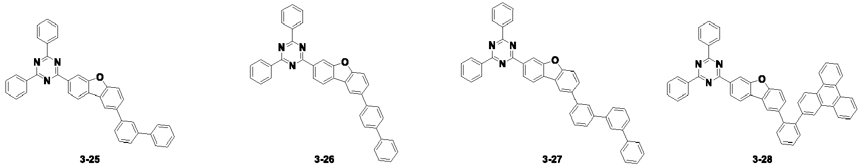

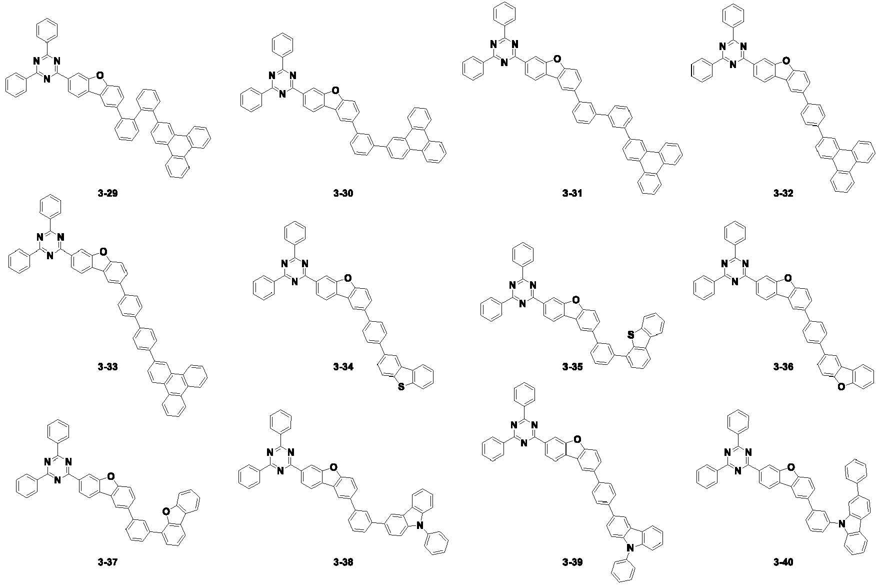

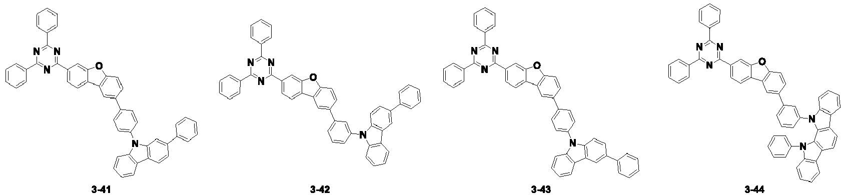

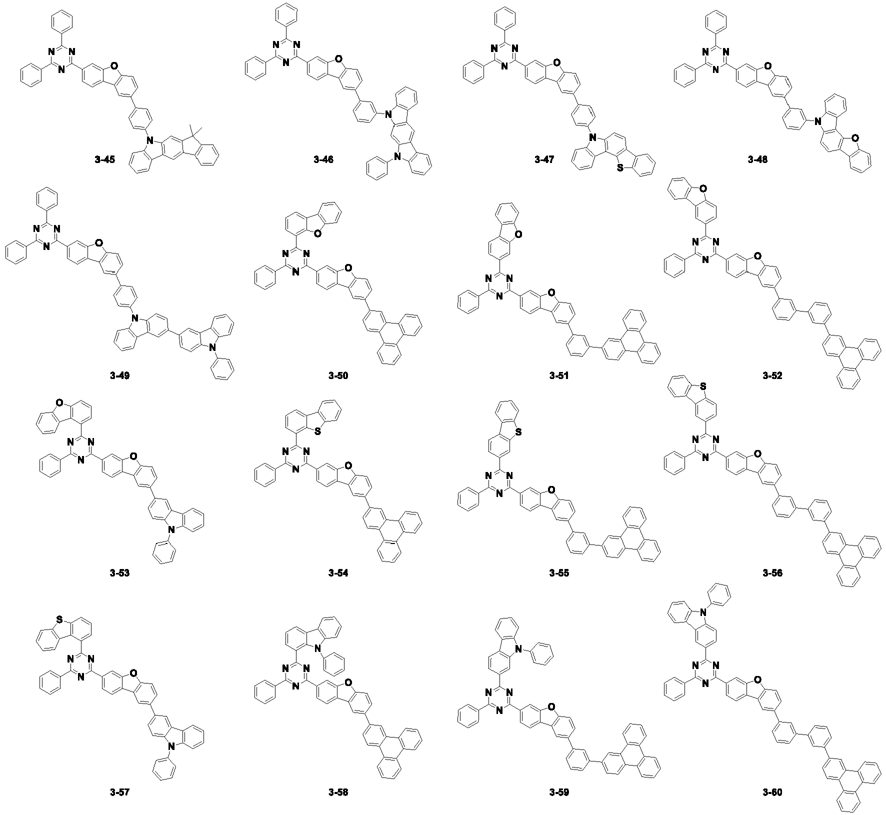

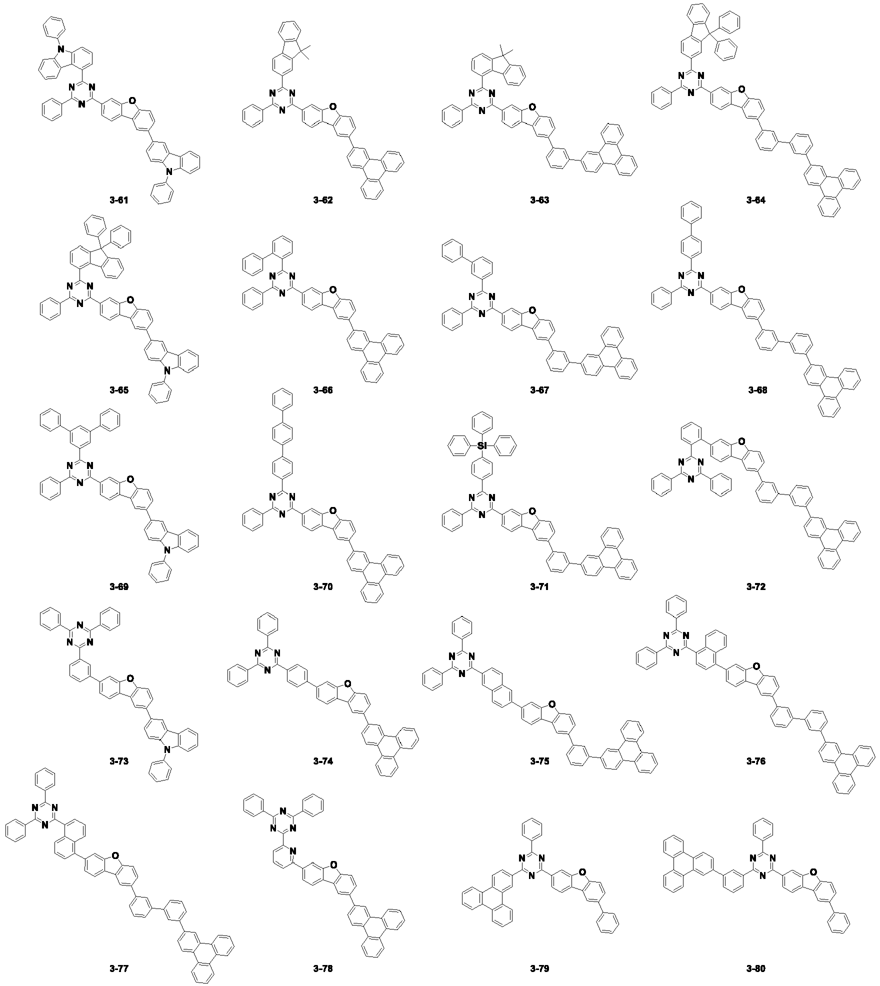

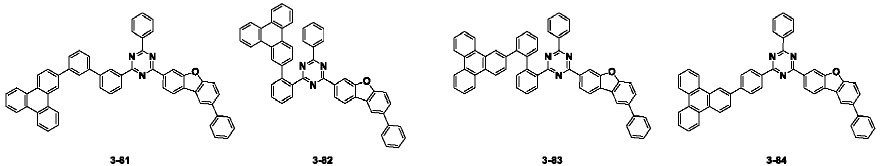

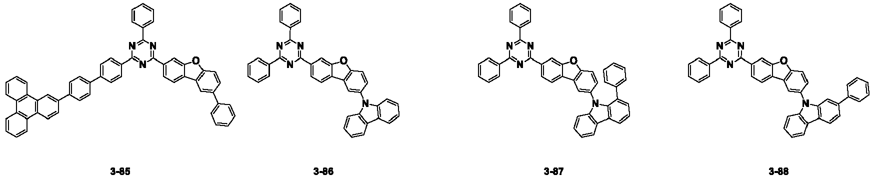

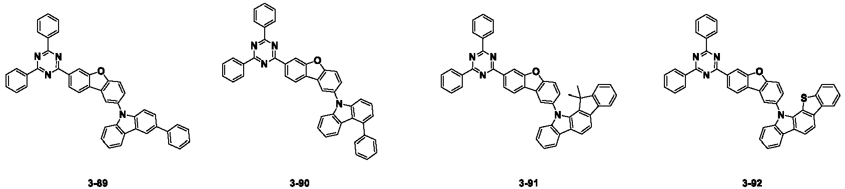

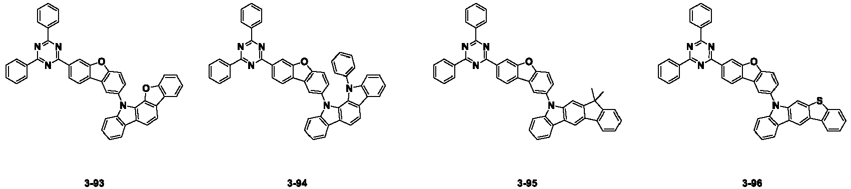

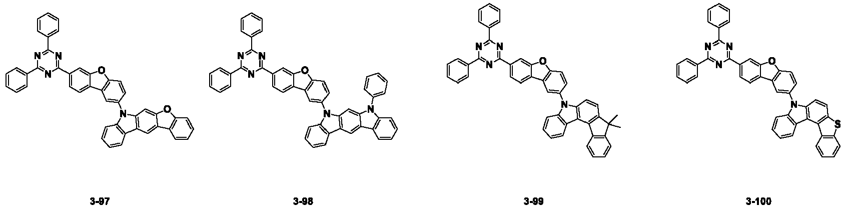

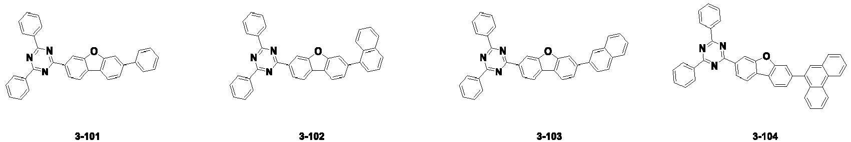

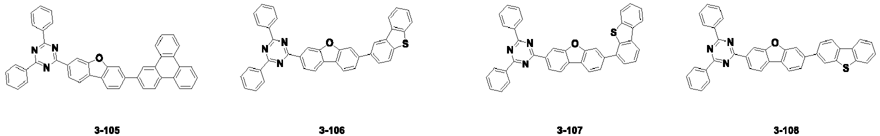

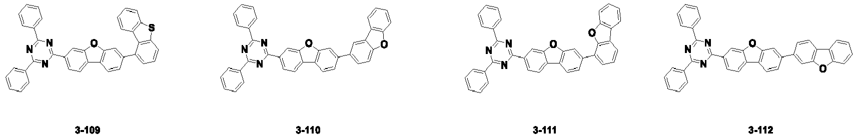

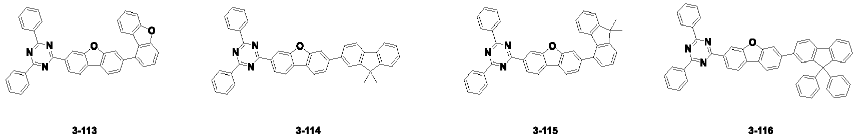

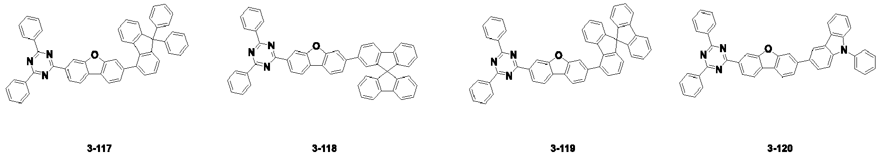

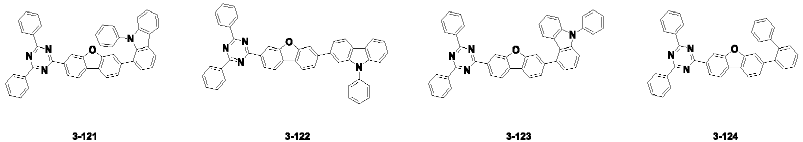

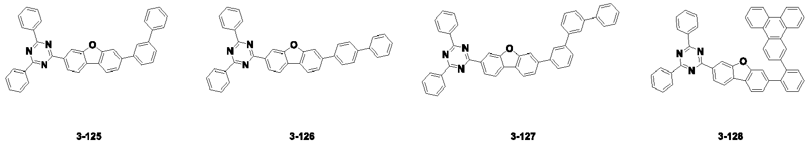

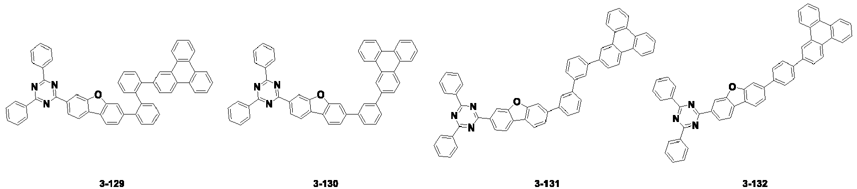

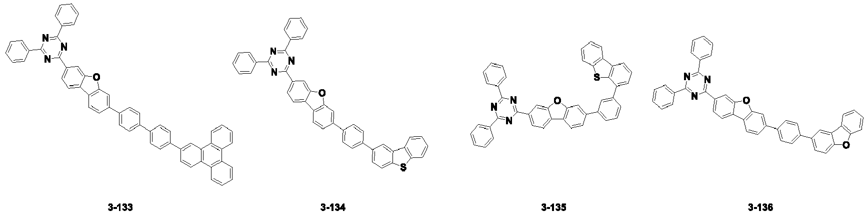

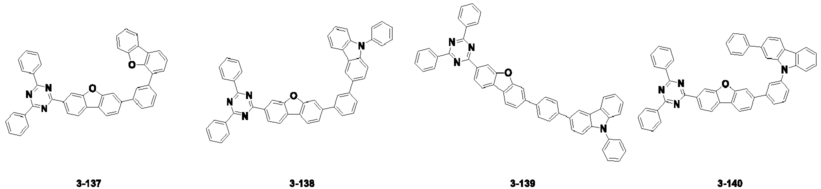

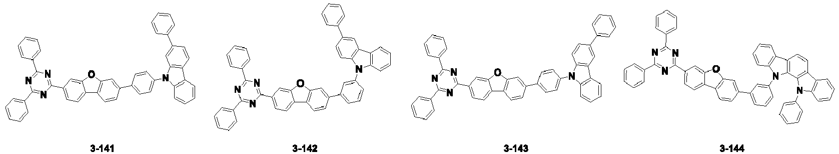

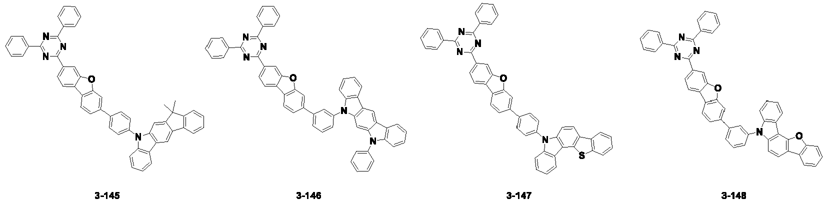

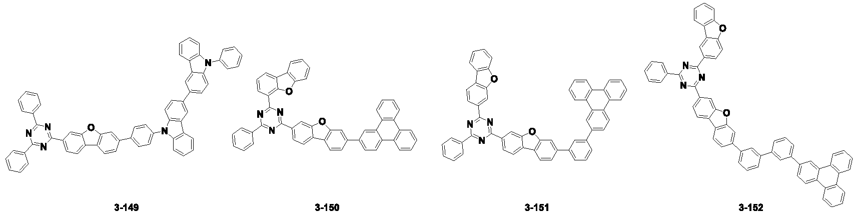

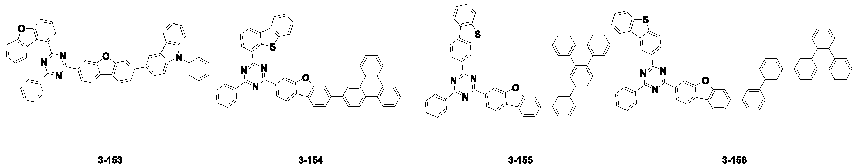

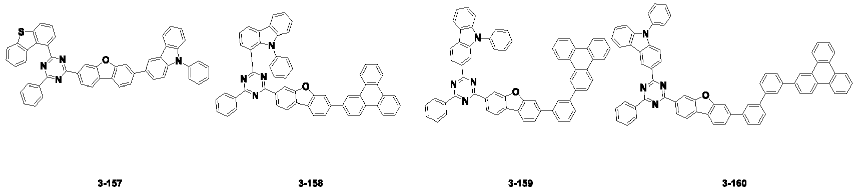

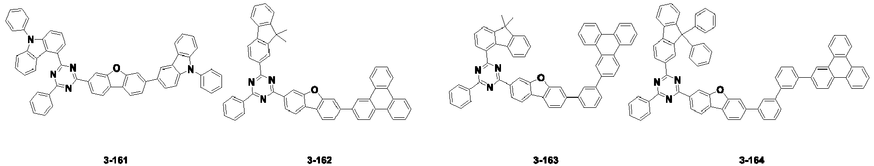

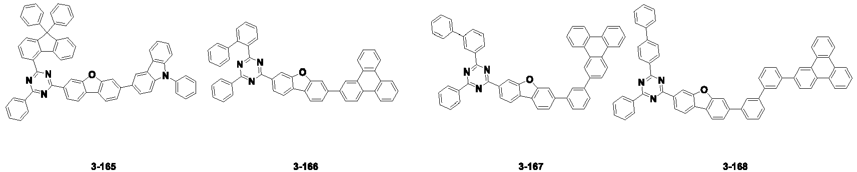

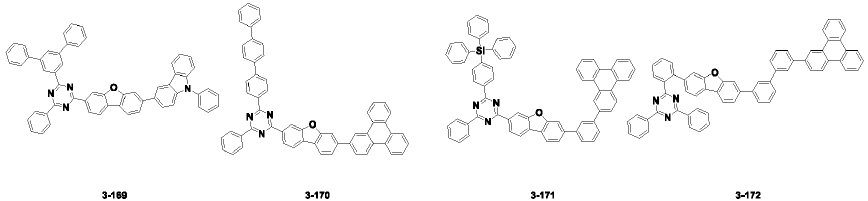

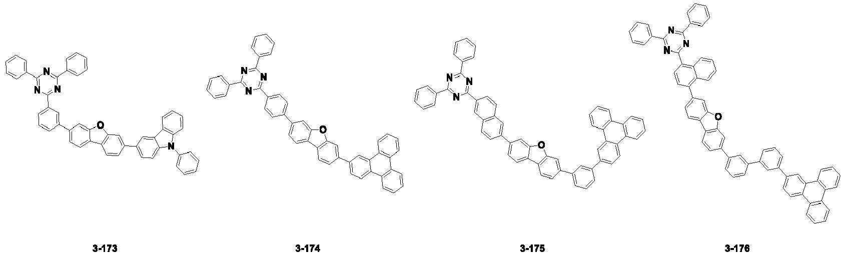

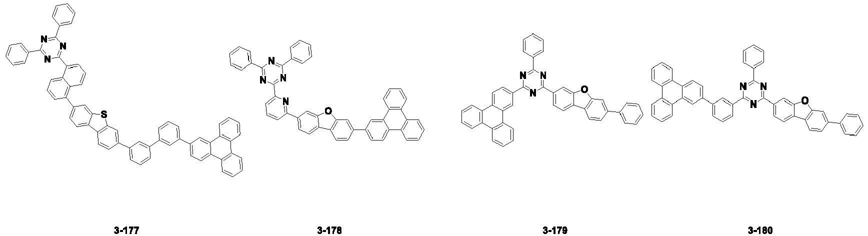

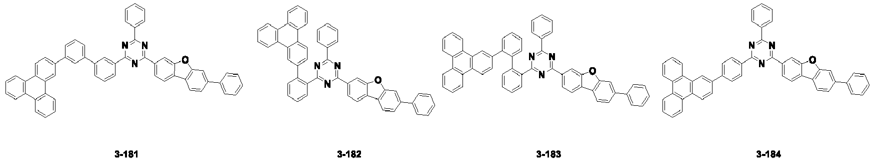

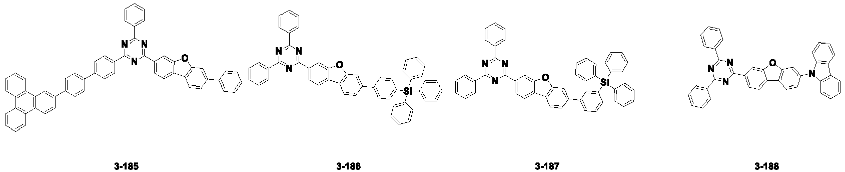

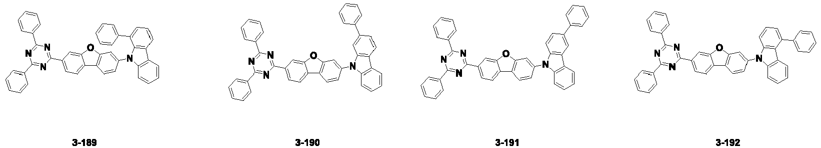

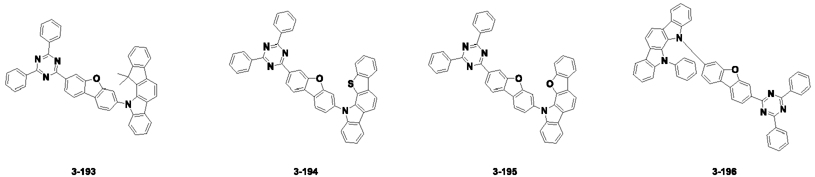

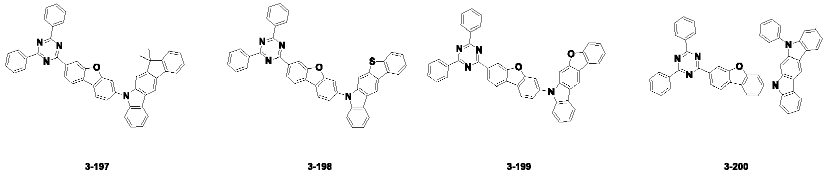

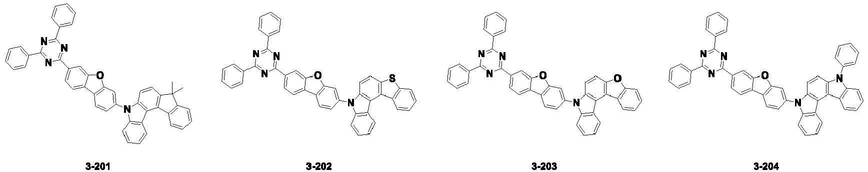

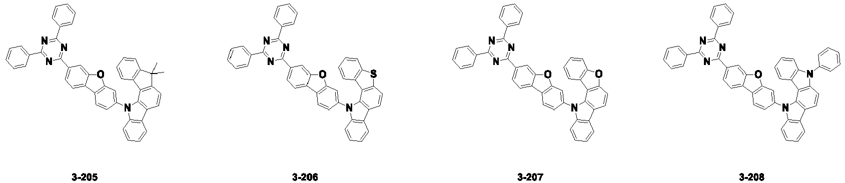

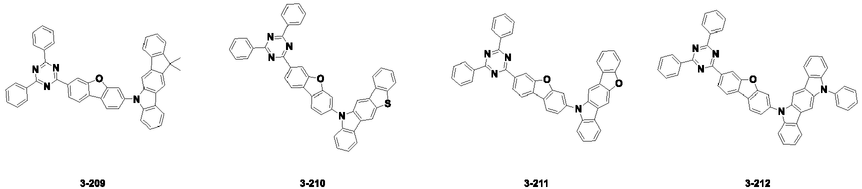

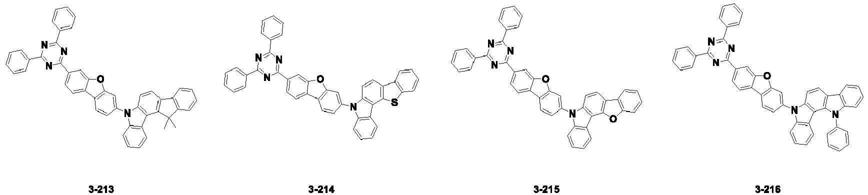

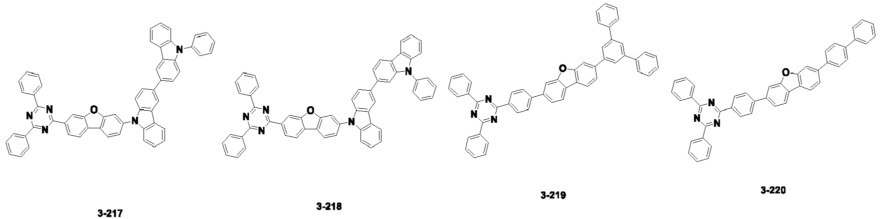

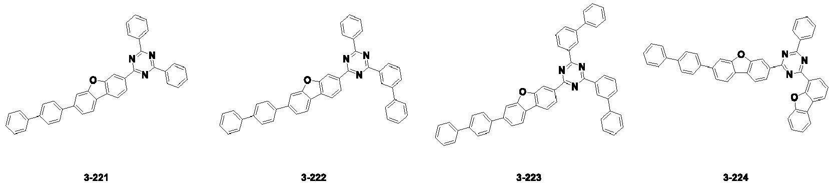

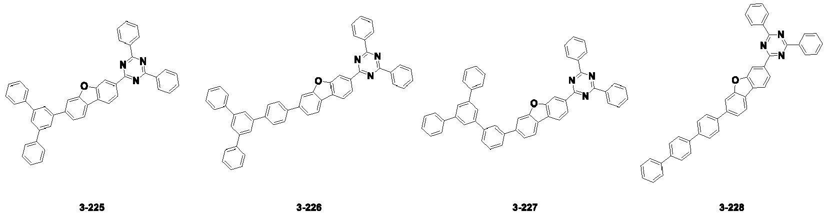

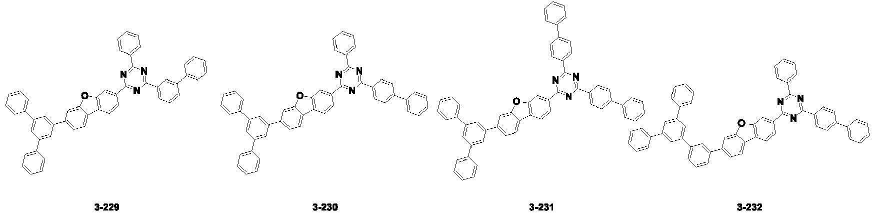

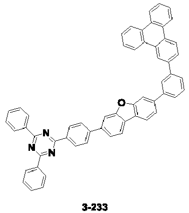

Hereinafter, the heterocyclic compound represented by Chemical Formula 3 will be described.

In one embodiment of the present application, Chemical Formula 3 may be represented by one of the following Chemical Formulae 2-2-1 to 5-2-1.

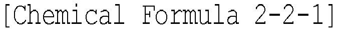

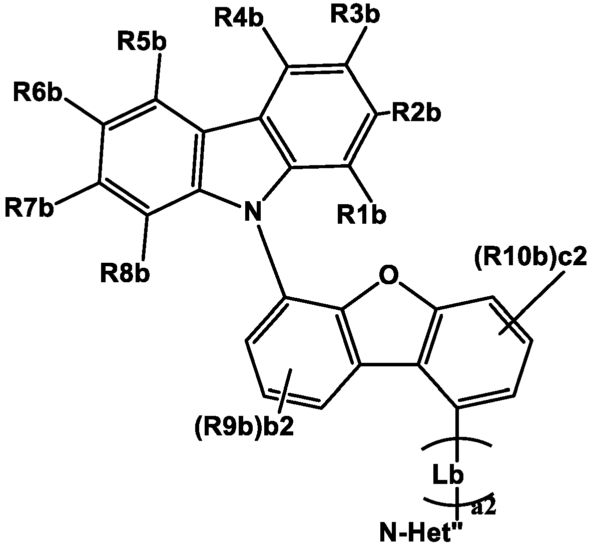

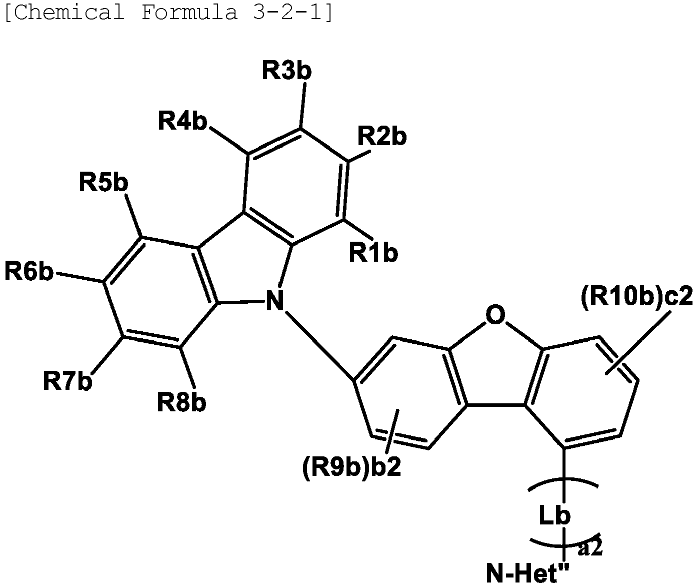

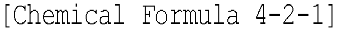

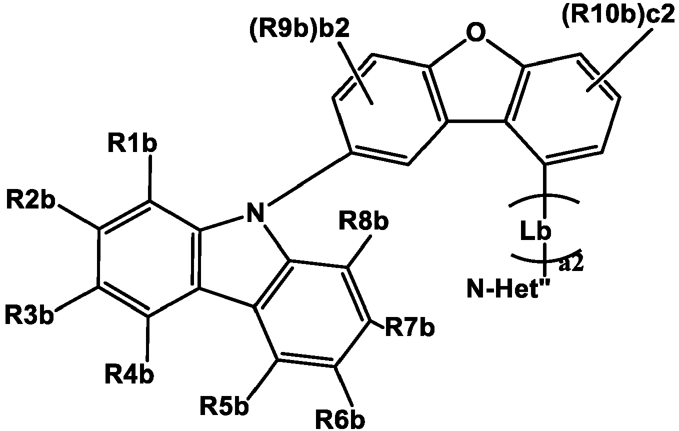

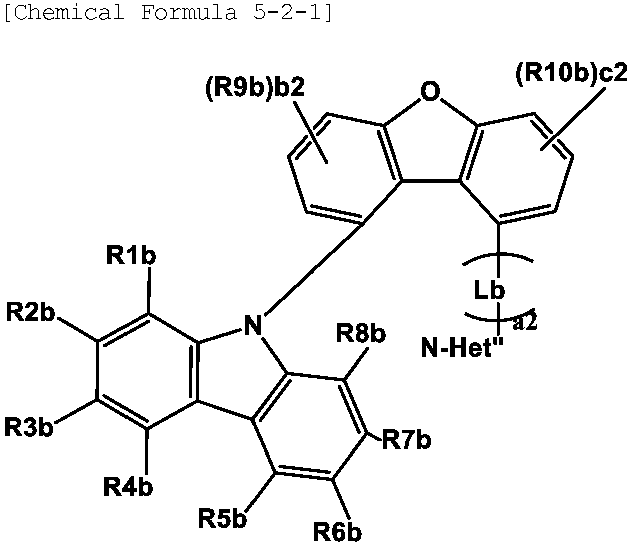

In Chemical Formulae 2-2-1 to 5-2-1,
substituents have the same definitions as in Chemical Formula 3.

In one embodiment of the present application, N-Het" is a monocyclic or polycyclic heteroring substituted or unsubstituted and including one or more Ns.

In another embodiment, N-Het" is a monocyclic or polycyclic heteroring unsubstituted or substituted with one or more substituents selected from the group consisting of an aryl group and a heteroaryl group, and including one or more Ns.

In another embodiment, N-Het" is a monocyclic or polycyclic heteroring unsubstituted or substituted with one or more substituents selected from the group consisting of a phenyl group, a biphenyl group, a naphthyl group, a dimethylfluorene group, a dibenzofuran group and a dibenzothiophene group, and including one or more Ns.

In another embodiment, N-Het" is a monocyclic or polycyclic heteroring unsubstituted or substituted with one or more substituents selected from the group consisting of a phenyl group, a biphenyl group, a naphthyl group, a dimethylfluorene group, a dibenzofuran group and a dibenzothiophene group, and including one or more and three or less Ns.

In one embodiment of the present application, N-Het" is a monocyclic heteroring substituted or unsubstituted and including one or more Ns.

In one embodiment of the present application, N-Het" is a dicyclic or higher heteroring substituted or unsubstituted and including one or more Ns.

In one embodiment of the present application, N-Het" is a monocyclic or polycyclic heteroring substituted or unsubstituted and including two or more Ns.

In one embodiment of the present application, N-Het" is a dicyclic or higher polycyclic heteroring including two or more Ns.

In one embodiment of the present application, Chemical Formula 3 is represented by one of the following Chemical Formulae 6-2-1 to 8-2-1.

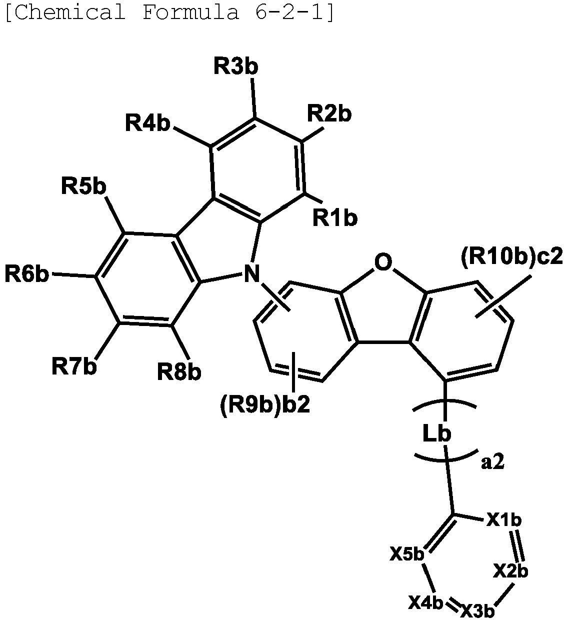

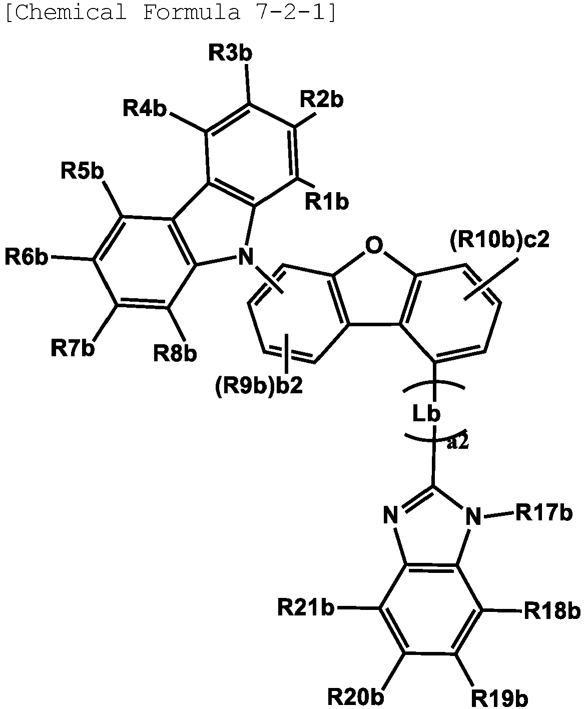

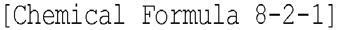

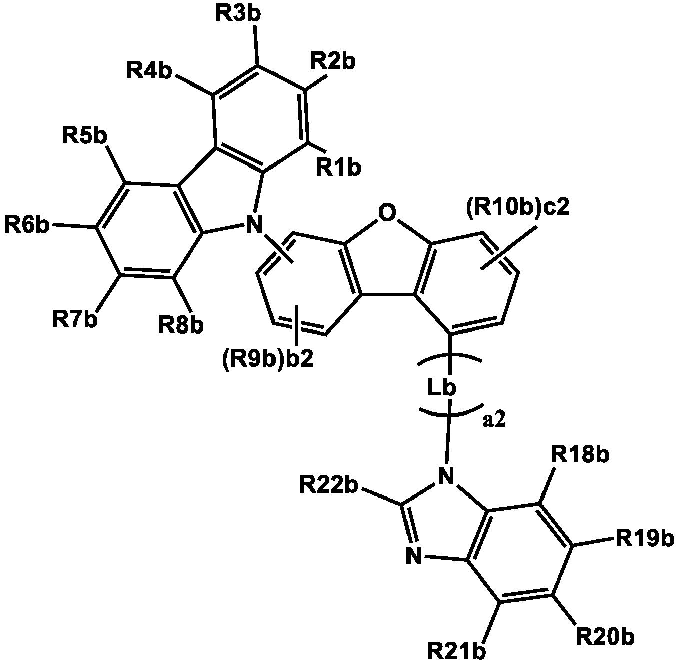

In Chemical Formulae 6-2-1 to 8-2-1,
X1b is CR11b or N, X2b is CR12b or N, X3b is CR13b or N, X4b is CR14b or N, and X5b is CR15b or N,
R11b to R15b and R17b to R22b are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, and
the rest of the substituents have the same definitions as in Chemical Formula 3.

In one embodiment of the present application, Chemical Formula 3 may be represented by any one of the following compounds.

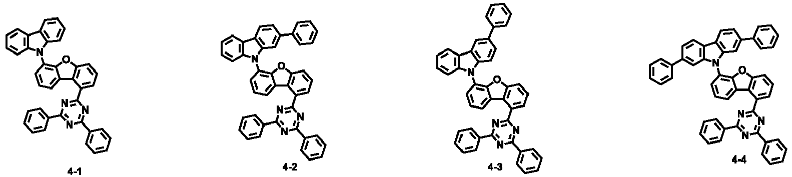

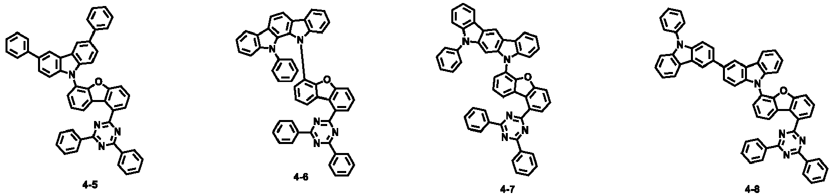

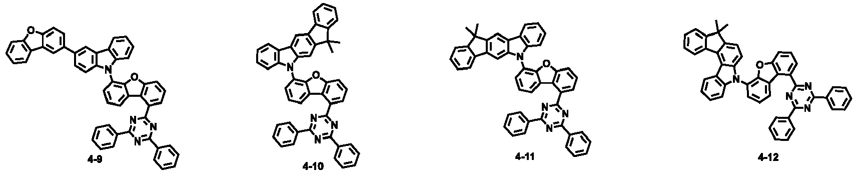

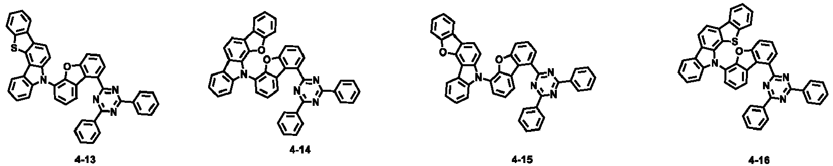

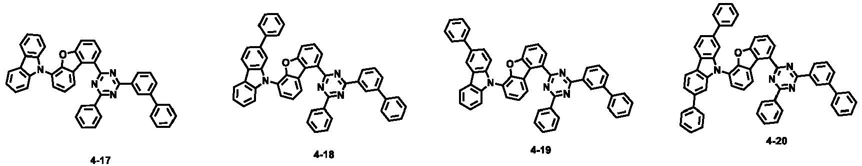

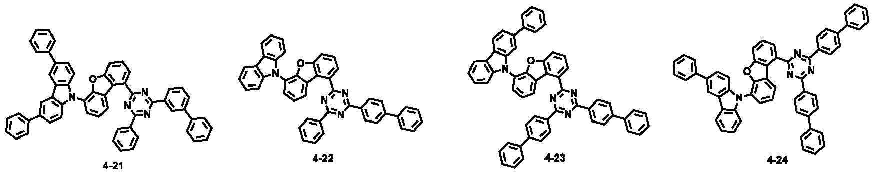

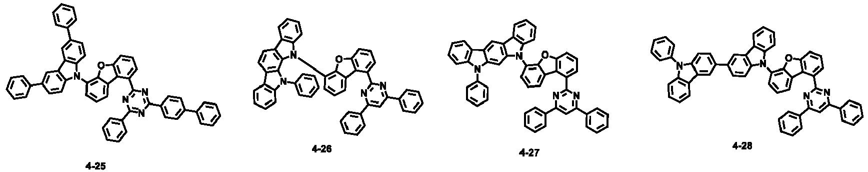

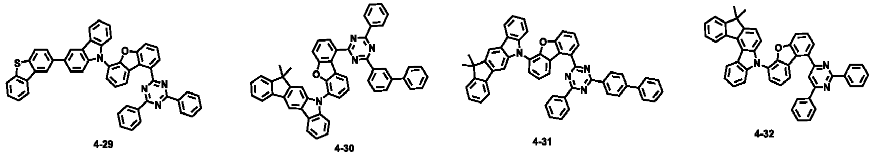

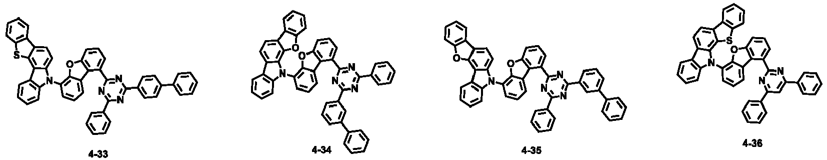

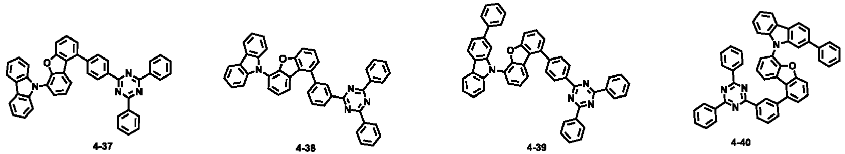

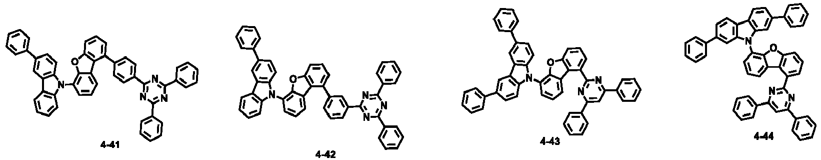

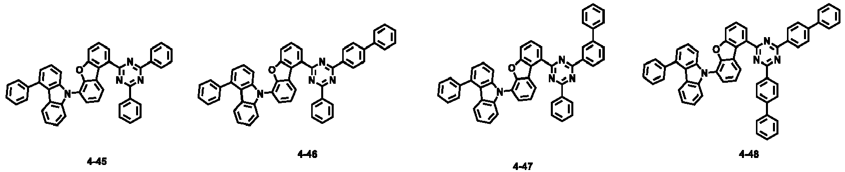

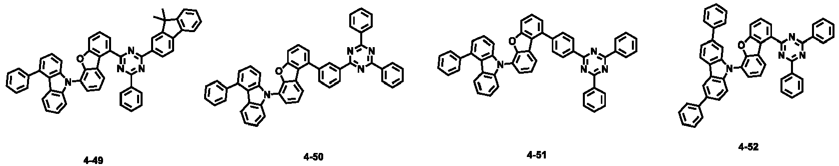

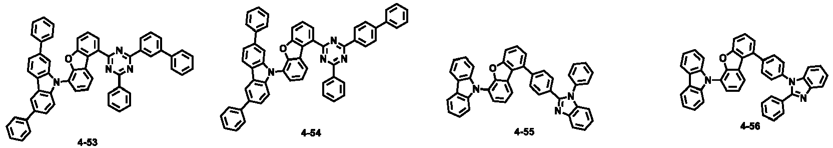

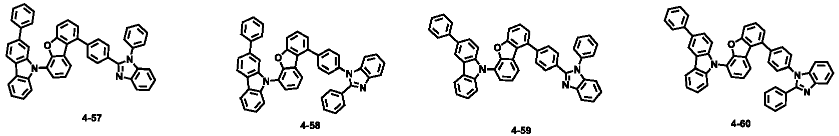

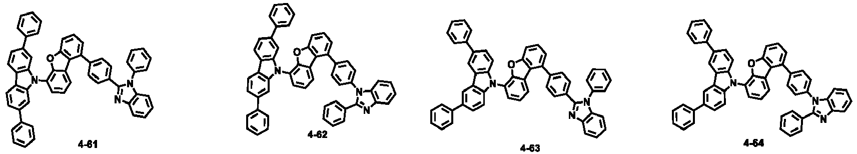

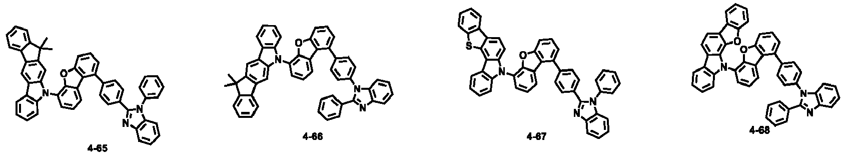

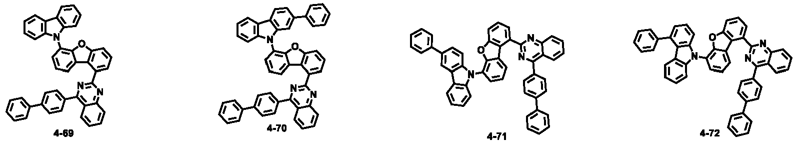

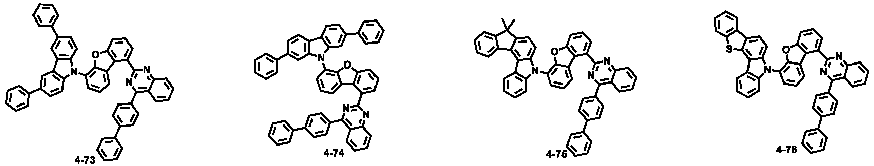

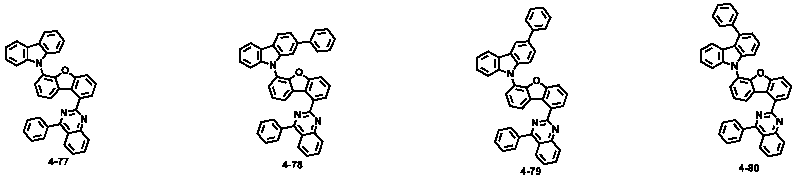

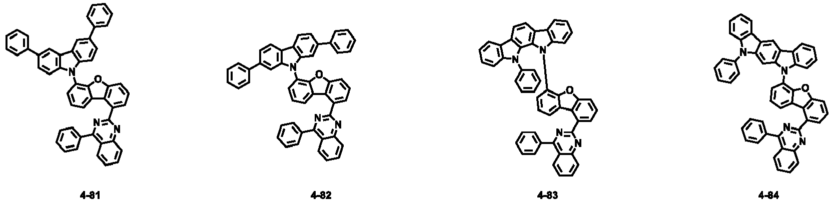

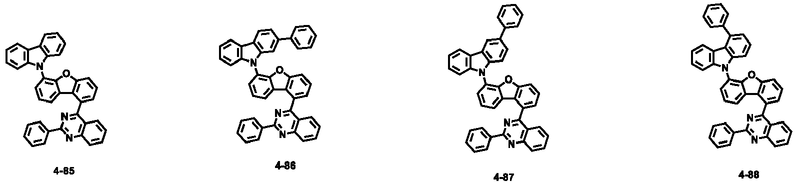

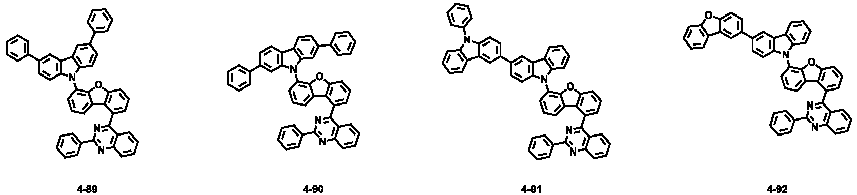

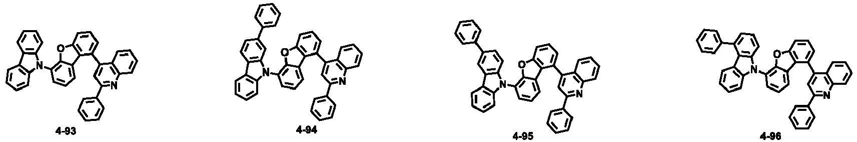

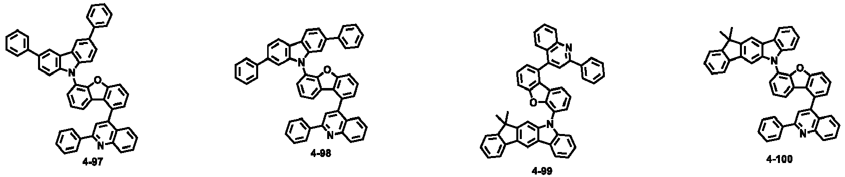

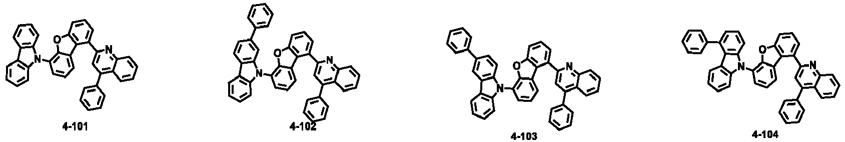

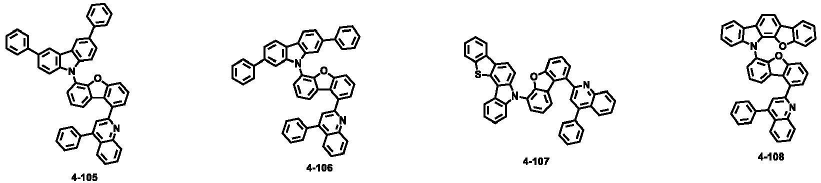

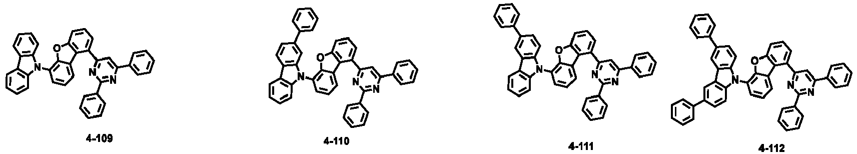

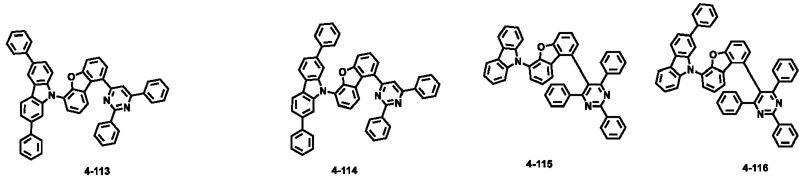

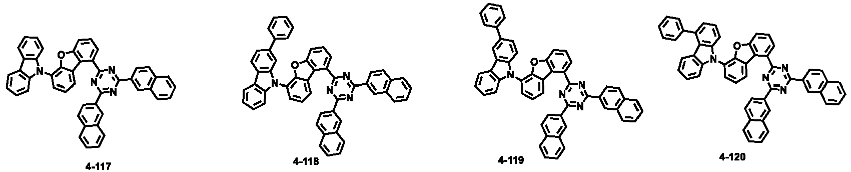

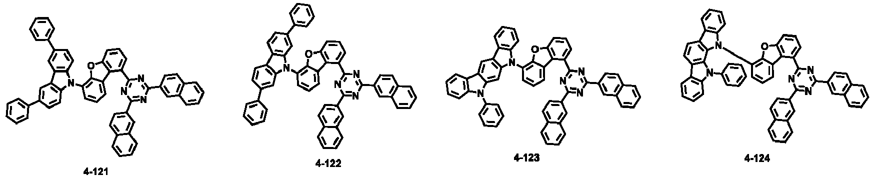

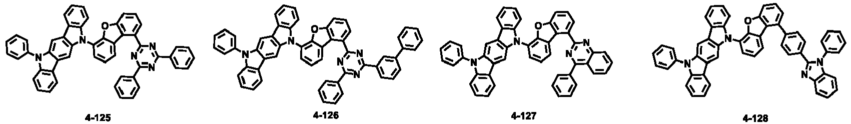

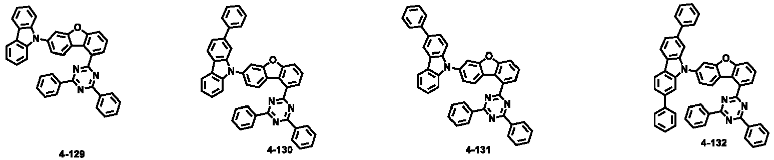

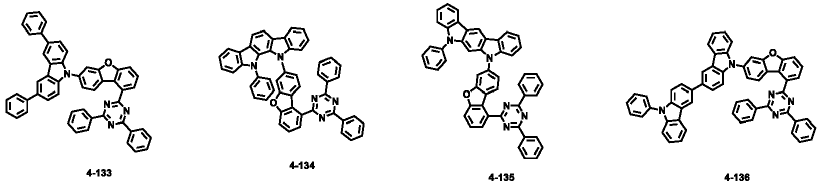

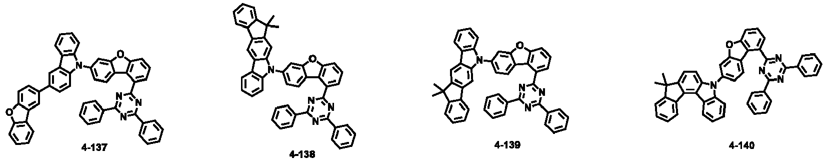

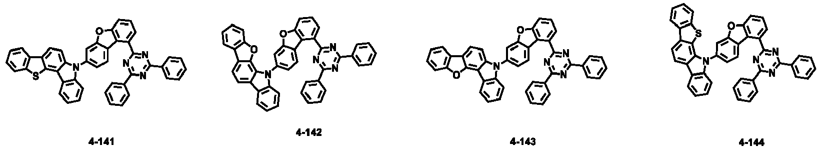

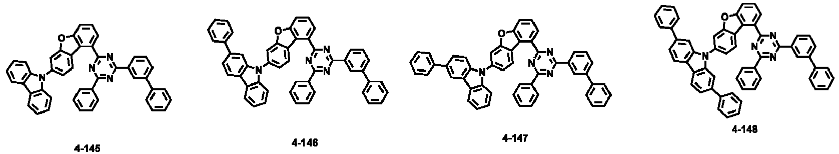

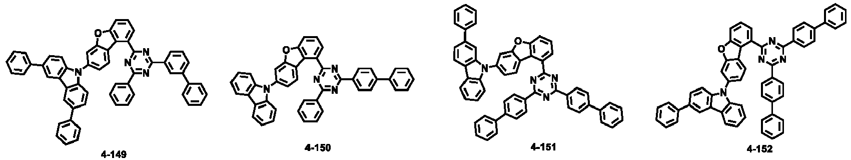

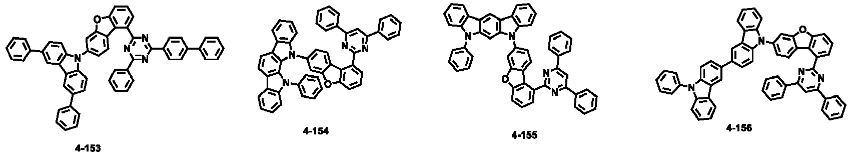

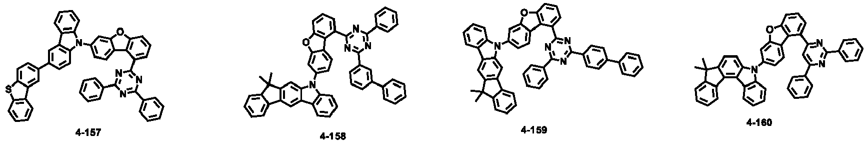

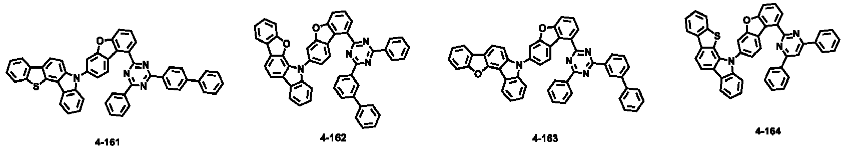

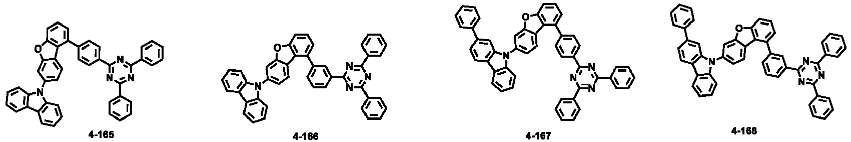

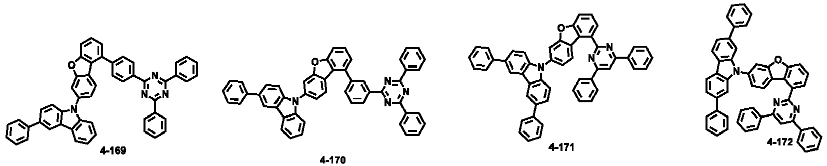

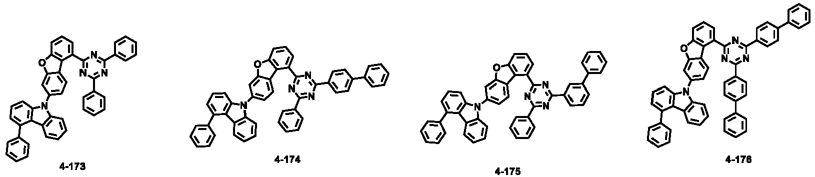

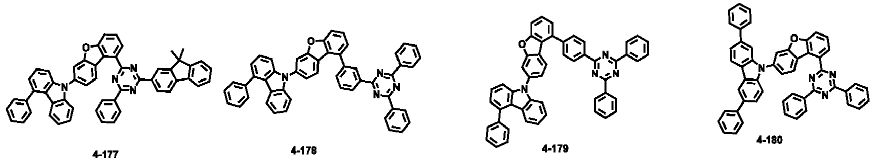

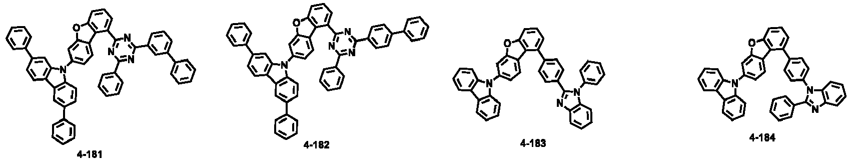

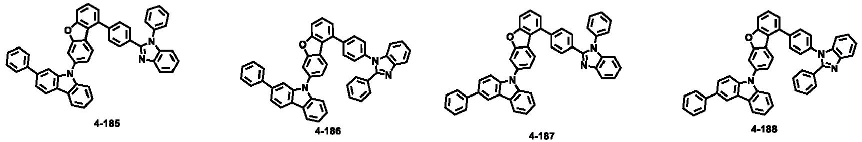

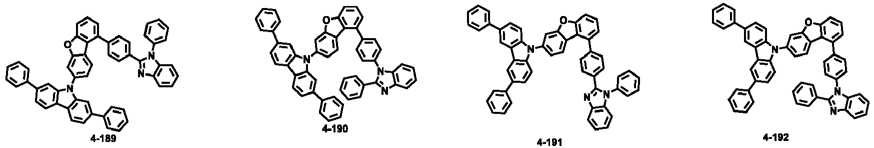

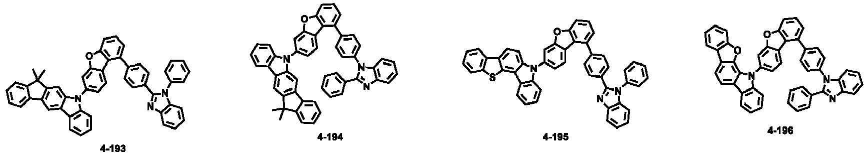

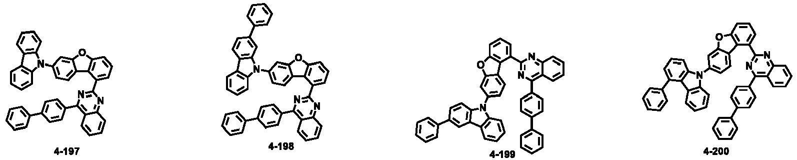

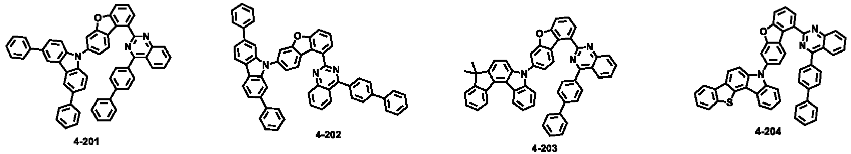

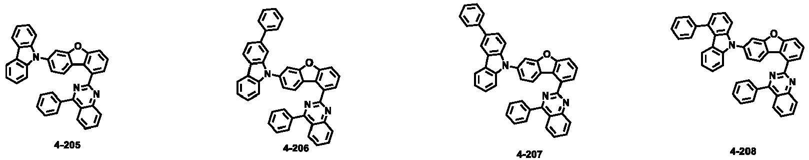

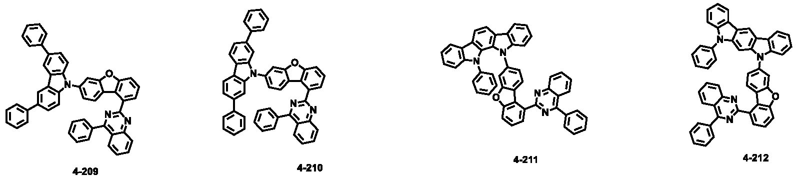

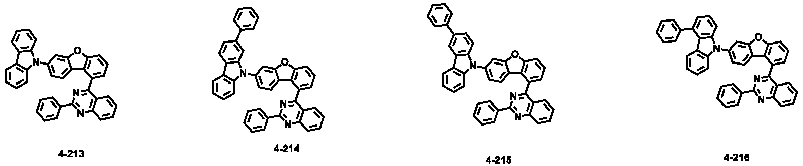

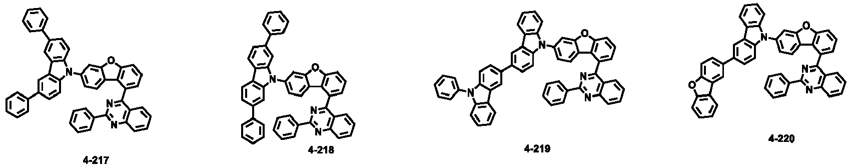

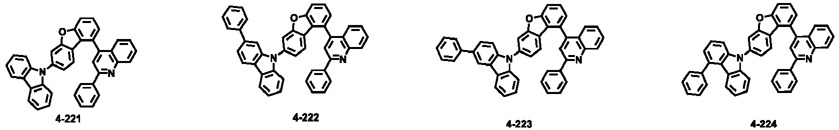

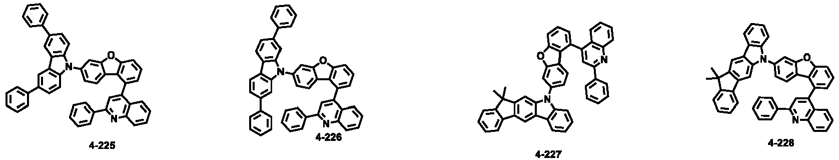

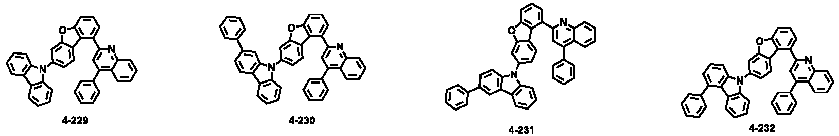

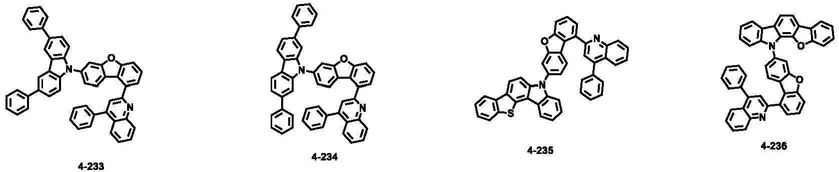

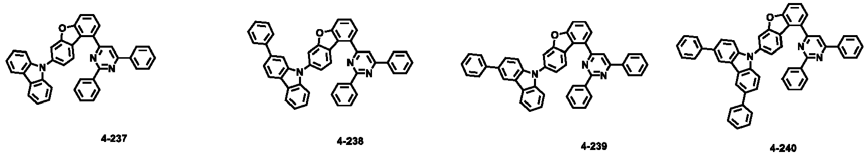

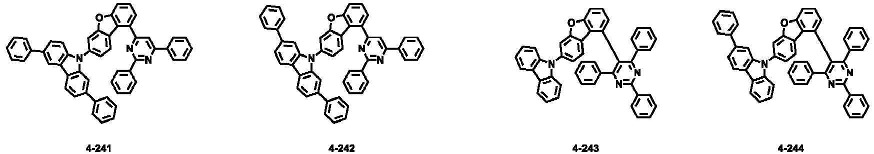

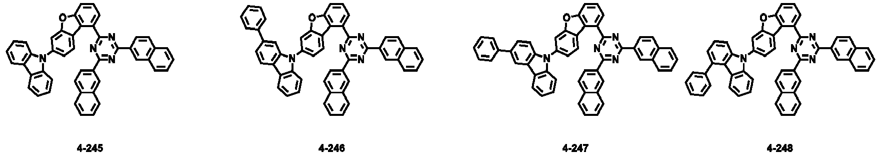

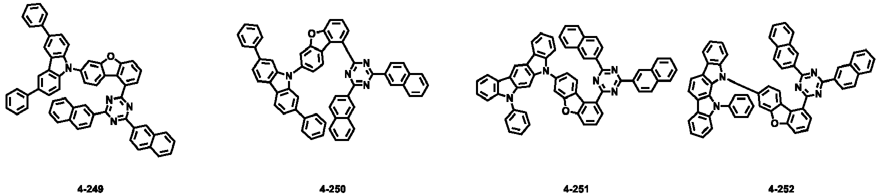

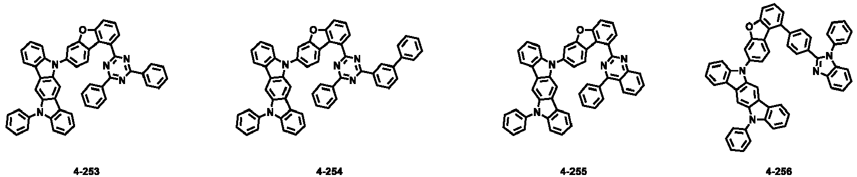

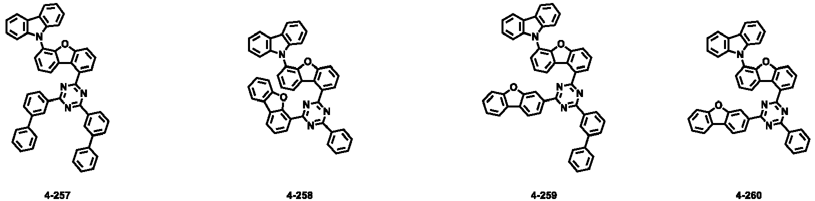

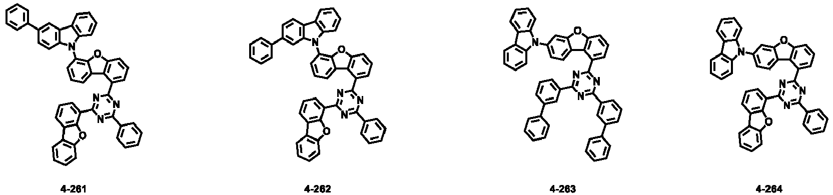

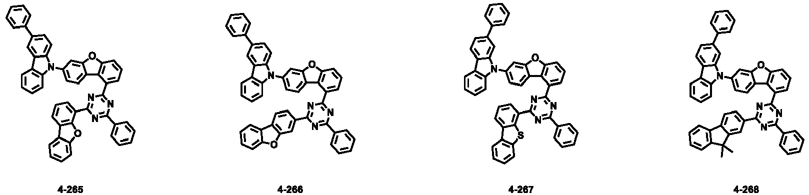

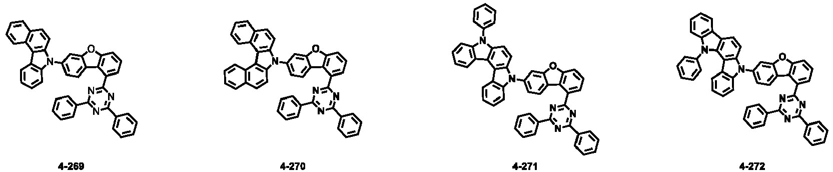

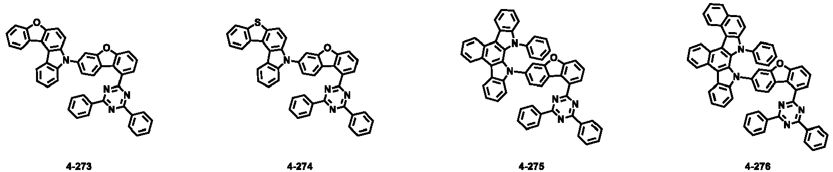

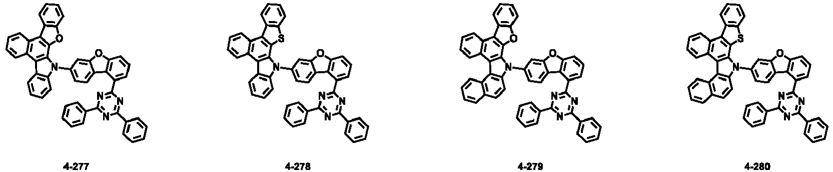

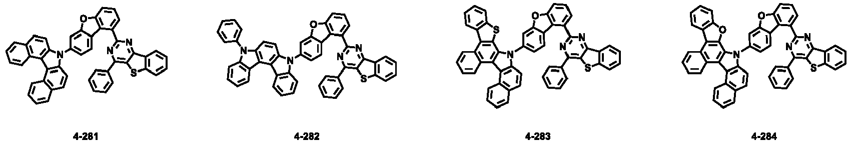

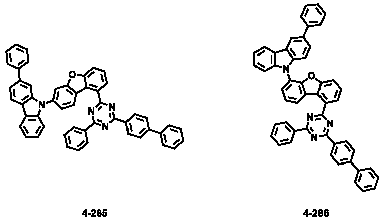

In the organic light emitting device according to one embodiment of the present application, the heterocyclic compound represented by Chemical Formula 2 or Chemical Formula 3 may be included in a light emitting layer of the organic material layer.

In the organic light emitting device according to one embodiment of the present application, the heterocyclic compound represented by Chemical Formula 2 or Chemical Formula 3 may be included in a light emitting layer of the organic material layer, and may specifically be used as a host material of the light emitting layer.

In one embodiment of the present application, the host material of the light emitting layer of the organic light emitting device may include the heterocyclic compound of Chemical Formula 1; and the heterocyclic compound of Chemical Formula 2 or the heterocyclic compound of Chemical Formula 3 at the same time.

One embodiment of the present application provides a composition for an organic material layer of an organic light emitting device, the composition including the heterocyclic compound represented by Chemical Formula 1; and the heterocyclic compound of Chemical Formula 2 or the heterocyclic compound of Chemical Formula 3.

In the composition, the heterocyclic compound represented by Chemical Formula 1:the heterocyclic compound of Chemical Formula 2 or the heterocyclic compound of Chemical Formula 3 may have a weight ratio of 1:10 to 10:1, 1:8 to 8:1, 1:5 to 5:1 or 1:2 to 2:1, however, the weight ratio is not limited thereto.

One embodiment of the present application provides a method for manufacturing an organic light emitting device, the method including preparing a substrate; forming a first electrode on the substrate; forming one or more organic material layers on the first electrode; and forming a second electrode on the organic material layers, wherein the forming of organic material layers includes forming one or more organic material layers using the composition for an organic material layer according to one embodiment of the present application.

In the method for manufacturing an organic light emitting device provided in one embodiment of the present application, the forming of organic material layers is forming using a thermal vacuum deposition method after pre-mixing the heterocyclic compound represented by Chemical Formula 1 and the heterocyclic compound represented by Chemical Formula 2.

The pre-mixing means first mixing the heterocyclic compound represented by Chemical Formula 1 and the heterocyclic compound represented by Chemical Formula 2 in one source of supply before depositing on the organic material layer.

The pre-mixing means first mixing the heterocyclic compound represented by Chemical Formula 1 and the heterocyclic compound represented by Chemical Formula 3 in one source of supply before depositing on the organic material layer.

The pre-mixed material may be referred to as the composition for an organic material layer according to one embodiment of the present application.

In the organic light emitting device according to one embodiment of the present application, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material include metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material include metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being pre-mixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device including an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 1-1

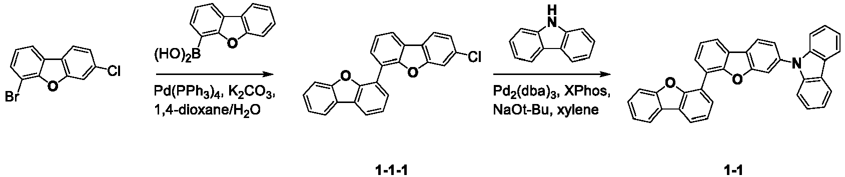

### 1) Preparation of Compound 1-1-1

6-Bromo-3-chlorodibenzo[b,d]furan (14.6 g, 51.9 mM), dibenzo[b,d]furan-4-ylboronic acid (12.1 g, 57.1 mM), tetrakis(triphenylphosphine)palladium(0) (Pd(PPh₃)₄) (3.0 g, 2.6 mM) and K₂CO₃ (14.5 g, 105.0 mM) were dissolved in 1,4-dioxane/H₂O (400 mL/80 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:hexane=1:5), and recrystallized with methanol to obtain target Compound 1-1-1 (15.3 g, 80%).

### 2) Preparation of Compound 1-1

Compound 1-1-1 (13.8 g, 37.4 mM), 9H-carbazole (6.9 g, 41.1 mM), tris(dibenzylideneacetone)dipalladium (Pd₂(dba)₃) (1.7 g, 1.9 mM), dicyclohexyl(2',4',6'-triisopropyl-[1,1'-biphenyl]-2-yl)phosphine (Xphos) (1.8 g, 3.8 mM) and sodium t-butoxide (NaOt-Bu) (7.2 g, 74.8 mM) were dissolved in xylene (400 mL), and refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 1-1 (15.9 g, 85%).

The following target compounds were synthesized in the same manner as in Preparation Example 1 except that Intermediate A of the following Table 1 was used instead of 6-bromo-3-chlorodibenzo[b,d]furan, Intermediate B of the following Table 1 was used instead of dibenzo[b,d]furan-4-ylboronic acid, and Intermediate C of the following Table 1 was used instead of 9H-carbazole.

**[Table 1]**

| Compoun d No. | Intermediate A | Intermediate B | Intermediate C | Target Compound |
|---|---|---|---|---|
| 1-2 | 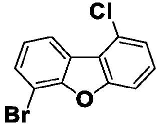 | 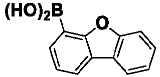 | 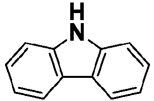 | 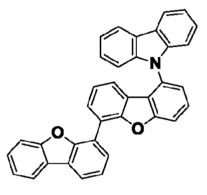 |
| 1-3 | 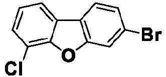 | | | 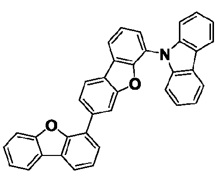 |
| 1-4 | 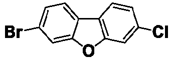 | | | 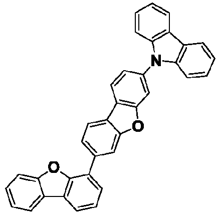 |
| 1-5 | 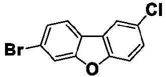 | | | 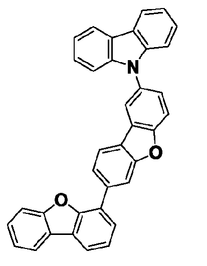 |
| 1-6 | 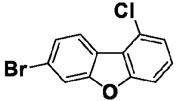 | | | 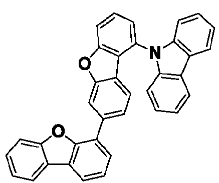 |
| 1-7 | 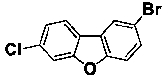 | | | 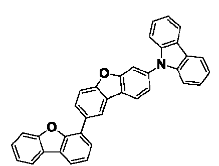 |
| 1-8 | 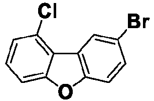 | | | 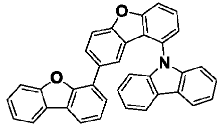 |
| 1-9 | 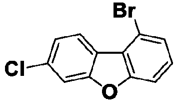 | | | 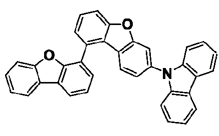 |
| 1-10 | 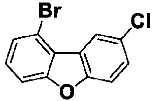 | | | 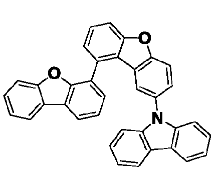 |
| 1-11 | 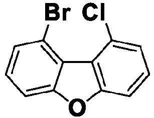 | | | 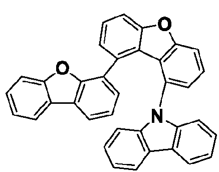 |
| 1-15 | 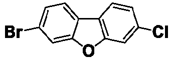 | 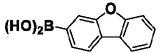 | | 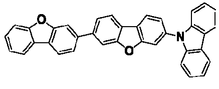 |
| 1-21 | 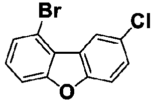 | | | 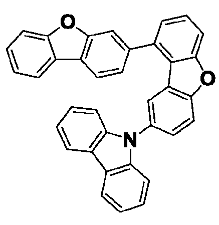 |
| 1-33 | 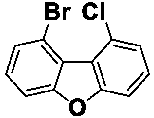 | 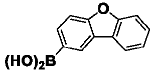 | | 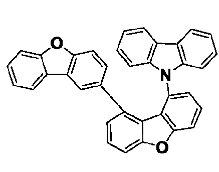 |
| 1-43 | 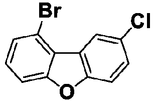 | 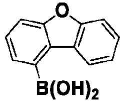 | | 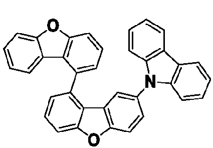 |
| 1-48 | 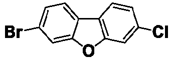 | 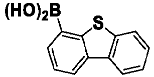 | | 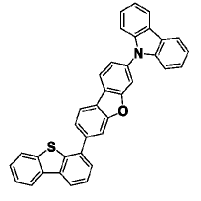 |
| 1-49 | 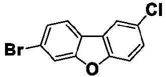 | | | 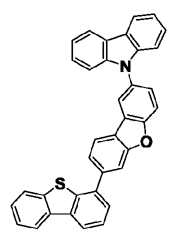 |
| 1-53 | 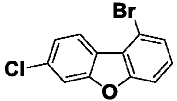 | | | 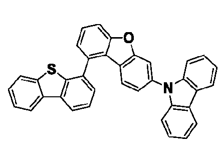 |
| 1-54 | 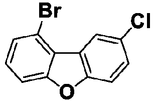 | | | 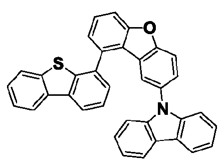 |
| 1-240 | 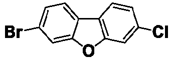 | 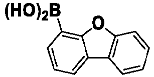 | 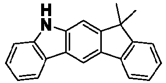 | 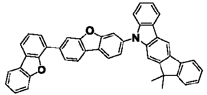 |
| 1-241 | 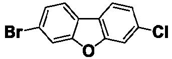 | 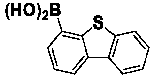 | 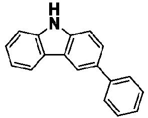 | 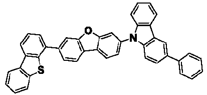 |
| 1-250 | 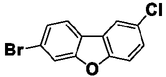 | | 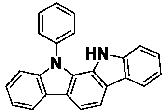 | 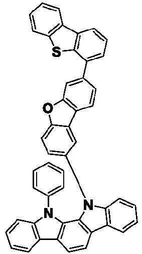 |
| 1-252 | 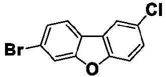 | | 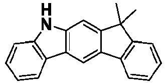 | 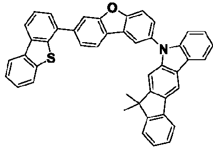 |
| 1-264 | 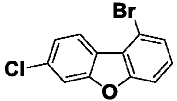 | 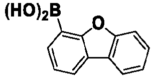 | | 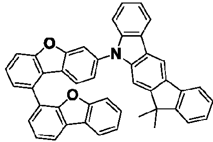 |
| 1-265 | 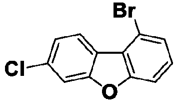 | 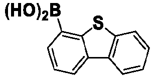 | 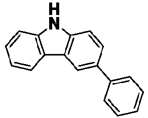 | 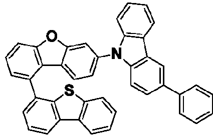 |
| 1-275 | 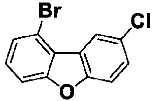 | 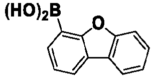 | 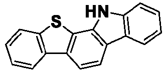 | 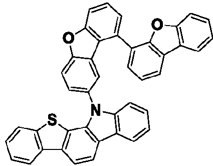 |
| 1-276 | 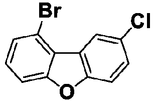 | 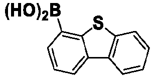 | 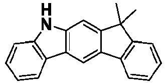 | 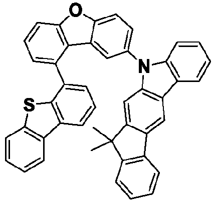 |
| 1-299 | 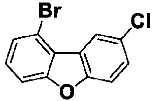 | 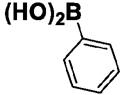 | | 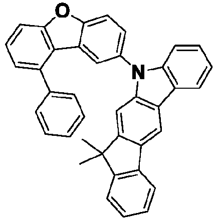 |
| 2-10 | 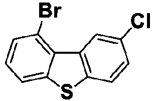 | 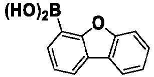 | 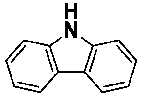 | 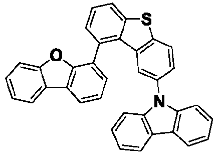 |
| 2-48 | 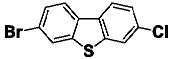 | 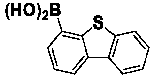 | | 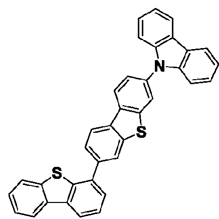 |
| 2-240 | 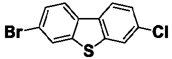 | 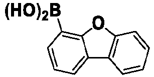 | 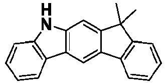 | 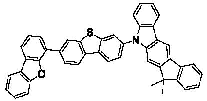 |
| 2-275 | 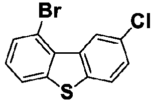 | | 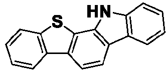 | 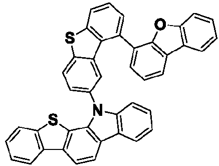 |
| 2-276 | 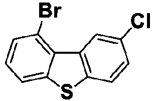 | 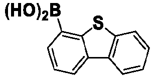 | 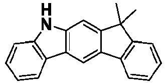 | 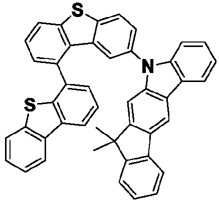 |

### <Preparation Example 2> Preparation of Compound 3-191

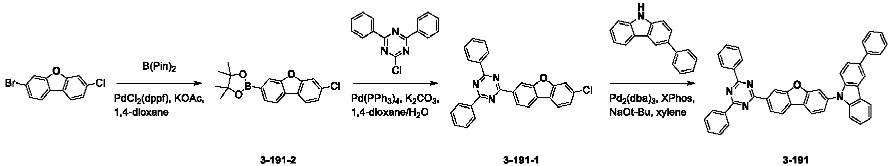

### 1) Preparation of Compound 3-191-2

3-Bromo-7-chlorodibenzo[b,d]furan (14.6 g, 51.9 mM), B(Pin)₂ (17.1 g, 67.5 mM), PdCl₂(dppf) (3.8 g, 5.2mM) and KOAc (15.3 g, 155.7 mM) were dissolved in 1,4-dioxane (300 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:hexane=1:2), and recrystallized with methanol to obtain target Compound 3-191-2 (14.2 g, 83%).

### 2) Preparation of Compound 3-191-1

Compound 3-191-2 (17.1 g, 51.9 mM), 2-chloro-4,6-diphenyl-1,3,5-triazine (15.3 g, 57.1 mM), tetrakis(triphenylhosphine)palladium(0) (Pd(PPh₃)₄) (3.0 g, 2.6 mM) and K₂CO₃ (14.5 g, 105.0 mM) were dissolved in 1,4-dioxane/H₂O (400 mL/80 mL), and refluxed for 24 hours. After the reaction was completed, the result was extracted by introducing distilled water and dichloromethane (DCM) thereto at room temperature, and after drying the organic layer with MgSO₄, the solvent was removed using a rotary evaporator. The reaction material was purified by column chromatography (DCM:hexane=1:3), and recrystallized with methanol to obtain target Compound 3-191-1 (18.0 g, 80%).

### 3) Preparation of Compound 3-191

Compound 3-191-1 (16.2 g, 37.4 mM), 9H-carbazole (6.9 g, 41.1 mM), tris(dibenzylideneacetone)dipalladium (Pd₂(dba)₃) (1.7 g, 1.9 mM), dicyclohexyl(2',4',6'-triisopropyl-[1,1'-biphenyl]-2-yl)phosphine (Xphos) (1.8 g, 3.8 mM) and sodium t-butoxide (NaOt-Bu) (7.2 g, 74.8 mM) were dissolved in xylene (400 mL), and refluxed for 24 hours. After the reaction was completed, the result was extracted by introducing distilled water and DCM thereto at room temperature, and after drying the organic layer with MgSO₄, the solvent was removed using a rotary evaporator. The reaction material was purified by column chromatography (DCM:hexane=1:3), and recrystallized with methanol to obtain target Compound 3-191 (20.4 g, 85%).

The following target compounds were synthesized in the same manner as in Preparation Example 2 except that Intermediate A of the following Table 2 was used instead of 3-bromo-7-chlorodibenzo[b,d]furan, and Intermediate B of the following Table 2 was used instead of 9H-carbazole.

**[Table 2]**

| Compound No. | Intermediate A | Intermediate B | Target Compound |
|---|---|---|---|
| 4-129 | 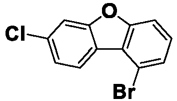 | 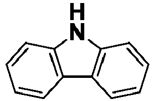 | 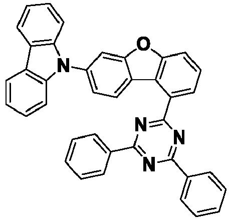 |
| 4-144 | | 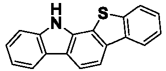 | 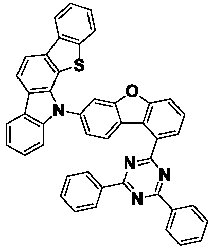 |

The rest of the compounds other the compounds described in Preparation Examples 1 and 2, and Tables 1 and 2 were also prepared in the same manner as in the preparation examples described above.

Synthesis identification data for the compounds prepared above are as described in the following [Table 3] and [Table 4] .

**[Table 3]**

| Compound No. | ¹H NMR (CDCl₃, 200 Mz) |
|---|---|
| 1-1 | δ=8.55 (1H, d), 8.19 (1H, d), 8.08-7.94 (7H, m), 7.58-7.50 (6H, m), 7.39-7.16 (6H, m) |
| 1-2 | δ=8.55 (1H, d), 8.19 (1H, d), 8.08-7.94 (6H, m), 7.58-7.50 (7H, m), 7.39-7.16 (6H, m) |
| 1-3 | δ=8.55 (1H, d), 8.19 (1H, d), 8.08-7.94 (6H, m), 7.82 (1H, d), 7.76 (1H, s), 7.58-7.31 (9H, m), 7.20-7.16 (2H, m) |
| 1-4 | δ=8.55 (1H, d), 8.19 (1H, d), 8.08-7.94 (6H, m), 7.82 (1H, d), 7.76 (1H, s), 7.58-7.50 (5H, m), 7.39-7.16 (6H, m) |
| 1-5 | δ=8.55 (1H, d), 8.19 (1H, d), 8.08-7.94 (4H, m), 7.82-7.74 (3H, m), 7.61-7.16 (11H, m) |
| 1-6 | δ=8.55 (1H, d), 8.19 (1H, d), 8.08-7.94 (5H, m), 7.82-7.76 (2H, m), 7.58-7.50 (6H, m), 7.39-7.16 (6H, m) |
| 1-7 | δ=8.55 (1H, d), 8.19 (1H, d), 8.08-7.79 (8H, m), 7.54-7.50 (5H, m), 7.39-7.16 (6H, m) |
| 1-8 | δ=8.55 (1H, d), 8.19 (1H, d), 8.08-7.79 (7H, m), 7.58-7.50 (6H, m), 7.39-7.16 (6H, m) |
| 1-9 | δ=8.55 (1H, d), 8.19 (1H, d), 8.08-7.94 (5H, m), 7.82 (1H, d), 7.69 (1H, d), 7.58-7.50 (6H, m), 7.39-7.16 (6H, m) |
| 1-10 | δ=8.55 (1H, d), 8.19 (1H, d), 8.08-7.94 (4H, m), 7.82-7.16 (15H, m) |
| 1-11 | δ=8.55 (1H, d), 8.19 (1H, d), 8.08-7.94 (4H, m), 7.82 (1H, d), 7.69 (1H, d), 7.57-7.50 (7H, m), 7.39-7.16 (6H, m) |
| 1-15 | δ=8.55 (1H, d), 8.19 (1H, d), 8.03-7.94 (5H, m), 7.82-7.76 (4H, m), 7.58-7.50 (4H, m), 7.39-7.16 (6H, m) |
| 1-21 | δ=8.55 (1H, d), 8.19 (1H, d), 8.03-7.94 (3H, m), 7.82-7.50 (10H, |
| | m), 7.39-7.31 (4H, m), 7.20-7.16 (2H, m) |
| 1-33 | δ=8.55 (1H, d), 8.19 (1H, d), 7.98-7.79 (6H, m), 7.69 (1H, d), 7.58-7.52 (6H, m), 7.39-7.16 (6H, m) |
| 1-43 | δ=8.55 (1H, d), 8.19 (1H, d), 7.98-7.94 (2H, m), 7.82-7.50 (11H, m), 7.39-7.31 (4H, m), 7.20-7.16 (2H, m) |
| 1-48 | δ=8.55 (1H, d), 8.45 (1H, d), 8.32 (1H, d), 8.19 (1H, d), 8.03-7.93 (4H, m), 7.82-7.70 (3H, m), 7.58-7.49 (5H, m), 7.35 (1H, t), 7.25-7.16 (3H, m) |
| 1-49 | δ=8.55 (1H, d), 8.45 (1H, d), 8.32 (1H, d), 8.19 (1H, d), 8.03-7.93 (3H, m), 7.74-7.49 (9H, m), 7.35-7.31 (2H, m), 7.20-7.16 (2H, m) |
| 1-53 | δ=8.55 (2H, d), 8.45 (1H, d), 8.32 (1H, d), 8.19 (1H, d), 7.98-7.93 (3H, m), 7.82 (1H, d), 7.70-7.69 (2H, m), 7.58-7.49 (6H, m), 7.35 (1H, t), 7.25-7.16 (3H, m) |
| 1-54 | δ=8.55 (2H, d), 8.45 (1H, d), 8.32 (1H, d), 8.19 (1H, d), 7.98 (1H, d), 7.94-7.49 (10H, m), 7.35-7.31 (2H, m), 7.20-7.16 (2H, m) |
| 1-240 | δ=8.55 (1H, d), 8.24 (1H, d), 8.08-7.94 (6H, m), 7.88 (1H, s), 7.82-7.74 (3H, m), 7.57-7.49 (5H, m), 7.39-7.25 (5H, m), 7.16 (1H, t), 1.69 (6H, s) |
| 1-241 | δ=8.55 (2H, d), 8.45 (1H, d), 8.32 (1H, d), 8.03-7.70 (12H, m), 7.56-7.35 (7H, m), 7.25 (1H, d), 7.16 (1H, t) |
| 1-250 | δ=8.55 (3H, d), 8.45 (1H, d), 8.32 (1H, d), 8.12 (1H, d), 8.03 (1H, d), 7.94-7.93 (3H, m), 7.82-7.49 (13H, m), 7.35-7.31 (3H, m), 7.16 (2H, t) |
| 1-252 | δ=8.55 (2H, d), 8.45 (1H, d), 8.32 (1H, d), 8.24 (1H, d), 8.03 (1H, d), 7.94-7.88 (3H, m), 7.82-7.70 (5H, m), 7.61-7.49 (5H, m), 7.38-7.31 (3H, m), 7.16 (1H, t), 1.69 (6H, s) |
| 1-264 | δ=8.55 (1H, d), 8.24 (1H, d), 8.08-7.94 (5H, m), 7.88 (1H, s), 7.82 (1H, d), 7.74-7.69 (2H, m), 7.57-7.49 (6H, m), 7.39-7.25 (5H, m), 1.69 (6H, s) |
| 1-265 | δ=8.55 (1H, d), 8.45 (1H, d), 8.32 (1H, d), 7.99-7.69 (11H, m), 7.57-7.35 (8H, m), 7.25-7.16 (2H, m) |
| 1-275 | δ=8.55 (1H, d), 8.45 (1H, d), 8.08-7.93 (6H, m), 7.82-7.31 (14H, m), 7.16 (1H, t) |
| 1-276 | δ=8.55 (2H, d), 8.45 (1H, d), 8.32 (1H, d), 8.24 (1H, d), 7.94-7.88 (3H, m), 7.74-7.69 (4H, m), 7.61-7.49 (6H, m), 7.38-7.31 (3H, m), 7.16 (1H, t), 1.69 (6H, s) |
| 1-299 | δ=8.55 (1H, d), 8.24 (1H, d), 7.94 (1H, d), 7.88 (1H, s), 7.82-7.69 (6H, m), 7.61-7.57 (3H, m), 7.49-7.31 (7H, m), 7.16 (1H, t) |
| 2-10 | δ=8.55 (1H, d), 8.19-7.94 (9H, m), 7.68 (1H, t), 7.58-7.31 (8H, m), |
| | 7.20-7.16 (2H, m) |
| 2-48 | δ=8.55 (2H, d), 8.45 (1H, d), 8.32 (1H, d), 8.24-8.17 (5H, m), 7.98-7.93 (3H, m), 7.70 (1H, t), 7.58-7.45 (5H, m), 7.35 (1H, t), 7.20-7.16 (2H, m) |
| 2-240 | δ=8.55 (1H, d), 8.24-8.17 (5H, m), 8.08-7.94 (5H, m), 7.88 (1H, s), 7.74 (1H, d), 7.57-7.31 (9H, m), 7.16 (1H, t), 1.69 (6H, s) |
| 2-275 | δ=8.55 (1H, d), 8.45 (1H, d), 8.10-7.90 (10H, m), 7.68-7.31 (10H, m), 7.16 (1H, t) |
| 2-276 | δ=8.55 (2H, d), 8.45 (1H, d), 8.32 (1H, d), 8.24 (1H, d), 8.10-8.03 (2H, m), 7.94-7.88 (5H, m), 7.74-7.68 (3H, m), 7.57-7.38 (7H, m), 7.16 (1H, t), 1.69 (6H, s) |
| 3-191 | δ=8.55 (1H, d), 8.36 (4H, d), 8.03-7.89 (5H, m), 7.86-7.75 (5H, m), 7.54-7.35 (11H, m), 7.25 (1H, d), 7.16 (1H, t) |
| 4-129 | δ=8.55 (1H, d), 8.36 (4H, d), 8.19 (1H, d), 7.98-7.94 (2H, m), 7.82 (1H, d), 7.69 (1H, d), 7.58-7.50 (10H, m), 7.35 (1H, t), 7.25-7.16 (3H, t) |
| 4-144 | δ=8.55 (1H, d), 8.45 (1H, d), 8.36 (4H, d), 8.05 (1H, d), 7.98-7.93 (3H, m), 7.82 (1H, d), 7.69 (1H, d), 7.60-7.49 (11H, m), 7.35 (1H, t), 7.25 (1H, d),7.16 (1H, t) |

**[Table 4]**

| Compound | FD-MS | Compound | FD-MS |
|---|---|---|---|
| 1-1 | m/z=499.16 (C₃₆H₂₁NO₂=499.57) | 1-2 | m/z=499.16 (C₃₆H₂₁NO₂=499.57) |
| 1-3 | m/z=499.16 (C₃₆H₂₁NO₂=499.57) | 1-4 | m/z=499.16 (C₃₆H₂₁NO₂=499.57) |
| 1-5 | m/z=499.16 (C₃₆H₂₁NO₂=499.57) | 1-6 | m/z=499.16 (C₃₆H₂₁NO₂=499.57) |
| 1-7 | m/z=499.16 (C₃₆H₂₁NO₂=499.57) | 1-8 | m/z=499.16 (C₃₆H₂₁NO₂=499.57) |
| 1-9 | m/z=499.16 (C₃₆H₂₁NO₂=499.57) | 1-10 | m/z=499.16 (C₃₆H₂₁NO₂=499.57) |
| 1-11 | m/z=499.16 (C₃₆H₂₁NO₂=499.57) | 1-15 | m/z=499.16 (C₃₆H₂₁NO₂=499.57) |
| 1-21 | m/z=499.16 (C₃₆H₂₁NO₂=499.57) | 1-33 | m/z=499.16 (C₃₆H₂₁NO₂=499.57) |
| 1-43 | m/z=499.16 (C₃₆H₂₁NO₂=499.57) | 1-48 | m/z=515.13 (C₃₆H₂₁NOS=515.63) |
| 1-49 | m/z=515.13 (C₃₆H₂₁NOS=515.63) | 1-53 | m/z=515.13 (C₃₆H₂₁NOS=515.63) |
| 1-54 | m/z=515.13 (C₃₆H₂₁NOS=515.63) | 1-240 | m/z=615.22 (C₄₅H₂₉NO₂=615.73) |
| 1-241 | m/z=591.17 (C₄₅H₂₅NOS=591.73) | 1-250 | m/z=680.19 (C₄₈H₂₈N₂OS=680.83) |
| 1-252 | m/z=631.20 (C₄₅H₂₉NOS=631.79) | 1-264 | m/z=615.22 (C₄₅H₂₉NO₂=615.73) |
| 1-265 | m/z=591.17 (C₄₅H₂₅NOS=591.73) | 1-275 | m/z=605.14 (C₄₅H₂₃NO₂S=605.71) |
| 1-276 | m/z=631. 20 (C₄₅H₂₉NOS=631.79) | 1-299 | m/z=525.21 (C₃₉H₂₇NO=525.65) |
| 2-10 | m/z=515.13 (C₃₆H₂₁NOS=515.63) | 2-48 | m/z=531.11 (C₃₆H₂₁NS₂=531.69) |
| 2-240 | m/z=631.20 (C₄₅H₂₉NOS=631.79) | 2-275 | m/z=621.12 (C₄₂H₂₃NOS₂=621.77) |
| 2-276 | m/z=647.17 (C₄₅H₂₉NS₂=647.85) | 3-191 | m/z=640.23 (C₄₅H₂₈N₄O=640.75) |
| 4-129 | m/z=564.20 (C₃₉H₂₄N₄O=564.65) | 4-144 | m/z=670.18 (C₄₅H₂₈N₄OS=670.79) |

### <Experimental Example 1>

### (1) Manufacture of Organic Light Emitting Device

A glass substrate on which ITO (indium tin oxide) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO (ultraviolet ozone) treatment was conducted for 5 minutes using UV in a UV (ultraviolet) cleaner. After that, the substrate was transferred to a plasma cleaner (PT), and after conducting plasma treatment under vacuum for increasing the ITO work function and removing the residual film, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, the compound of Chemical Formula 1 described in the following Table 5 was deposited to a thickness of 400 Å as a host, and a green phosphorescent dopant [Ir(ppy)₃] was doped and deposited by 7% of the deposited thickness of the light emitting layer. When manufacturing a blue phosphorescent device, a blue phosphorescent dopant [Firpic] was deposited. After that, BCP (bathocuproine) was deposited to a thickness of 60 Å as a hole blocking layer, and Alq₃ was deposited to a thickness of 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### (2) Driving Voltage and Light Emission Efficiency of Organic Light Emitting Device

For each of the organic light emitting devices manufactured as above, electroluminescent (EL) properties were measured using M7000 manufactured by McScience Inc., and with the measurement results, T₉₀ of the green phosphorescent device was measured when standard luminance was 6,000 cd/m² and T₉₀ of the blue phosphorescent device was measured when standard luminance was 1,000 cd/m² through a lifetime measurement system (M6000) manufactured by McScience Inc.. Herein, T₉₀ means a lifetime (unit: h, hour), time taken to become 90% with respect to initial luminance.

Results of measuring driving voltage, light emission efficiency, color (EL color) and lifetime of the organic light emitting devices manufactured according to the present disclosure are as shown in the following Table 5.

**[Table 5]**

| | Light Emitting Layer Compound | Driving Voltage (V) | Efficiency (cd/A) | Color EL color | Lifetime (T₉₀) |
|---|---|---|---|---|---|
| Example 1 | 1-1 | 5.85 | 11.8 | Blue | 139 |
| Example 2 | 1-2 | 5.37 | 12.5 | | 146 |
| Example 3 | 1-3 | 5.33 | 12.2 | | 141 |
| Example 4 | 1-4 | 5.42 | 12.7 | | 159 |
| Example 5 | 1-5 | 5.42 | 12.1 | | 155 |
| Example 6 | 1-6 | 5.03 | 12.7 | | 150 |
| Example 7 | 1-7 | 5.66 | 12.8 | | 127 |
| Example 8 | 1-8 | 5.47 | 11.2 | | 120 |
| Example 9 | 1-9 | 5.52 | 12.8 | | 157 |
| Example 10 | 1-10 | 5.21 | 13.0 | | 152 |
| Example 11 | 1-11 | 5.42 | 12.7 | | 115 |
| Example 12 | 1-15 | 5.66 | 11.1 | | 130 |
| Example 13 | 1-21 | 5.33 | 12.2 | | 134 |
| Example 14 | 1-33 | 5.48 | 10.2 | | 111 |
| Example 15 | 1-43 | 5.69 | 12.2 | | 128 |
| Example 16 | 1-48 | 5.35 | 12.2 | | 102 |
| Example 17 | 1-49 | 5.41 | 12.8 | | 91 |
| Example 18 | 1-53 | 5.67 | 11.2 | | 94 |
| Example 19 | 1-54 | 5.38 | 12.4 | | 105 |
| Example 20 | 1-240 | 4.67 | 72.5 | Green | 390 |
| Example 21 | 1-241 | 4.45 | 72.8 | | 370 |
| Example 22 | 1-250 | 4.66 | 73.5 | | 360 |
| Example 23 | 1-252 | 4.35 | 76.1 | | 382 |
| Example 24 | 1-264 | 4.67 | 71.5 | | 326 |
| Example 25 | 1-265 | 4.56 | 70.3 | | 352 |
| Example 26 | 1-275 | 4.33 | 74.4 | | 405 |
| Example 27 | 1-276 | 4.61 | 73.6 | | 317 |
| Example 28 | 1-299 | 4.32 | 71.5 | | 309 |
| Example 29 | 2-10 | 5.40 | 11.3 | Blue | 88 |
| Example 30 | 2-48 | 5.42 | 10.1 | | 75 |
| Example 31 | 2-240 | 4.67 | 72.9 | Green | 277 |
| Example 32 | 2-275 | 4.69 | 70.9 | | 271 |
| Example 33 | 2-276 | 4.60 | 71.1 | | 269 |
| Comparative Example 1 | Ref. 1 | 6.48 | 10.2 | Blue | 43 |
| Comparative Example 2 | Ref. 2 | 6.69 | 9.9 | | 31 |
| Comparative Example 3 | Ref. 3 | 6.35 | 10.2 | | 38 |
| Comparative | Ref. 4 | 6.41 | 10.8 | | 30 |
| Example 4 | | | | | |
| Comparative Example 5 | Ref. 5 | 6.48 | 10.3 | | 40 |

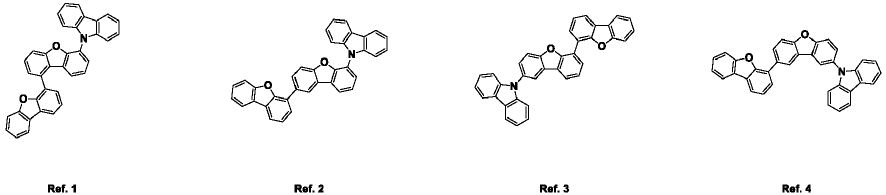

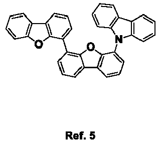

As seen from the results of Table 5, the organic electroluminescent device using the organic electroluminescent device light emitting layer material of the present disclosure had low driving voltage, enhanced light emission efficiency, and significantly improved lifetime as well compared to Comparative Examples 1 to 5.

Specifically, in the heterocyclic compound represented by Chemical Formula 1 according to the present disclosure, three or more aromatic rings bond through a single bond. By introducing at least one heteroring that pushes electrons, hole mobility and stability were enhanced, and device efficiency and lifetime were identified to be enhanced. The remaining two aromatic rings may adjust LUMO level, electron mobility and stability by delocalizing or localizing the LUMO.

In addition, it was identified that, by Compounds 1-3, 1-5 and 1-10, which are the heterocyclic compound represented by Chemical Formula 1 of the present disclosure, having a delocalized LUMO orbital and a low LUMO level compared to Ref. 1 to 5, electron stability and mobility were enhanced, and device efficiency and lifetime were enhanced by readily transferring electrons from the host to the dopant.

**[Table 6]**

| Compound | Structural Formula | LUMO Orbital |
|---|---|---|
| Ref. 1 | 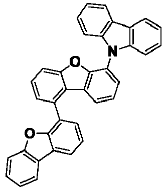 | 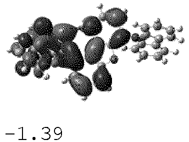 |
| Ref. 2 | 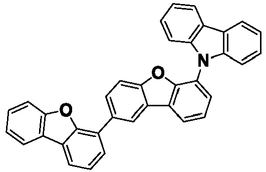 | 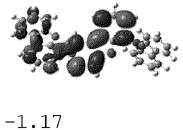 |
| Ref. 3 | 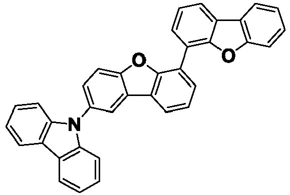 | 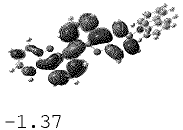 |
| Ref. 4 | 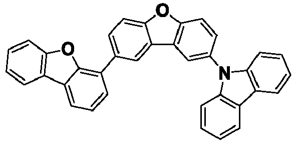 | 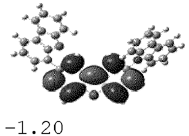 |
| Ref. 5 | 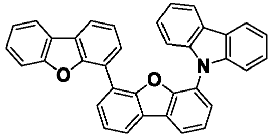 | 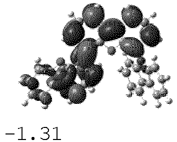 |
| 1-3 | 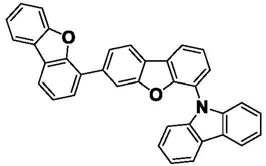 | 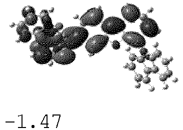 |
| 1-5 | 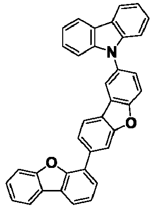 | 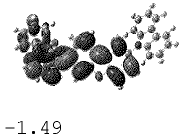 |
| 1-10 | 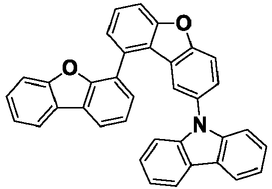 | 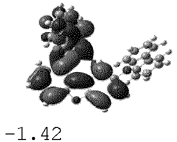 |

### <Experimental Example 2>

### (1) Manufacture of Organic Light Emitting Device

A glass substrate on which ITO (indium tin oxide) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO (ultraviolet ozone) treatment was conducted for 5 minutes using UV in a UV (ultraviolet) cleaner. After that, the substrate was transferred to a plasma cleaner (PT), and after conducting plasma treatment under vacuum for increasing the ITO work function and removing the residual film, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type (P-type) of the compound represented by Chemical Formula 1 of the present disclosure and one type (N-type) of the compound represented by Chemical Formula 2 or 3 of the present disclosure were premixed as described in the following Table 7 and then deposited to a thickness of 400 Å in one source of supply as a host, and a green phosphorescent dopant [Ir(ppy)₃] was doped and deposited by an amount of 7 wt% of the deposited thickness of the light emitting layer. When manufacturing a blue phosphorescent device, a blue phosphorescent dopant [Firpic] was deposited. After that, BCP (bathocuproine) was deposited to a thickness of 60 Å as a hole blocking layer, and Alq₃ was deposited to a thickness of 200 Å thereon as an electron transfer layer.

Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic light emitting device was manufactured.

Meanwhile, all the organic compounds required to manufacture the OLED were vacuum sublimation purified under 10⁻⁸ torr to 10⁻⁶ torr for each material to be used in the OLED manufacture.

### (2) Driving Voltage and Light Emission Efficiency of Organic Light Emitting Device

For each of the organic light emitting devices manufactured as above, electroluminescent (EL) properties were measured using M7000 manufactured by McScience Inc., and with the measurement results, T₉₀ of the green phosphorescent device was measured when standard luminance was 6,000 cd/m² and T₉₀ of the blue phosphorescent device was measured when standard luminance was 1,000 cd/m² through a lifetime measurement system (M6000) manufactured by McScience Inc.. Herein, T₉₀ means a lifetime (unit: h, hour), time taken to become 90% with respect to initial luminance.

Results of measuring driving voltage, light emission efficiency, color (EL color) and lifetime of the organic light emitting devices manufactured according to the present disclosure are as shown in the following Table 7.

**[Table 7]**

| | Light Emitting Layer Compound | Ratio | Driving Voltage (V) | Efficiency (cd/A) | Color EL color | Lifetime (T₉₀) |
|---|---|---|---|---|---|---|
| Example 17 | 4-129:1-10 | 1:8 | 4.73 | 10.8 | Blue | 116 |
| Example 18 | | 1:5 | 4.69 | 11.2 | | 174 |
| Example 19 | | 1:2 | 4.33 | 15.1 | | 269 |
| Example 20 | | 1:1 | 4.48 | 13.4 | | 243 |
| Example 21 | | 2:1 | 4.69 | 13.5 | | 218 |
| Example 22 | | 5:1 | 4.32 | 11.2 | | 181 |
| Example 23 | | 8:1 | 4.21 | 10.4 | | 136 |
| Example 24 | 3-191:2-240 | 1:2 | 4.33 | 74.2 | Green | 562 |
| Example 25 | | 1:1 | 4.42 | 72.2 | | 553 |
| Example 26 | | 2:1 | 4.66 | 71.2 | | 528 |
| Example 27 | 4-144:1-275 | 1:2 | 4.33 | 75.2 | | 620 |
| Example 28 | | 1:1 | 4.48 | 70.2 | | 582 |
| Example 29 | | 2:1 | 4.69 | 69.2 | | 543 |

As seen from the results of Table 7, it was identified that effects of more superior efficiency and lifetime were obtained when including the compound (P type) represented by Chemical Formula 1 of the present disclosure and the compound (N type) represented by Chemical Formula 2 or 3 of the present disclosure at the same time. This may lead to a forecast that an exciplex phenomenon occurs when including the two compounds at the same time.

The exciplex phenomenon is a phenomenon of releasing energy having sizes of a donor (p-host) HOMO energy level and an acceptor (n-host) LUMO energy level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime

In the present disclosure, it was identified that superior device properties were obtained when using the compound represented by Chemical Formula 1 performing a donor role and the compound represented by Chemical Formula 2 or the compound represented by Chemical Formula 3 performing an acceptor role as a host of the light emitting layer.

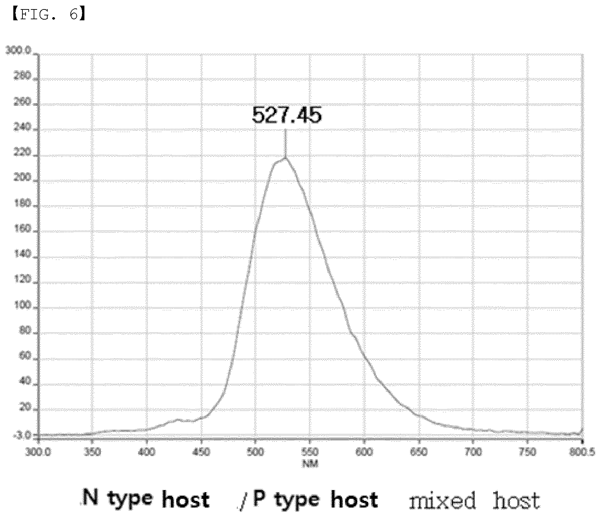

FIG. 5 is PL of Chemical Formula 1, the P type host of the present application, and it was identified that PL of Chemical Formula 1 of the present application appeared at from mid 300s to early 400s, and PL of Chemical Formula 2 or Chemical Formula 3, the N type host of the present application, appeared at from mid 400s to early 500s. Particularly, it was identified that the PL value was 527.45 in FIG. 6 when including the two compounds at the same time due to the occurrence of exciplex phenomenon.

## Claims

1. A heterocyclic compound represented by the following Chemical Formula 1: 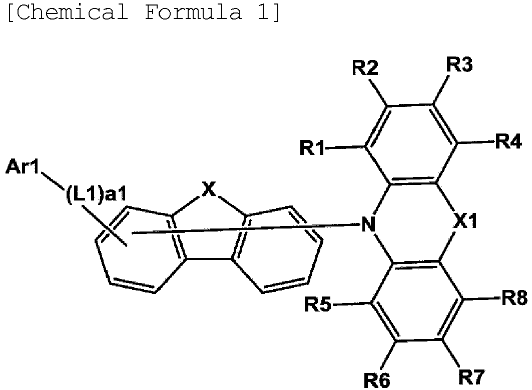 wherein, in Chemical Formula 1,
substitution positions of substituents of -(L1)a1-Ar1 and 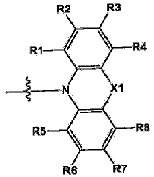
satisfy any one of the following Structural Formulae 1-A to 1-J, and in the following Structural Formulae 1-A to 1-J, * and ** mean positions linked to -(L1)a1-Ar1 and 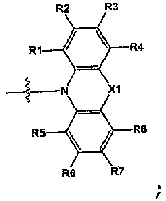 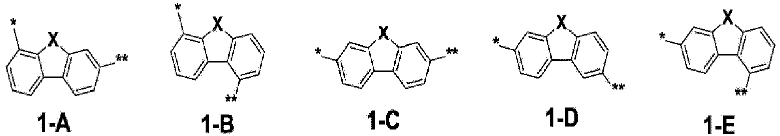 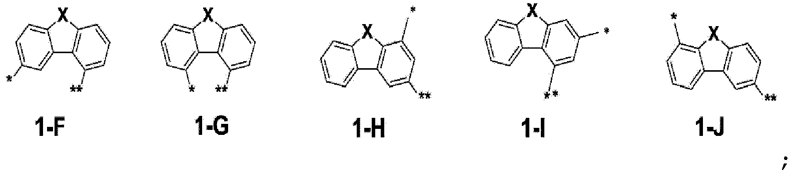
L1 is a direct bond; a substituted or unsubstituted C6 to C60 arylene group; or a substituted or unsubstituted C2 to C60 heteroarylene group;
X is O; or S;
X1 is a direct bond; O; S; NR; or CRR';
Ar1 is a C6 to C60 aryl group unsubstituted or substituted with deuterium, a C6 to C60 aryl group or a C1 to C60 alkyl group; or a C2 to C60 heteroaryl group unsubstituted or substituted with deuterium;
R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group including O or S as a heteroatom; -SiRR'R"; -P(=O)RR'; and - NRR', or two or more groups adjacent to each other bond to each other to form a substituted or unsubstituted C6 to C60 aromatic hydrocarbon ring or a substituted or unsubstituted C2 to C60 heteroring;
a1 is an integer of 1 to 4;
when, in the structural formulae of 1-B and 1-J, * is the linking position of 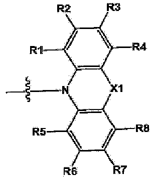 and ** is the linking position of -(L1)a1-Ar1, and R1 to R8 are all hydrogen or at least one of R1 to R8 has a phenyl group, Ar1 is a C6 to C60 aryl group unsubstituted or substituted with deuterium, a C6 to C60 aryl group or a C1 to C60 alkyl group;
when not expressed as substituents in Chemical Formula 1, they are hydrogen; or deuterium; and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

2. The heterocyclic compound of Claim 1, wherein -(L1)a1-Ar1 is represented by any one of the following Chemical Formulae 4 to 6: 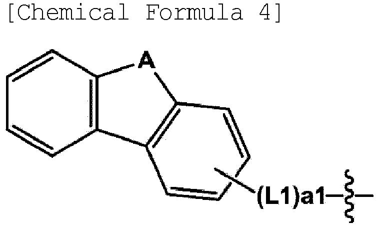 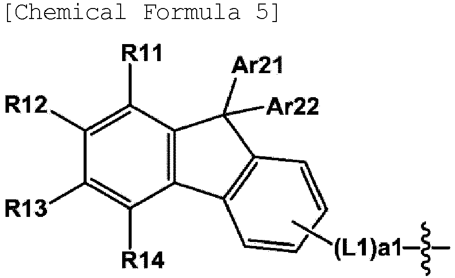 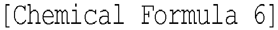 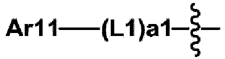 in Chemical Formulae 4 to 6,
L1 and a1 have the same definitions as in Chemical Formula 1;
A is O; or S;
R11 to R14 are the same as or different from each other, and each independently hydrogen; deuterium; a C6 to C60 aryl group; or a C1 to C60 alkyl group;
Ar21 and Ar22 are the same as or different from each other, and each independently a C1 to C60 alkyl group; or a C6 to C60 aryl group, or two groups adjacent to each other bond to each other to form a C6 to C60 aromatic hydrocarbon ring;
Ar11 is a C6 to C60 aryl group; and
when not expressed as substituents in Chemical Formulae 4 to 6, they are hydrogen; or deuterium.

3. The heterocyclic compound of Claim 2, wherein Chemical Formula 4 is represented by any one of the following Chemical Formulae 4-1 to 4-4: 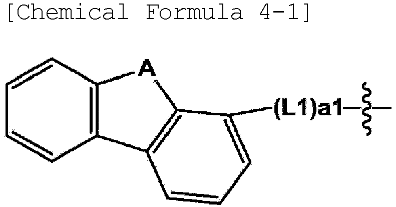 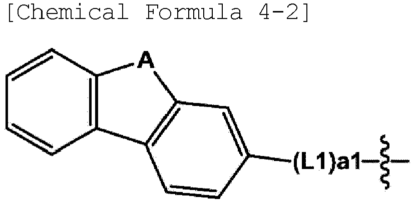 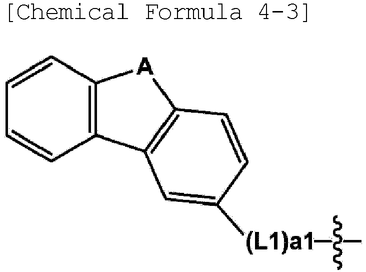 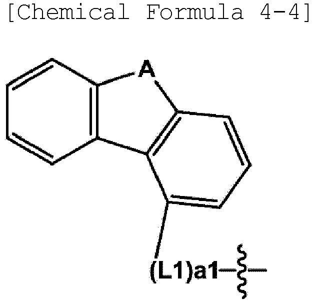 in Chemical Formulae 4-1 to 4-4,
L1, a1 and A have the same definitions as in Chemical Formula 4.

4. The heterocyclic compound of Claim 2, wherein Chemical Formula 5 is represented by the following Chemical Formula 5-1 or 5-2: 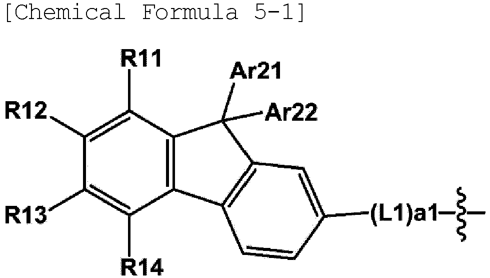 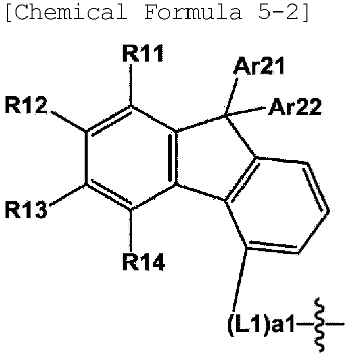 in Chemical Formulae 5-1 and 5-2,
each substituent has the same definition as in Chemical Formula 5.

5. The heterocyclic compound of Claim 1, wherein R1 to R8 are the same as or different from each other, and each independently hydrogen; deuterium; a dibenzofuran group; a dibenzothiophene group; a dimethylfluorenyl group; or a triphenylenyl group, or two or more groups adjacent to each other bond to each other to form an indole ring unsubstituted or substituted with a phenyl group; a benzothiophene ring; a benzofuran ring; or an indene ring unsubstituted or substituted with a methyl group.

6. The heterocyclic compound of Claim 1, wherein Chemical Formula 1 is represented by any one of the following compounds:

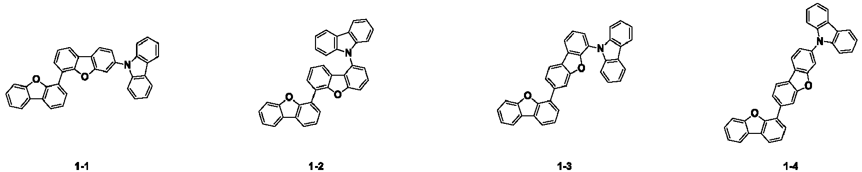

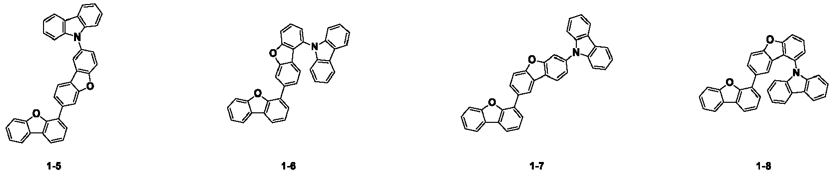

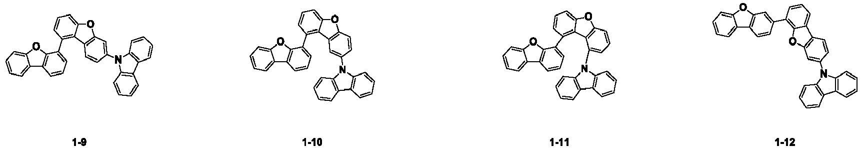

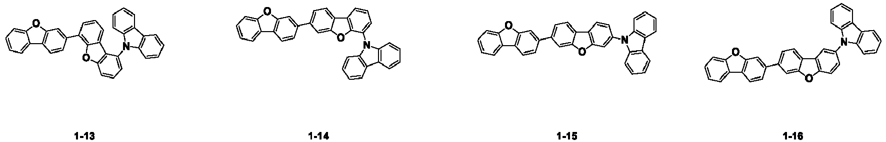

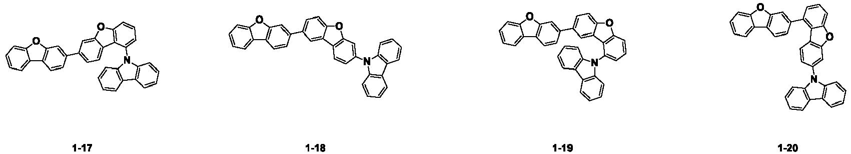

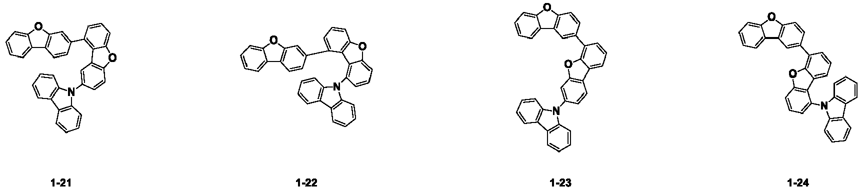

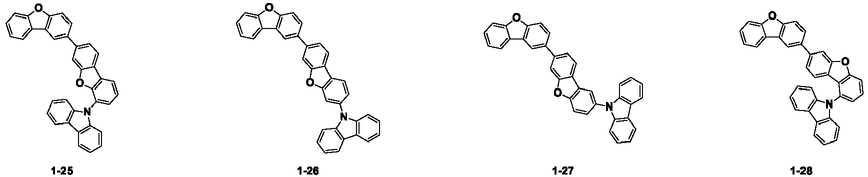

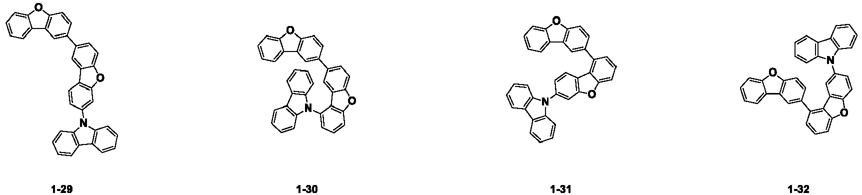

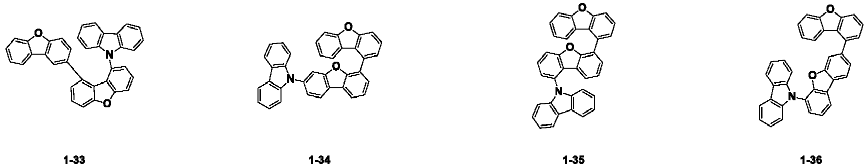

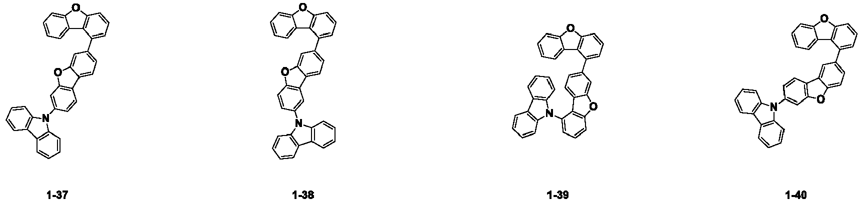

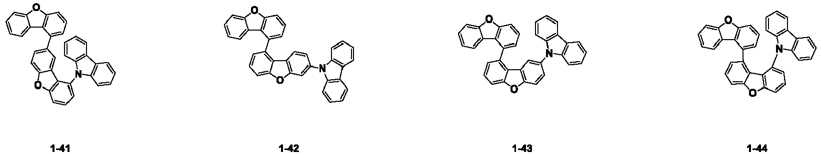

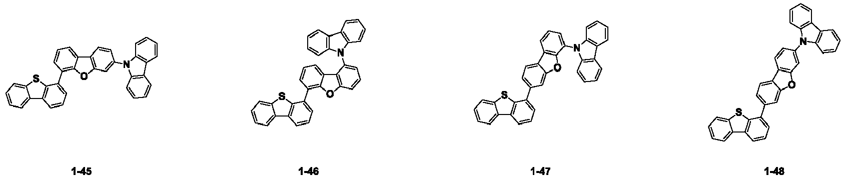

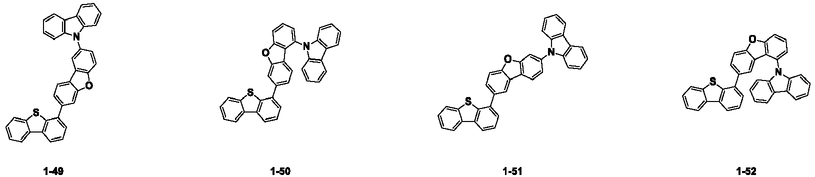

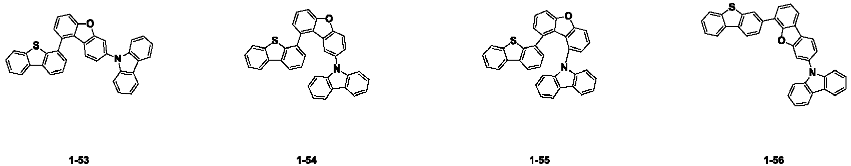

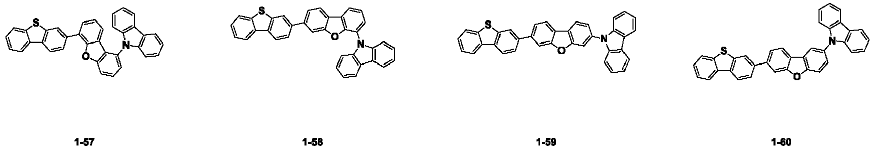

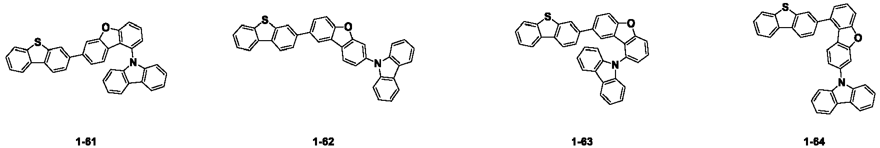

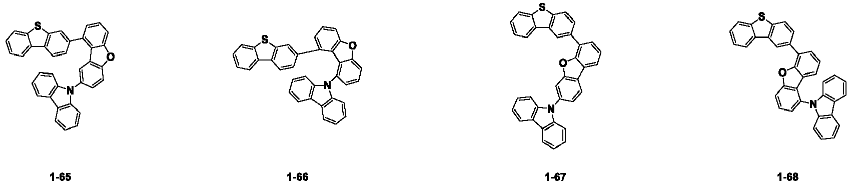

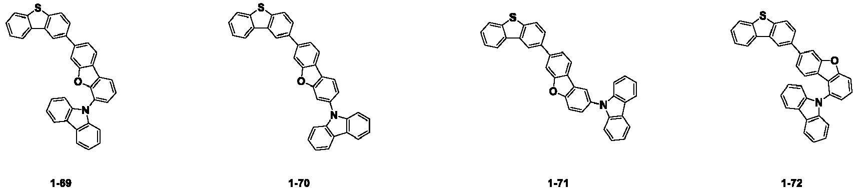

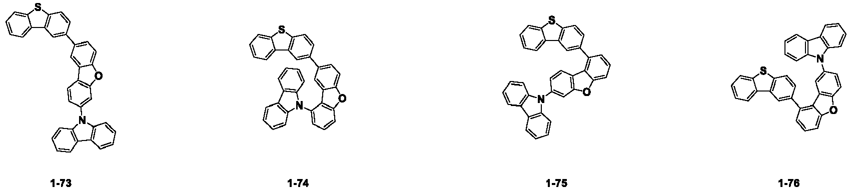

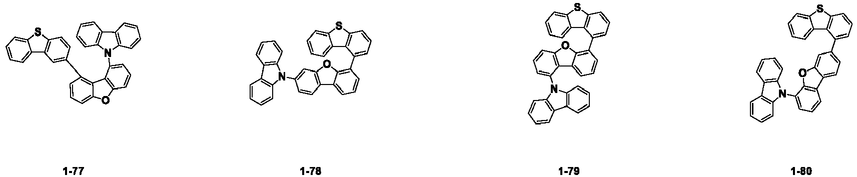

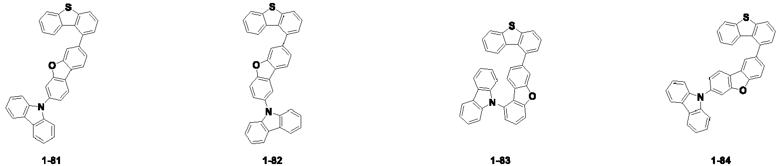

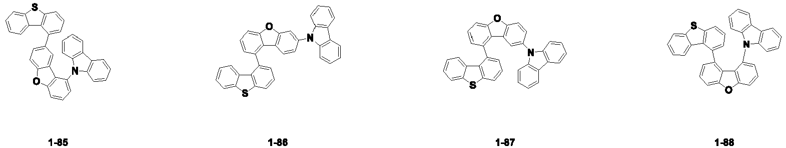

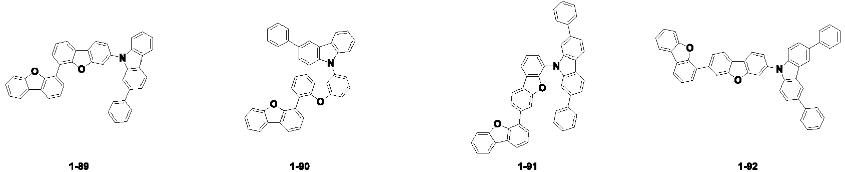

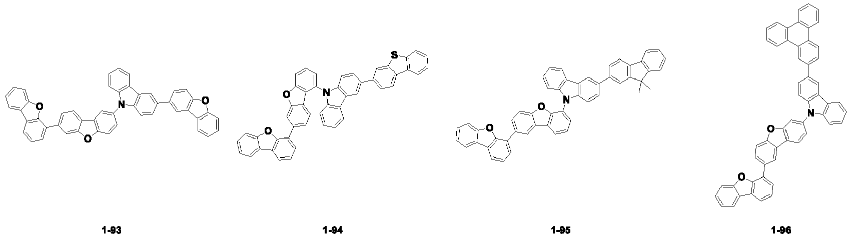

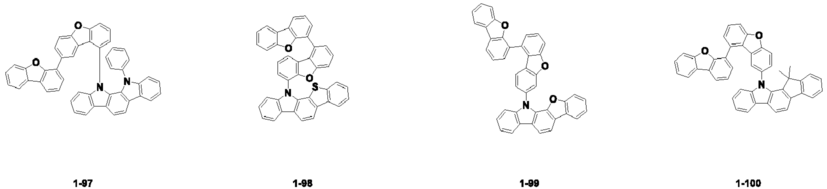

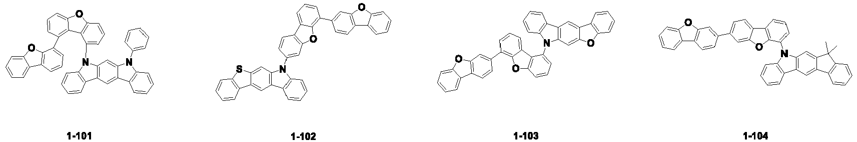

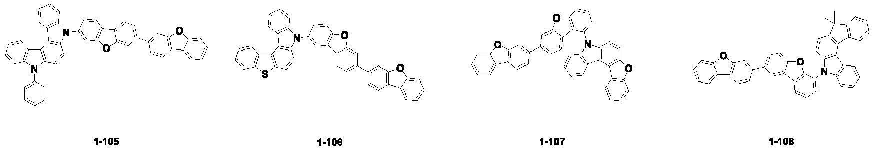

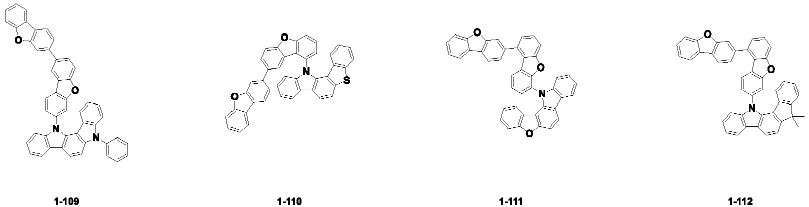

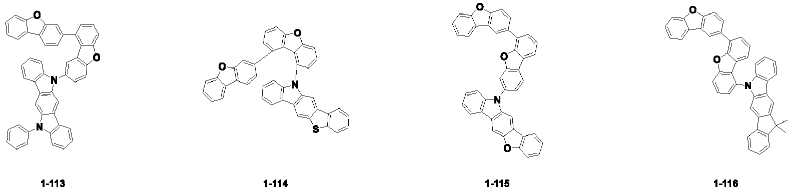

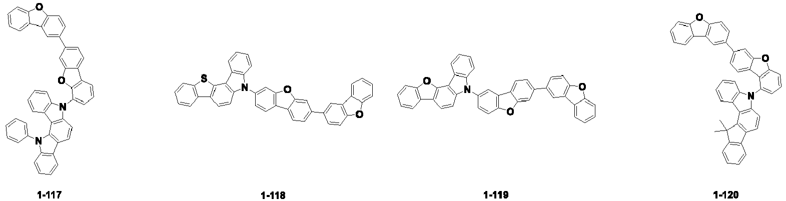

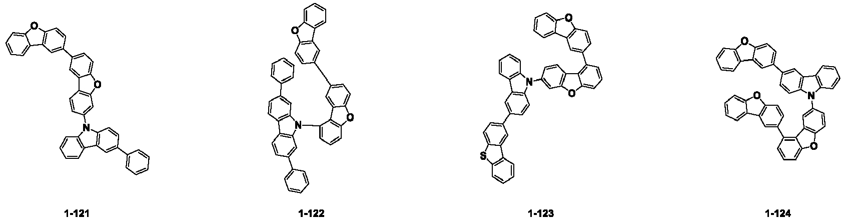

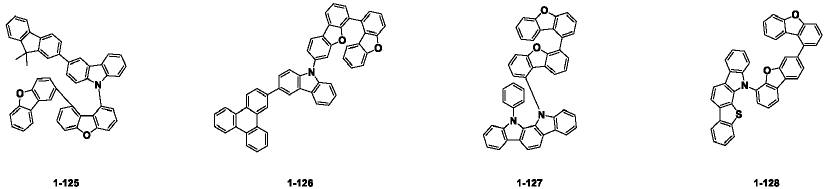

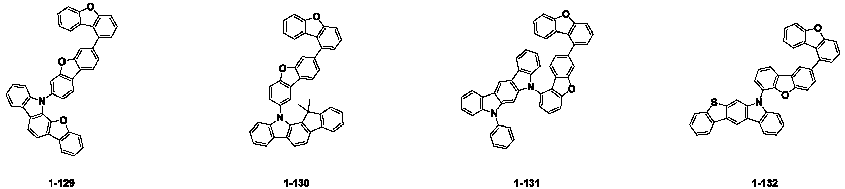

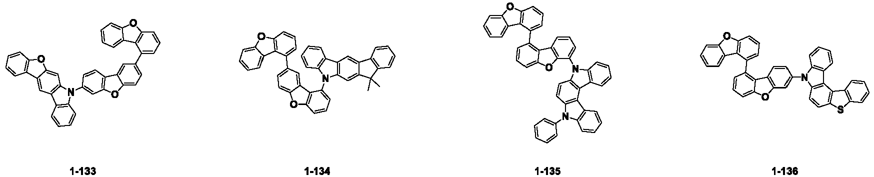

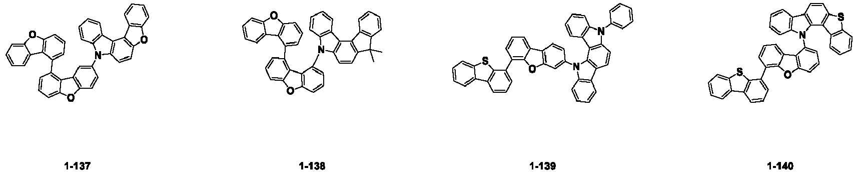

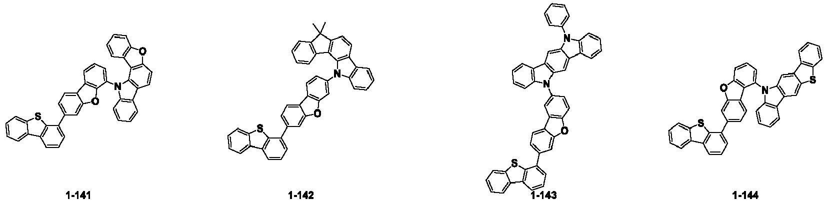

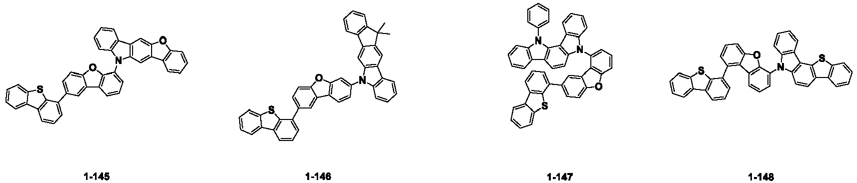

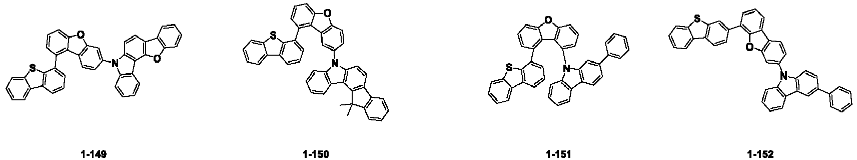

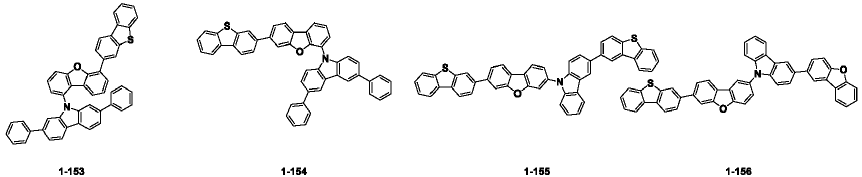

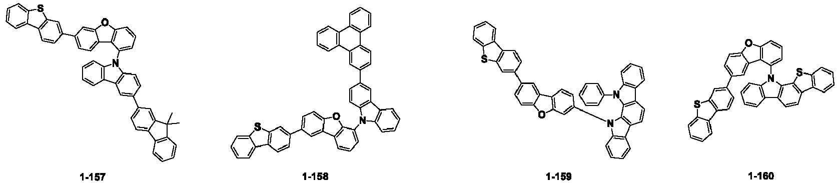

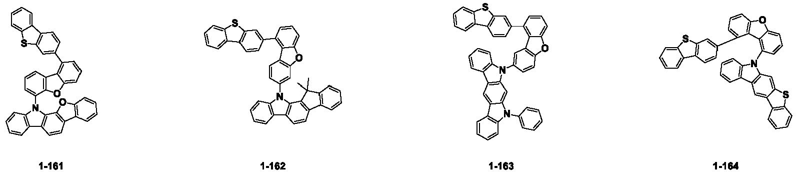

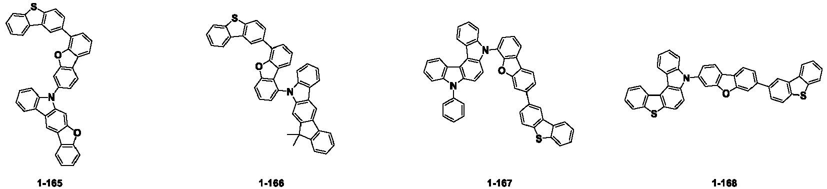

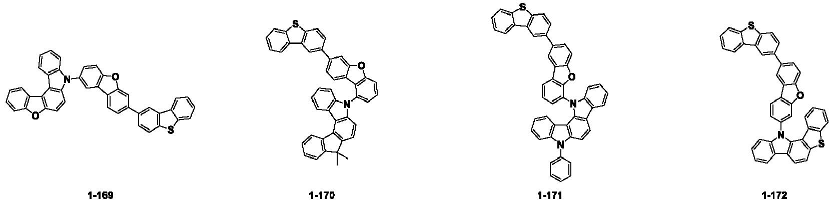

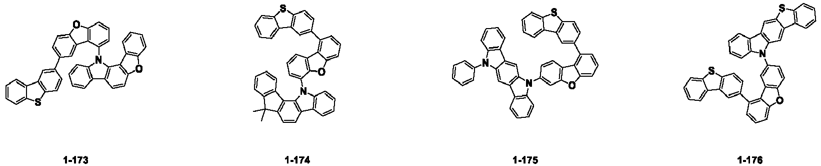

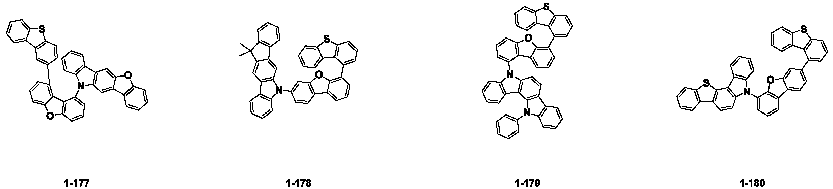

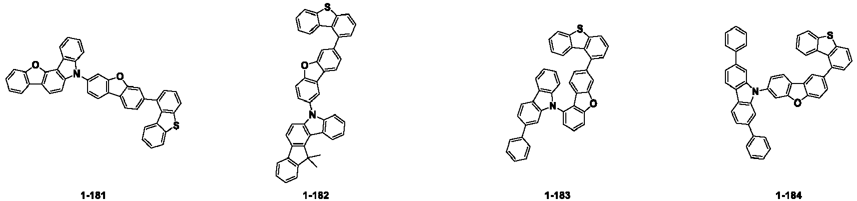

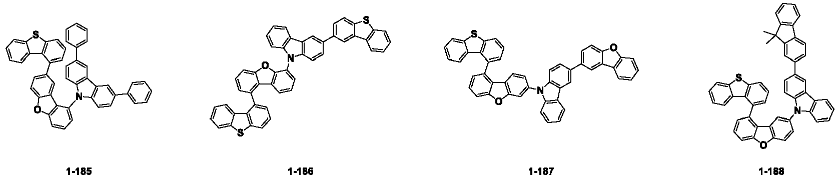

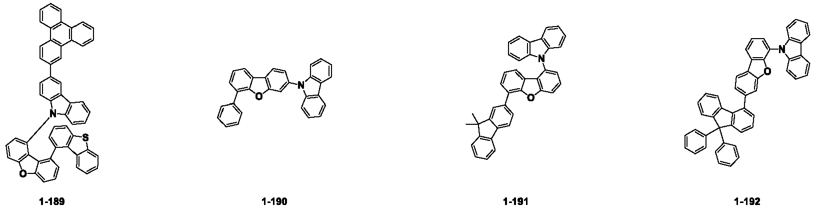

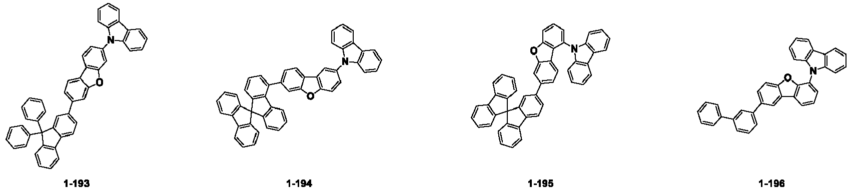

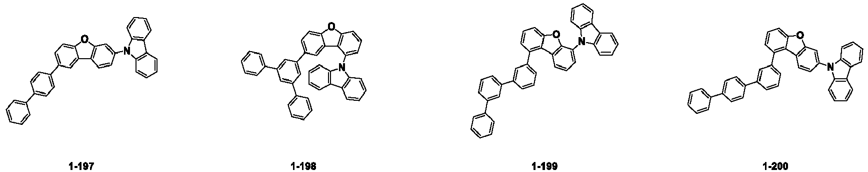

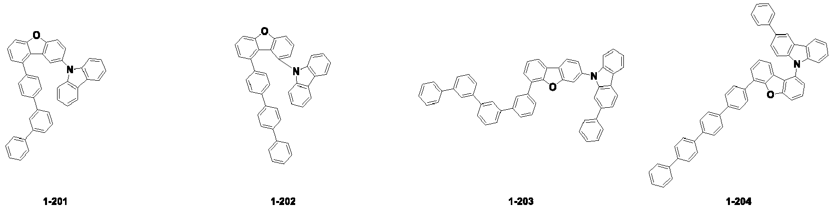

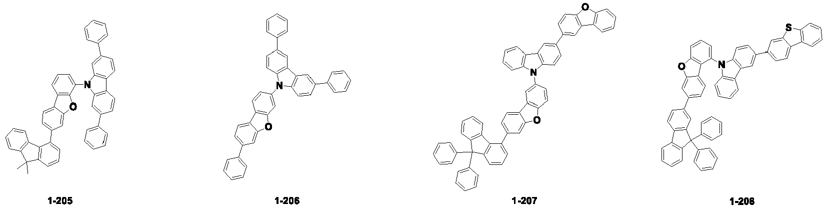

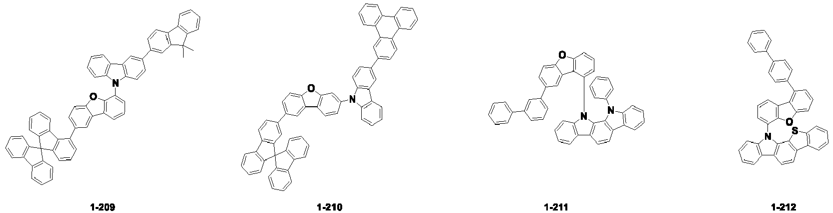

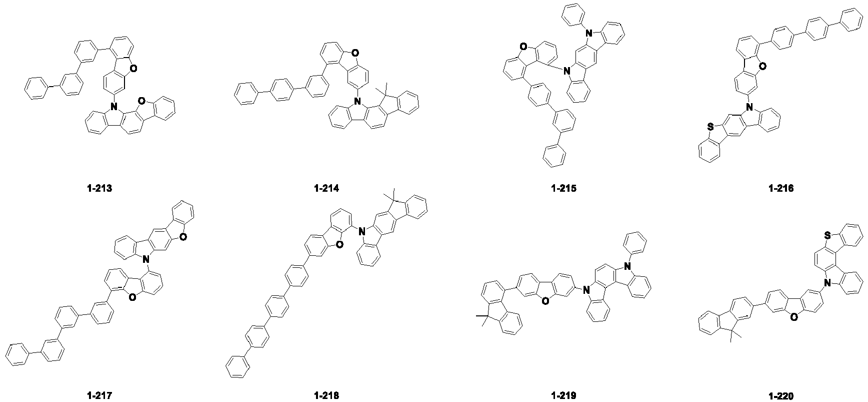

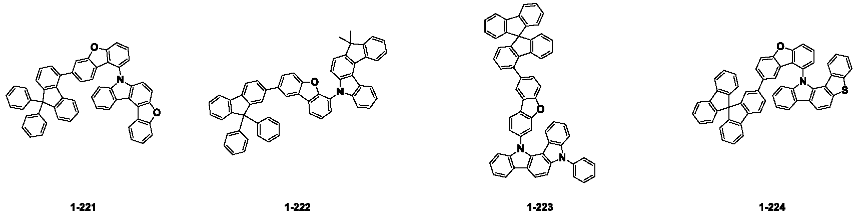

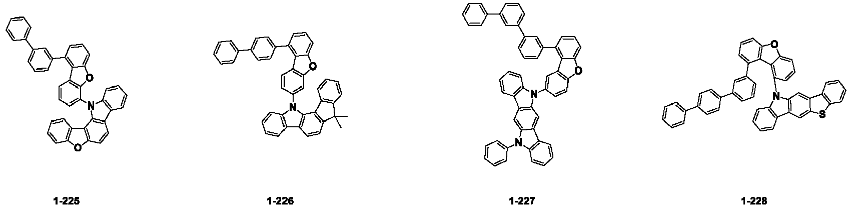

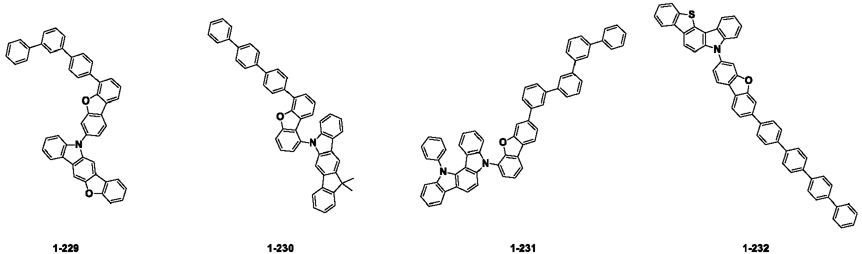

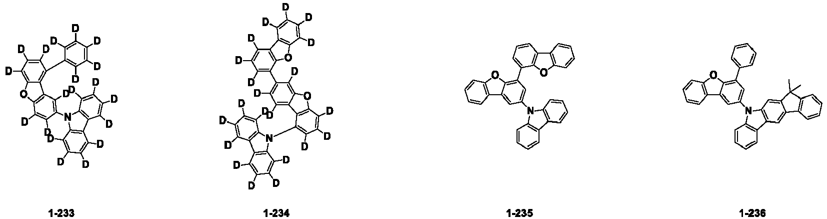

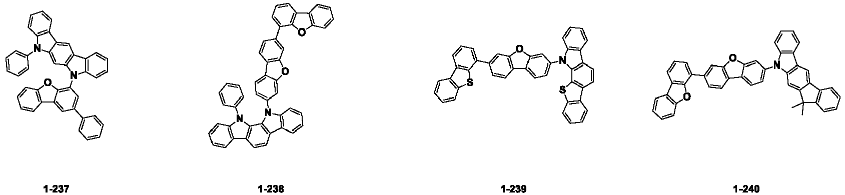

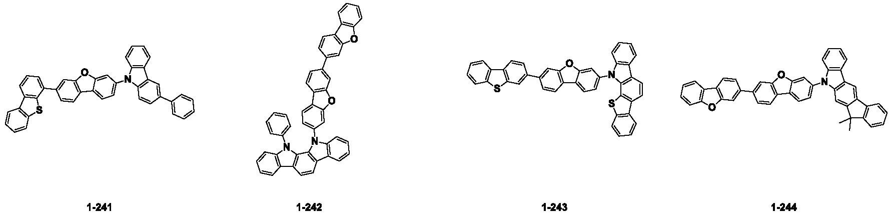

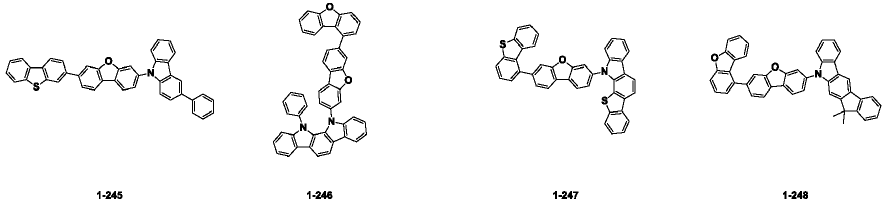

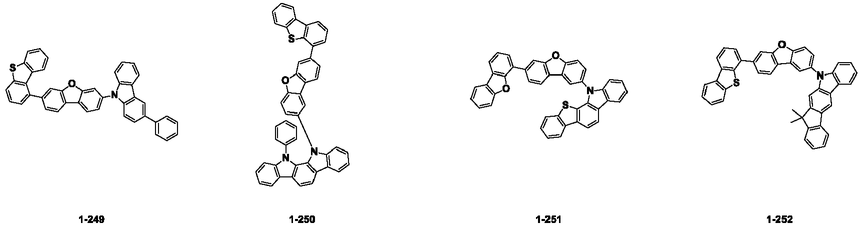

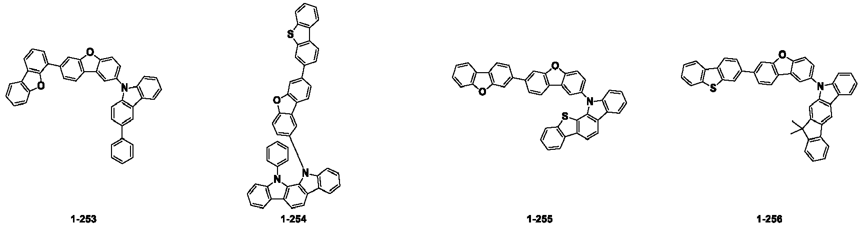

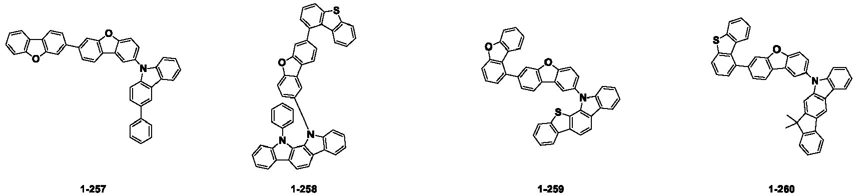

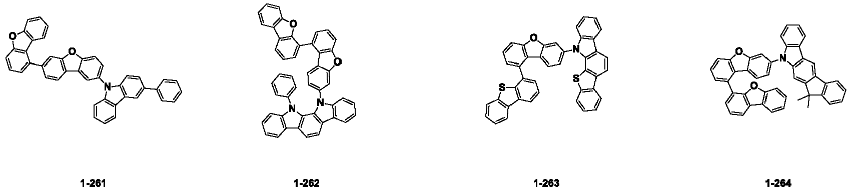

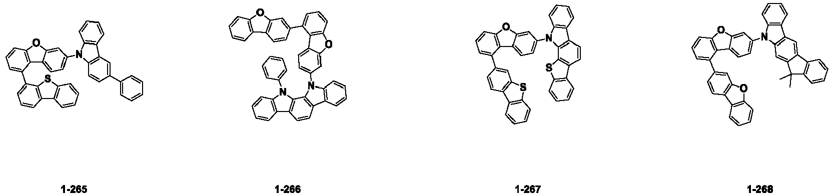

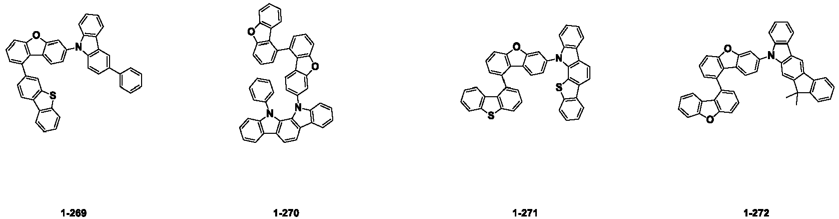

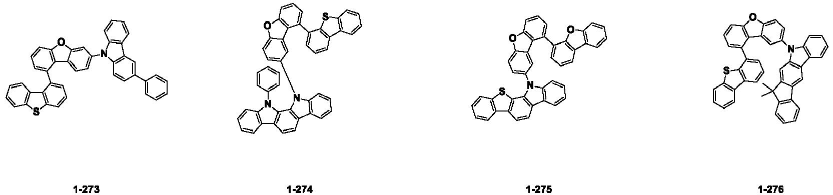

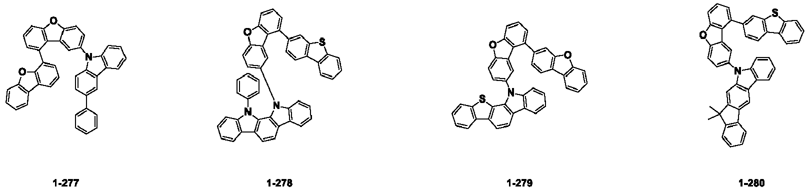

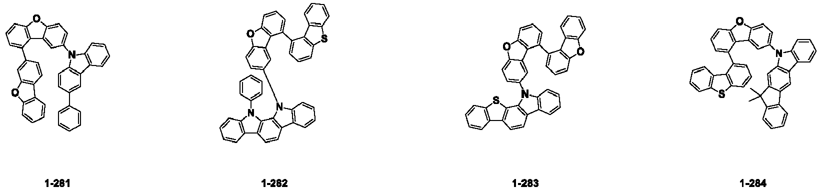

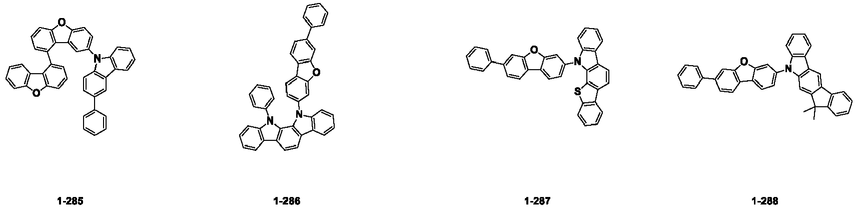

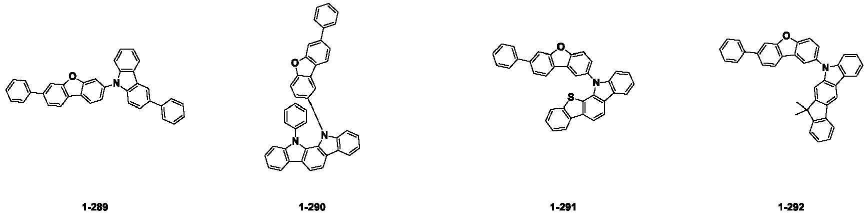

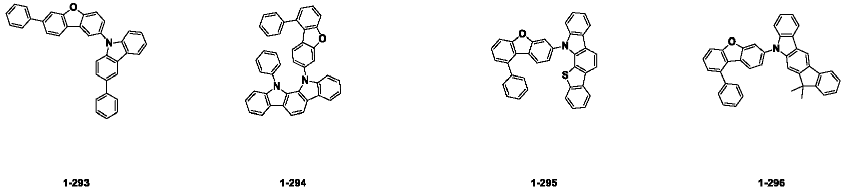

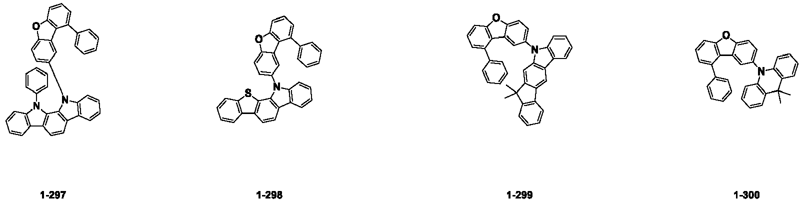

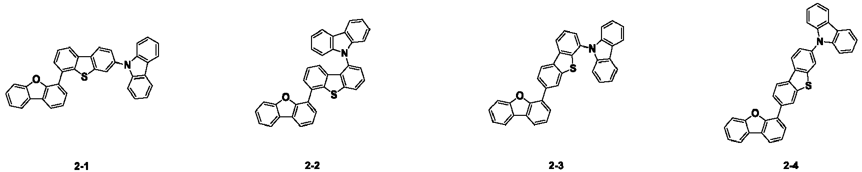

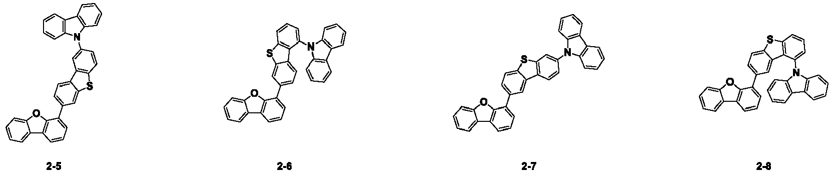

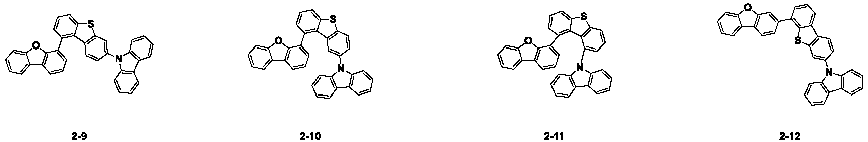

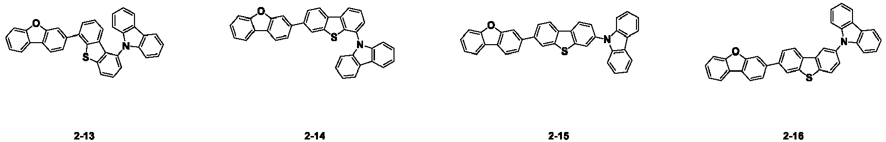

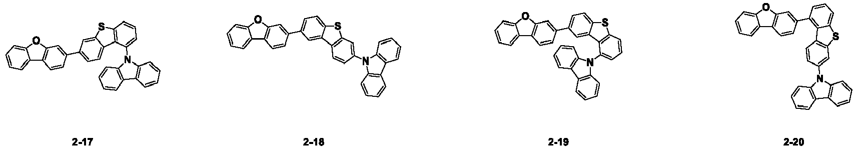

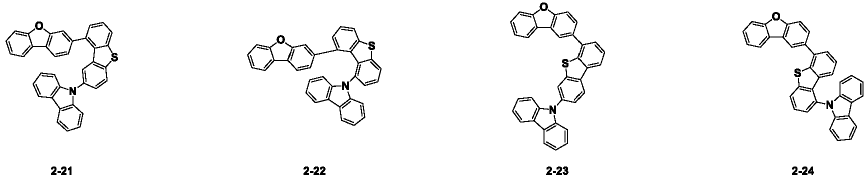

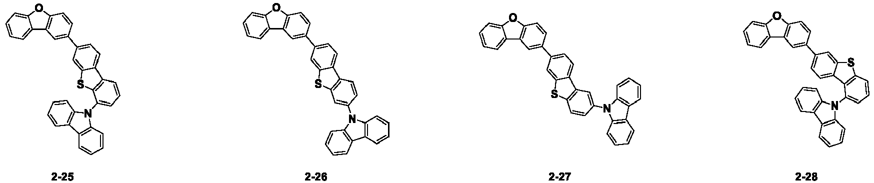

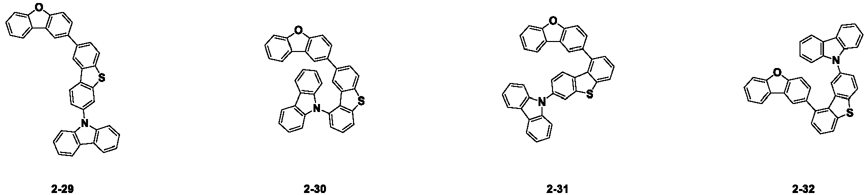

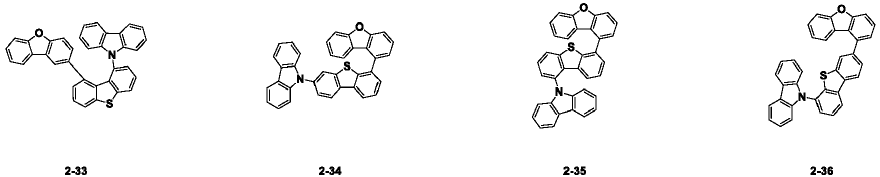

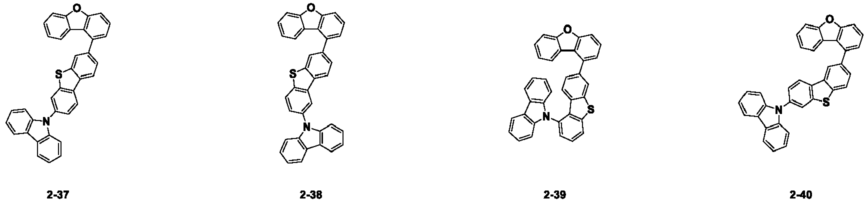

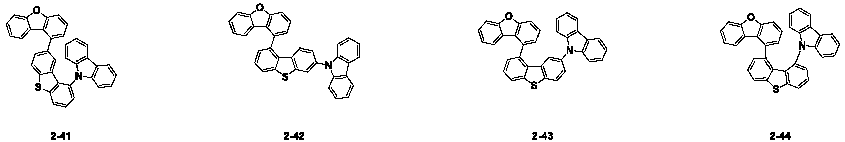

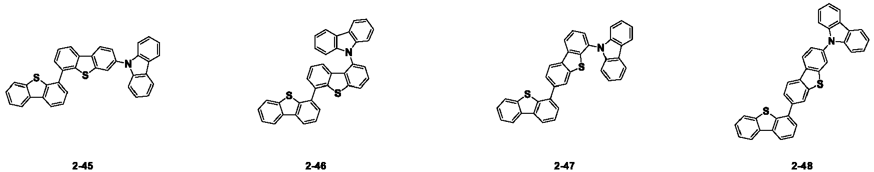

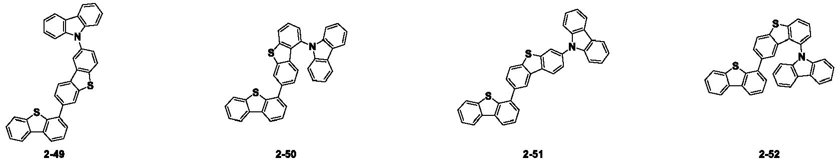

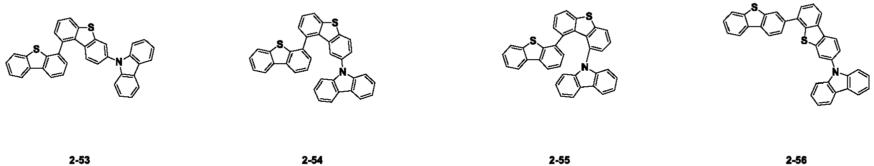

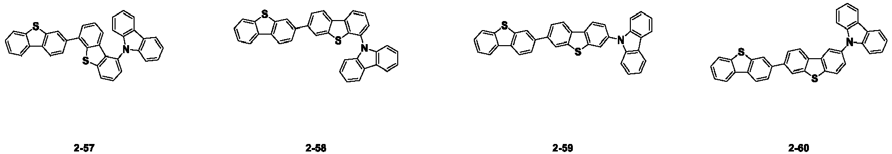

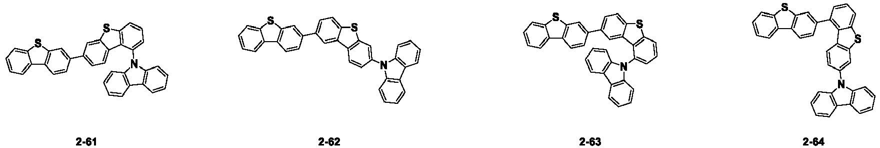

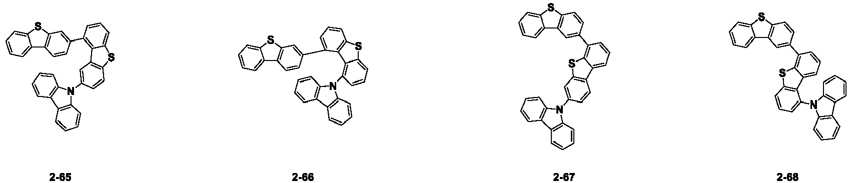

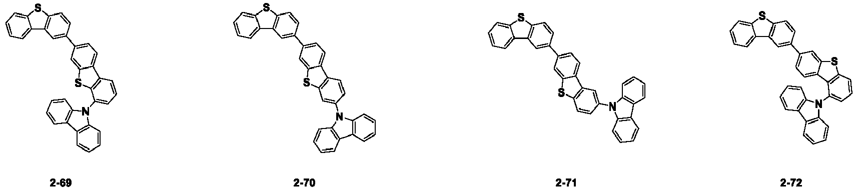

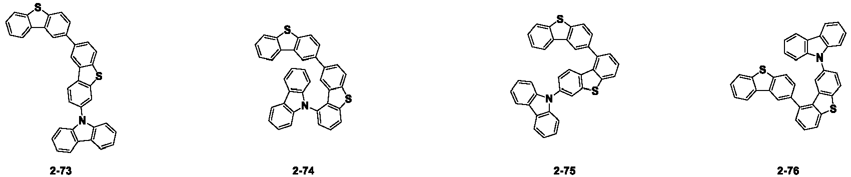

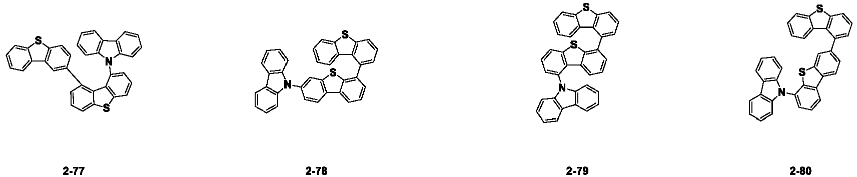

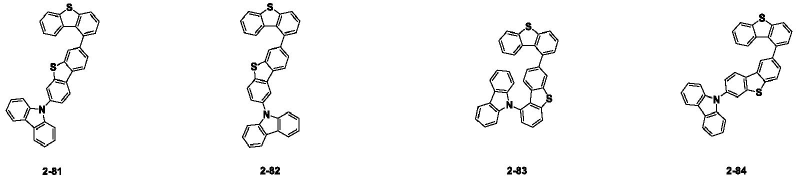

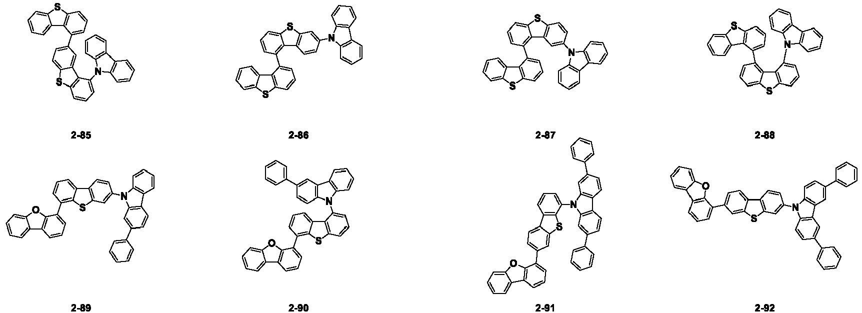

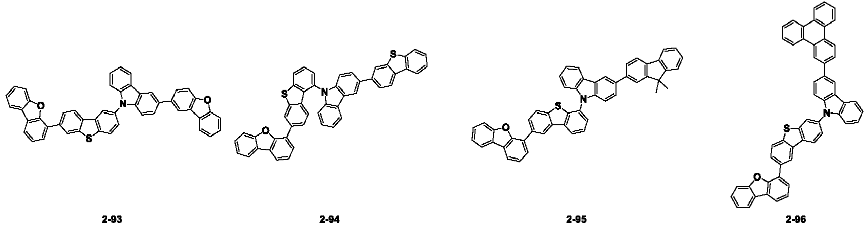

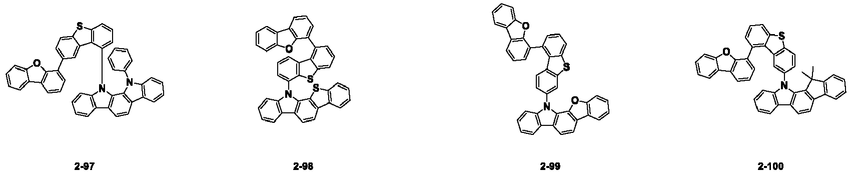

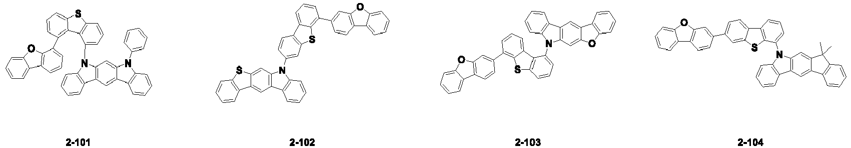

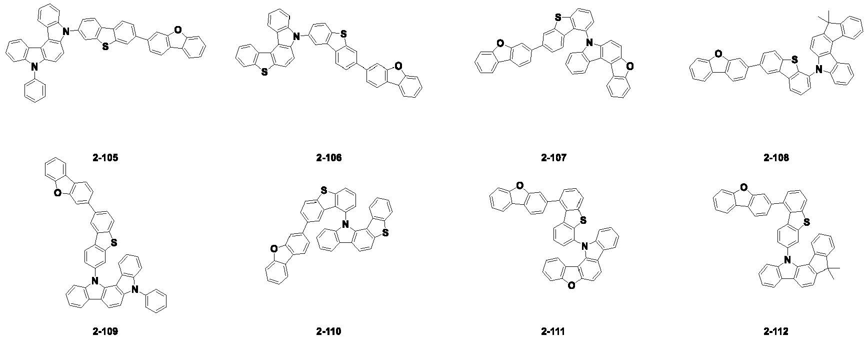

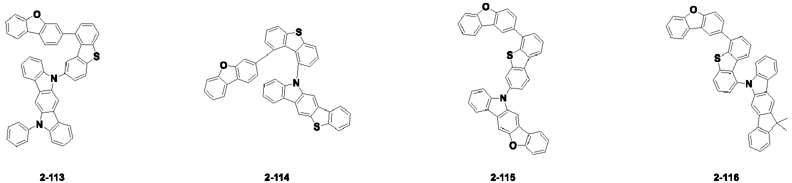

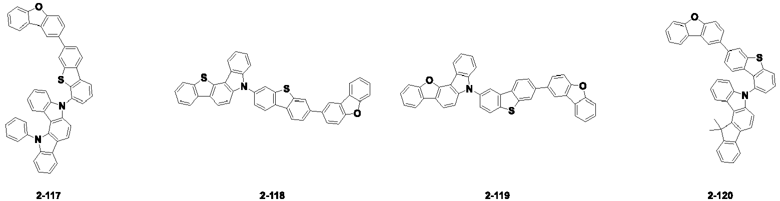

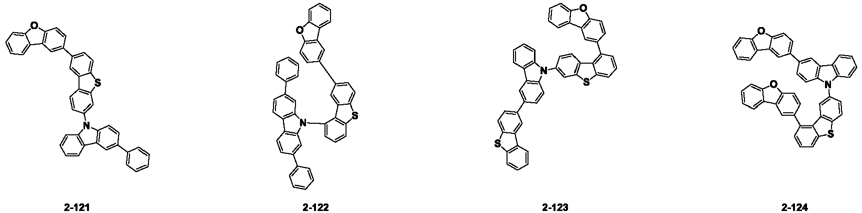

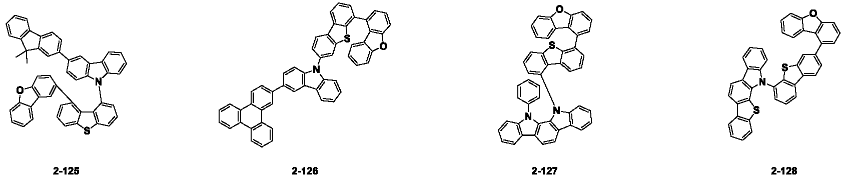

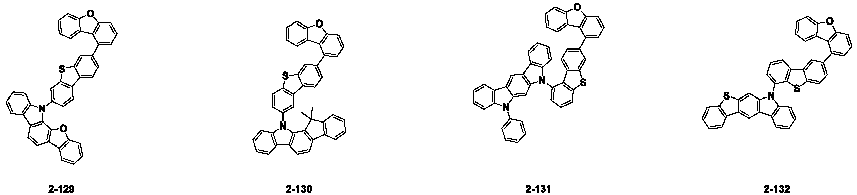

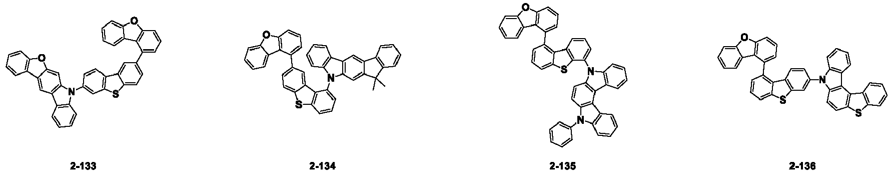

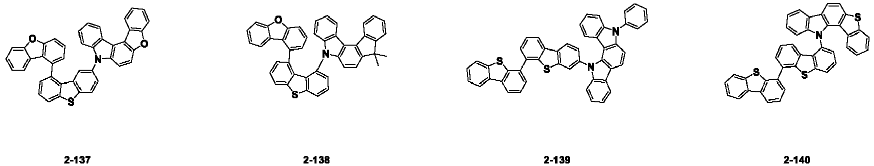

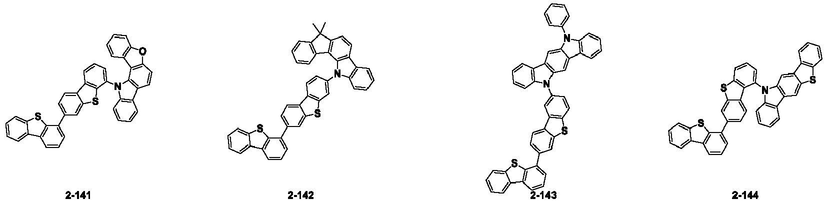

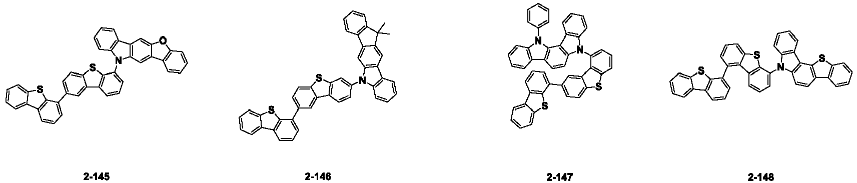

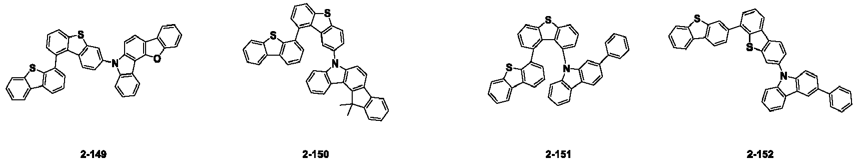

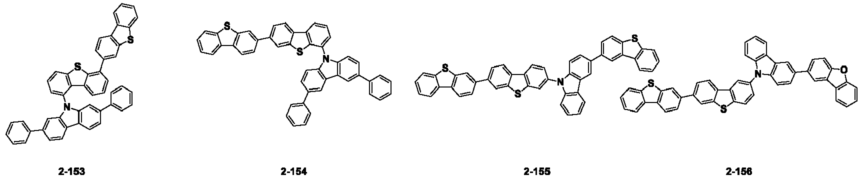

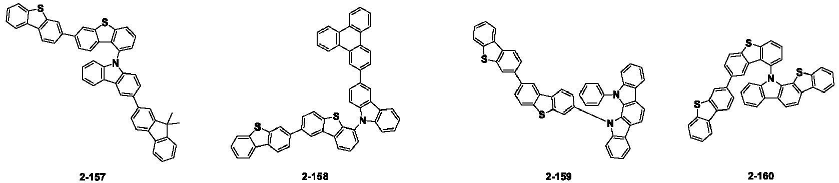

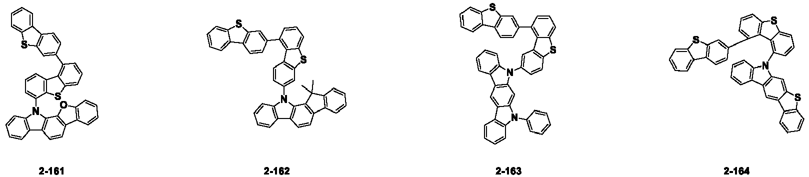

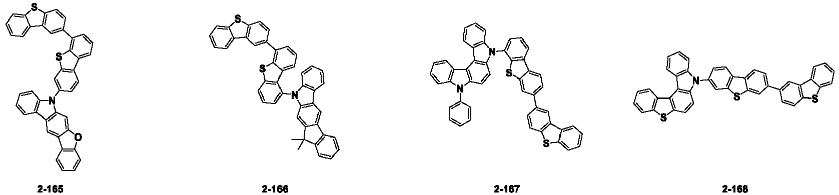

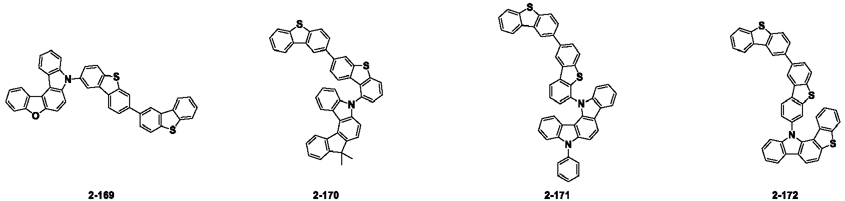

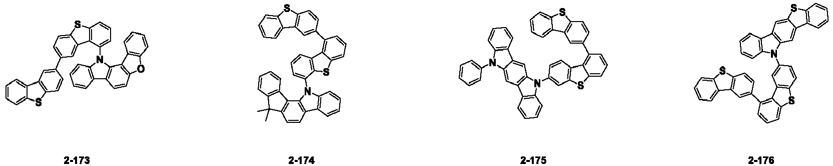

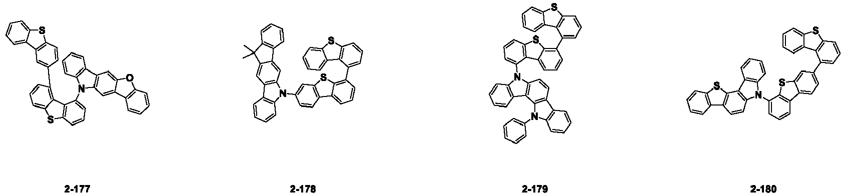

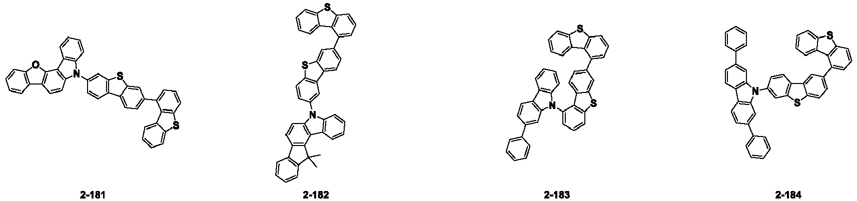

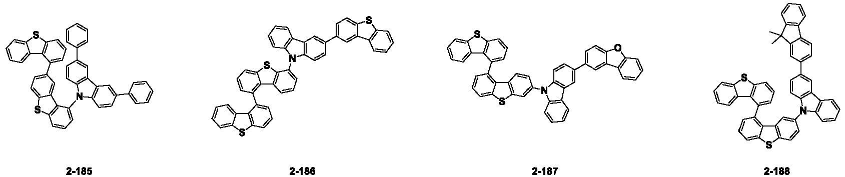

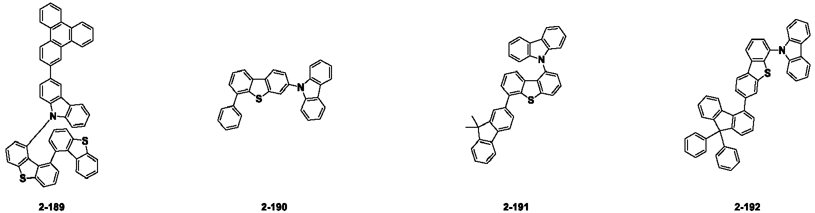

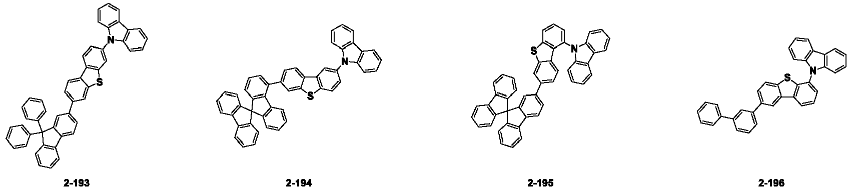

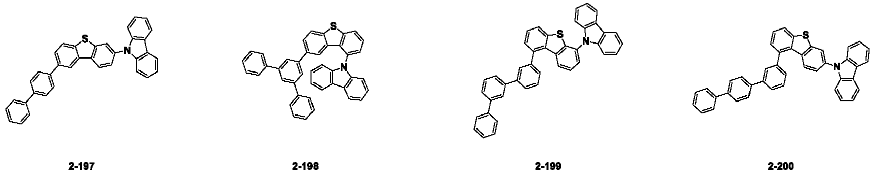

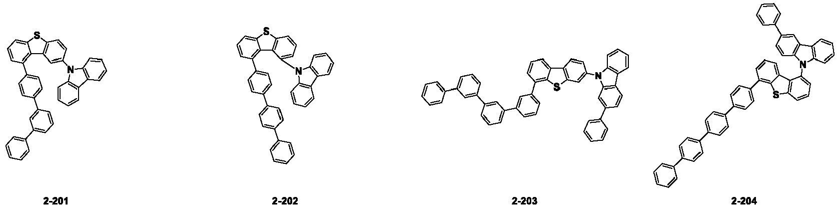

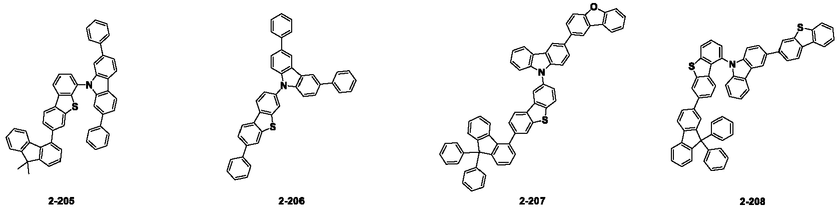

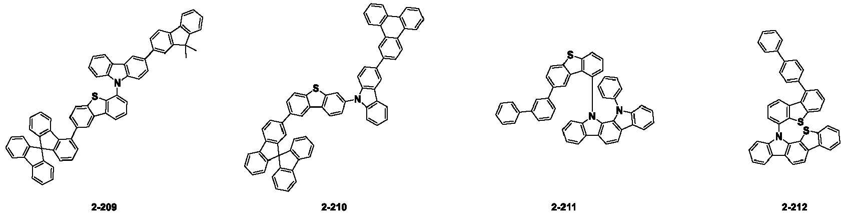

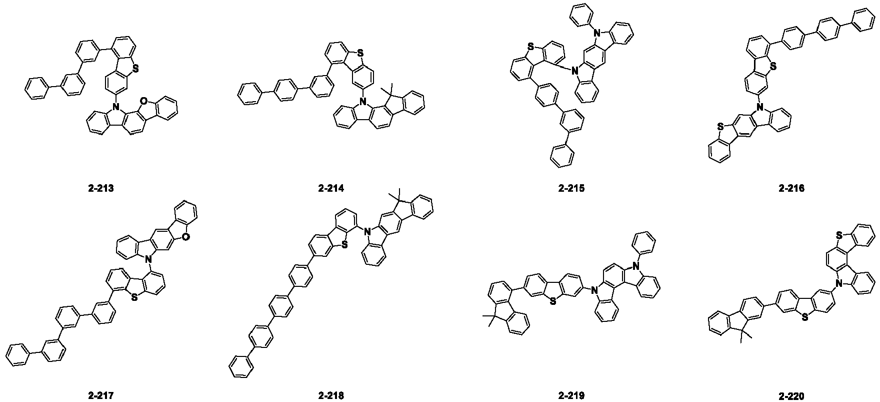

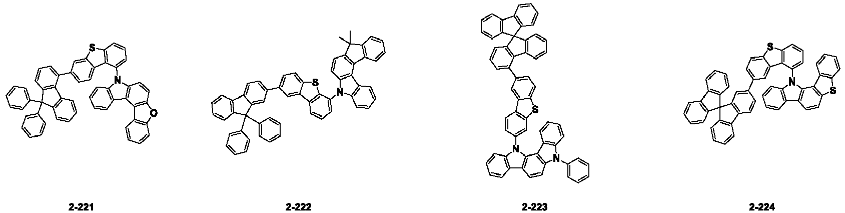

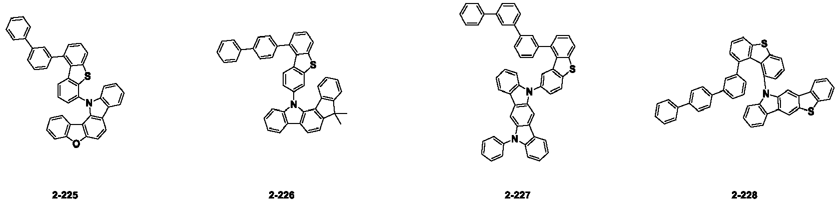

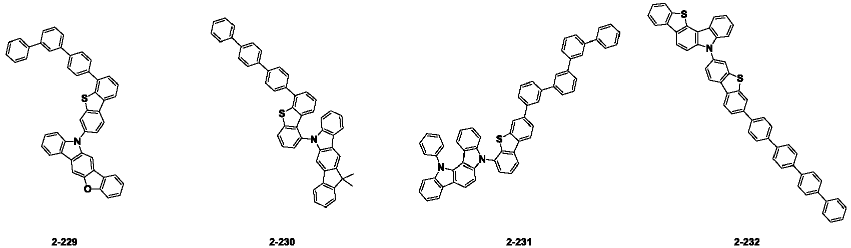

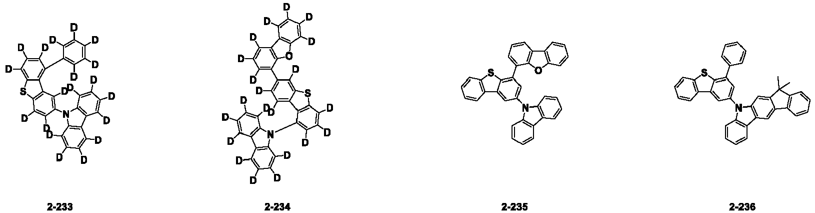

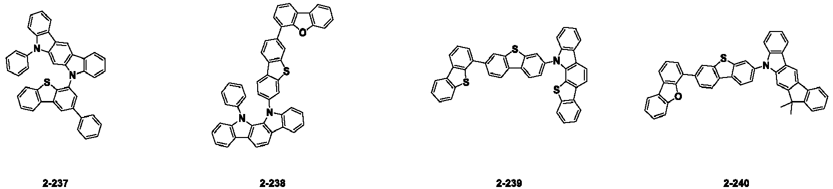

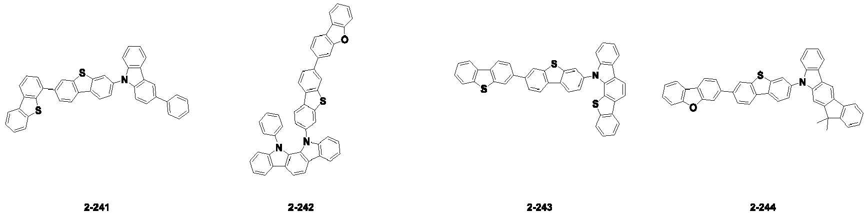

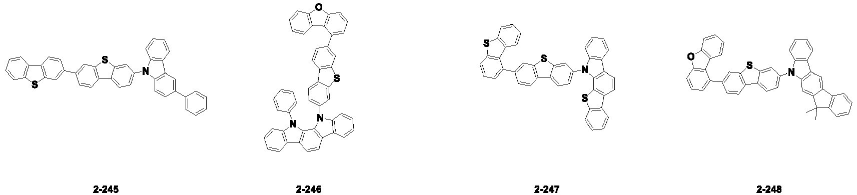

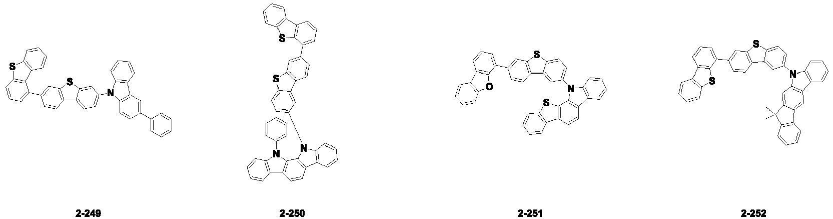

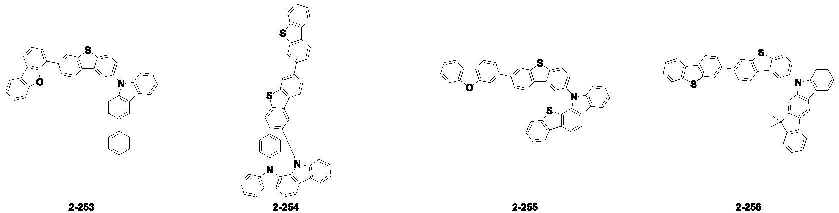

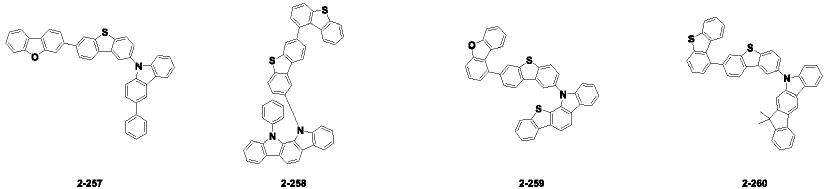

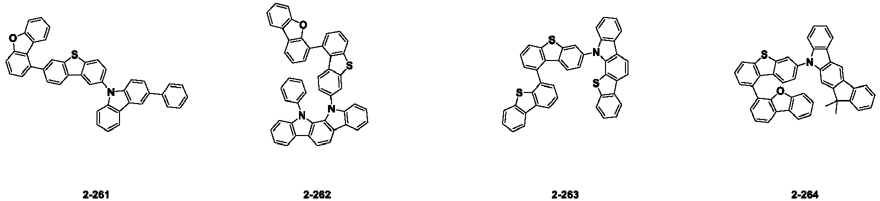

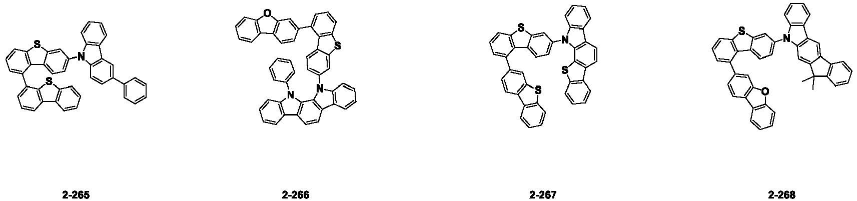

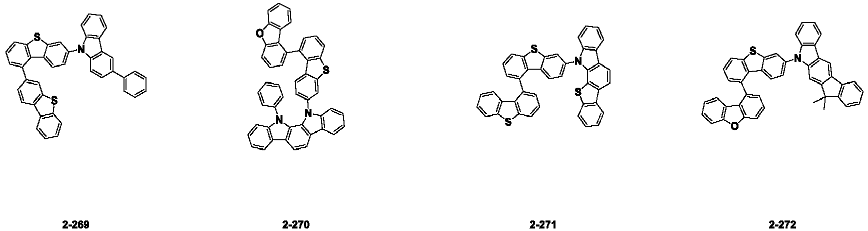

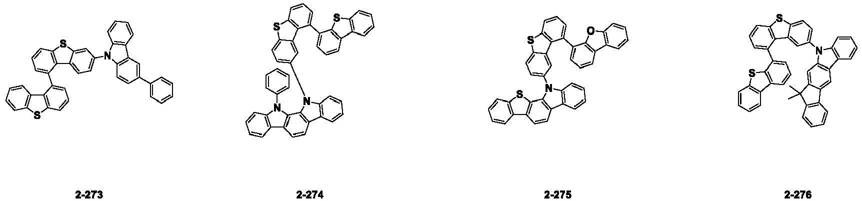

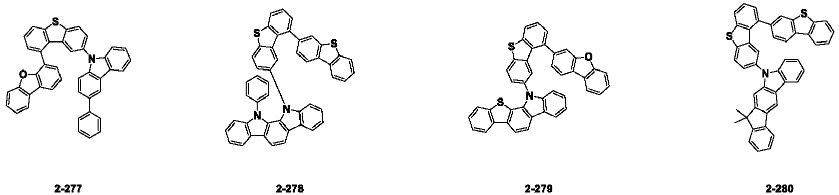

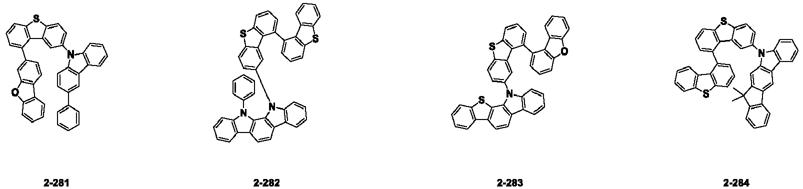

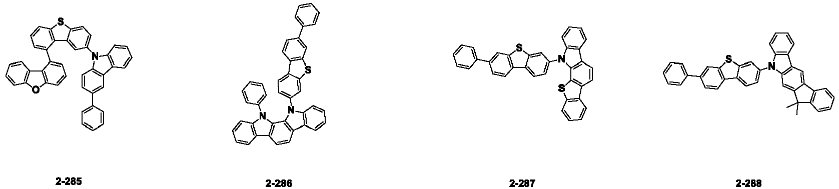

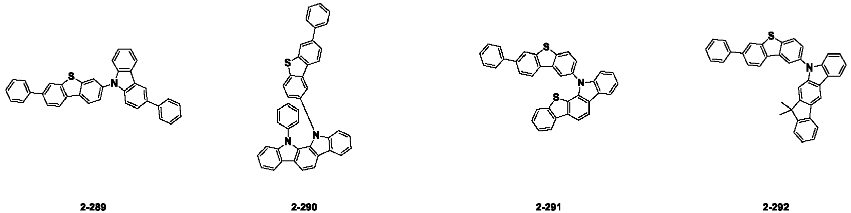

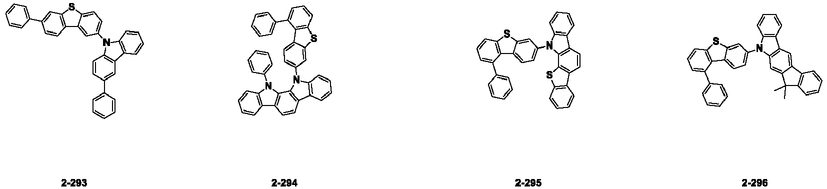

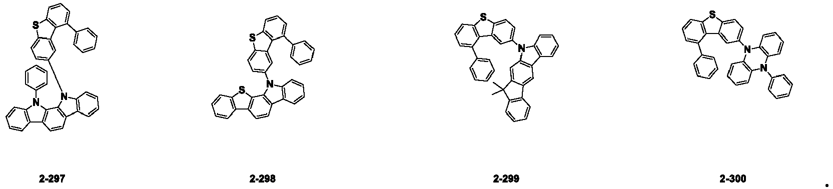

7. An organic light emitting device comprising:
a first electrode;
a second electrode provided opposite to the first electrode; and
one or more organic material layers provided between the first electrode and the second electrode,
wherein one or more layers of the organic material layers include one or more of the heterocyclic compound of any one of Claims 1 to 6.

8. The organic light emitting device of Claim 7, wherein the organic material layer includes a light emitting layer, and the light emitting layer includes the heterocyclic compound.

9. The organic light emitting device of Claim 7, wherein the organic material layer includes a light emitting layer, the light emitting layer includes a host material, and the host material includes the heterocyclic compound.

10. The organic light emitting device of Claim 7, wherein the organic material layer includes an electron injection layer or an electron transfer layer, and the electron injection layer or the electron transfer layer includes the heterocyclic compound.

11. The organic light emitting device of Claim 7, wherein the organic material layer includes an electron blocking layer or a hole blocking layer, and the electron blocking layer or the hole blocking layer includes the heterocyclic compound.

12. The organic light emitting device of Claim 7, further comprising one, two or more layers selected from the group consisting of a light emitting layer, a hole injection layer, a hole transfer layer, an electron injection layer, an electron transfer layer, an electron blocking layer and a hole blocking layer.

13. The organic light emitting device of Claim 7, wherein the organic material layer further includes a heterocyclic compound represented by the following Chemical Formula 2; or a heterocyclic compound represented by the following Chemical Formula 3: 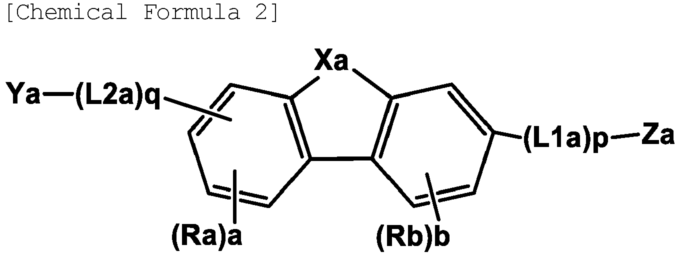 in Chemical Formula 2,
Xa is O or S;
Ya is a hole transferring group or a substituted or unsubstituted aryl group;
Za is an electron transferring group;
L1a and L2a are the same as or different from each other, and each independently a direct bond; a substituted or unsubstituted C6 to C60 arylene group; or a substituted or unsubstituted C2 to C60 heteroarylene group, p and q are each an integer of 0 to 3, when p is 2 or greater, L1as are the same as or different from each other, and when q is 2 or greater, L2as are the same as or different from each other; and
Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, a and b are each an integer of 0 to 3, when a is 2 or greater, Ras are the same as or different from each other, and when b is 2 or greater, Rbs are the same as or different from each other, 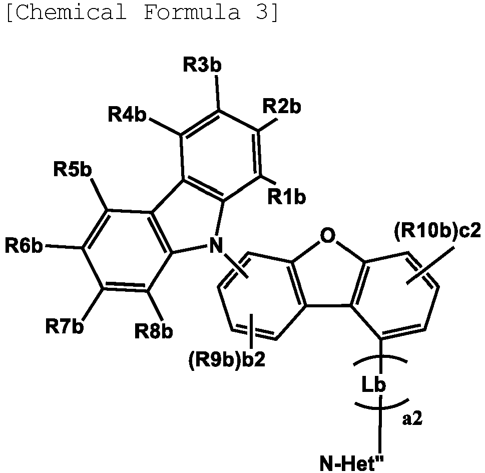 in Chemical Formula 3,
N-Het" is a monocyclic or polycyclic heterocyclic group substituted or unsubstituted and including one or more Ns;
Lb is a direct bond; a substituted or unsubstituted arylene group; or a substituted or unsubstituted heteroarylene group, a2 is an integer of 1 to 3, and when a2 is 2 or greater, Lbs are the same as or different from each other; and
R1b to R10b are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, b2 and c2 are each an integer of 0 to 3, when b2 is 2 or greater, R9bs are the same as or different from each other, and when c2 is 2 or greater, R10bs are the same as or different from each other.

14. A composition for an organic material layer of an organic light emitting device, the composition comprising:
the heterocyclic compound represented by Chemical Formula 1 of any one of Claims 1 to 6; and
a heterocyclic compound represented by the following Chemical Formula 2 or a heterocyclic compound represented by the following Chemical Formula 3: 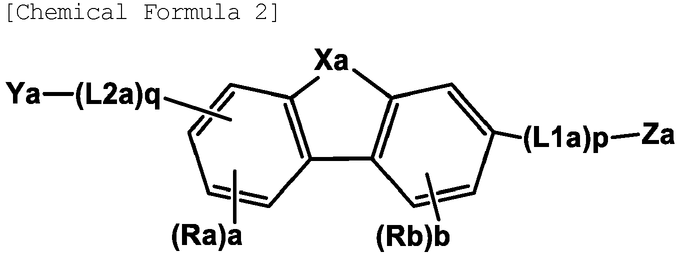 wherein, in Chemical Formula 2,
Xa is O or S;
Ya is a hole transferring group or a substituted or unsubstituted aryl group;
Za is an electron transferring group;
L1a and L2a are the same as or different from each other, and each independently a direct bond; a substituted or unsubstituted C6 to C60 arylene group; or a substituted or unsubstituted C2 to C60 heteroarylene group, p and q are each an integer of 0 to 3, when p is 2 or greater, L1as are the same as or different from each other, and when q is 2 or greater, L2as are the same as or different from each other; and
Ra and Rb are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, a and b are each an integer of 0 to 3, when a is 2 or greater, Ras are the same as or different from each other, and when b is 2 or greater, Rbs are the same as or different from each other, 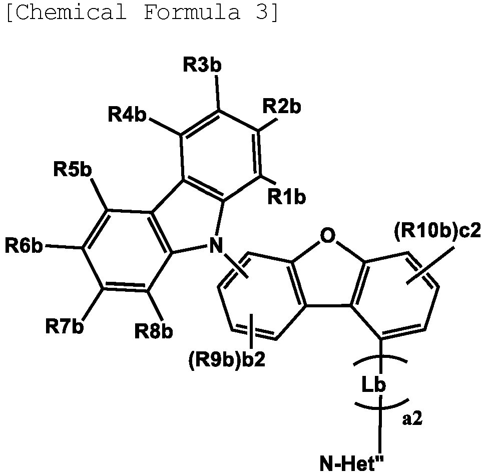 in Chemical Formula 3,
N-Het" is a monocyclic or polycyclic heterocyclic group substituted or unsubstituted and including one or more Ns;
Lb is a direct bond; a substituted or unsubstituted arylene group; or a substituted or unsubstituted heteroarylene group, a2 is an integer of 1 to 3, and when a2 is 2 or greater, Lbs are the same as or different from each other; and
R1b to R10b are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C3 to C60 heterocycloalkyl group; a substituted or unsubstituted C6 to C60 aryl group; and a substituted or unsubstituted C2 to C60 heteroaryl group, or two or more groups adjacent to each other bond to each other to form a substituted or unsubstituted C6 to C60 aromatic hydrocarbon ring or a substituted or unsubstituted C2 to C60 heteroring, b2 and c2 are each an integer of 0 to 3, when b2 is 2 or greater, R9bs are the same as or different from each other, and when c2 is 2 or greater, R10bs are the same as or different from each other.

15. The composition for an organic material layer of an organic light emitting device of Claim 14, wherein, in the composition, the heterocyclic compound represented by Chemical Formula 1: the heterocyclic compound represented by Chemical Formula 2 or the heterocyclic compound represented by Chemical Formula 3 have a weight ratio of 1:10 to 10:1.

16. A method for manufacturing an organic light emitting device, the method comprising:
preparing a substrate;
forming a first electrode on the substrate;
forming one or more organic material layers on the first electrode; and
forming a second electrode on the organic material layers,
wherein the forming of organic material layers includes forming one or more organic material layers using the composition for an organic material layer of Claim 14.

17. The method for manufacturing an organic light emitting device of Claim 16, wherein the forming of organic material layers is forming using a thermal vacuum deposition method after pre-mixing the heterocyclic compound of Chemical Formula 1; and the heterocyclic compound represented by Chemical Formula 2 or the heterocyclic compound represented by Chemical Formula 3.
